# FASCICULE DE BREVET EUROPEEN

(11) **EP 2 513 108 B1**
(45) Date de publication et mention de la délivrance du brevet: **01.04.2015**
(21) Numéro de dépôt: 10801662.7
(22) Date de dépôt: 13.12.2010
(51) Int. Cl.: C07D 471/04, C07D 495/04, C07D 513/04, A61K 31/429, A61K 31/381

(54) **NOUVEAUX DERIVES D' (HETEROCYCLE-PIPERIDINE CONDENSEE)-(PIPERAZINYL)-1-ALCANONE OU D' (HETEROCYCLE-PYRROLIDINE CONDENSEE)-(PIPERAZINYL)-1-ALCANONE ET LEUR UTILISATION COMME INHIBITEURS DE p75**
NEUE (HETEROCYCLISCHE/KONDENSIERTE PIPERIDIN)-(PIPERAZINYL)-1-ALCANON- ODER (HETEROCYCLISCHE/KONDENSIERTE PYRROLIDIN)-(PIPERAZINYL)-1-ALCANON-DERIVATE UND IHRE VERWENDUNG ALS P75-HEMMER
NOVEL (HETEROCYCLE/CONDENSED PIPERIDINE)-(PIPERAZINYL)-1-ALCANONE OR (HETEROCYCLE/CONDENSED PYRROLIDINE)-(PIPERAZINYL)-1-ALCANONE DERIVATIVES, AND USE THEREOF AS P75 INHIBITORS

(30) Priorité: 14.12.2009 FR 0906023
(43) Date de publication de la demande: 24.10.2012
(73) Titulaire: SANOFI, 75008 Paris (FR)
(72) Inventeur: BARONI, Marco, F-75013 Paris (FR); BONO, Françoise, F-75013 Paris (FR); DELBARY-GOSSART, Sandrine, F-75013 Paris (FR); VERCESI, Valentina, F-75013 Paris (FR)
(74) Mandataire: Quellari, Mylène Audrey Séverine
(86) Numéro de dépôt international: PCT/FR2010/052685
(87) Numéro de publication internationale: WO 2011/080444

(56) Documents cités:
- WO-A1-00/51984
- WO-A1-03/104225
- DATABASE CHEMCATS [Online] 21 août 2009 (2009-08-21), "3-chloro-1-(5,6,7,8-tetrahydro-1,6-naphth yridin-6-yl)propan-1-one", XP002588482, Database accession no. 2096188619
- DATABASE CHEMCATS [Online] 21 août 2009 (2009-08-21), "2-chloro-1-(5,6,7,8-tetrahydro-1,6-naphth yridin-6-yl)ethan-1-one", XP002588494, Database accession no. 2096188618

## Description

La présente invention a pour objet des dérivés d' (hétérocycle-pipéridine condensée)-(pipérazinyl)-1-alcanone et d' (hétérocycle-pyrrolidine condensée)-(pipérazinyl)-1-alcanone, leur préparation et leur application en thérapeutique.

Les composés selon la présente invention présentent une affinité pour le récepteur p75^{NTR} des neurotrophines.

Les neurotrophines appartiennent à une famille de protéines ayant notamment pour effet biologique la survie et la différenciation cellulaires.

Le récepteur p75^{NTR} , récepteur de toutes les neurotrophines, est une glycoprotéine transmembranaire de la famille du récepteur du facteur de nécrose tumorale (TNF, de l'anglais Tumor Necrosis Factor) (W. J. Friedman et L. A. Greene, Exp. Cell. Res., 1999,253, 131-142). Le récepteur p75^{NTR} est exprimé dans plusieurs types cellulaires, et plusieurs fonctions biologiques lui sont attribuées : d'une part, la modulation de l'affinité des neurotrophines pour les récepteurs tyrosine kinase (trk) ; d'autre part, en l'absence de trk, une induction d'un signal de mort cellulaire par apoptose. Par ailleurs, les précurseurs des neurotrophines, les proneurotrophines sont capables de se fixer sur p75^{NTR} avec une haute affinité, et sont considérés comme de puissants inducteurs de l'apoptose dépendant de p75^{NTR} dans les neurones et certaines lignées cellulaires.

Au niveau du système nerveux central, de nombreux travaux montrent que l'apoptose intervient dans plusieurs pathologies comme la sclérose latérale amyotrophique, la sclérose en plaques, les maladies d'Alzheimer, de Parkinson et d'Huntington et les maladies à prion. P75^{NTR} est également connu pour être surexprimé dans différents types de maladies neurodégénératives comme la maladie d'Alzheimer et la sclérose latérale amyotrophique (ALS) (Longo F. M. et al., Curr. Alzheimer Res. 2007;4: 503-506; Lowry K.S. et al., Amyotroph. Lateral. Scler. Other. Motor. Neuron. Disord. 2001; 2:127-34).

Des résultats suggèrent que p75^{NTR} peut jouer un rôle prépondérant dans les mécanismes conduisant à la mort neuronale par apoptose post-ischémie (P. P. Roux et al., J. Neurosci., 1999, 19, 6887- 6896).

Des résultats (V. Della-Bianca et al., J. Biol. Chem., 2001, 276 : 38929-33), (S. Rabizadeh et al., Proc. Natl. Acad. Sci. USA, 1994,91, 10703-10706) supportent l'hypothèse selon laquelle p75^{NTR} jouerait un rôle important dans la mort neuronale induite par la protéine prion infectieuse (encéphalopathie spongiforme transmissible) ou par la protéine beta Amyloide (maladie d'Alzheimer).

Le récepteur p75^{NTR} est également associé au récepteur Nogo et impliqué dans la signalisation des effets inhibiteurs de ces protéines de la myéline vis-à-vis de la croissance axonale. De ce fait, le récepteur p75^{NTR} joue un rôle majeur dans la régulation de la plasticité neuronale et dans les interactions neurones-glie et représente ainsi une cible thérapeutique de choix pour promouvoir la régénération nerveuse.

Au-delà du système nerveux et des maladies neurodégénératives, il a été suggéré que p75^{NTR} pouvait jouer un rôle dans des maladies cardiovasculaires telles que l'athérosclérose et l'ischémie du myocarde (M. L. Bochaton-Pialat et al., Am. J. Pathol., 1995,146, 1-6 ; H. Perlman, Circulation, 1997,95, 981-987). Des travaux récents montrent une augmentation de l'expression de p75^{NTR} et des neurotrophines, et une apoptose massive dans des lésions d'athérosclérose.

Plusieurs études suggèrent également que p75^{NTR} est un médiateur de l'inflammation (Rihl M. et al., Ann. Rheum. Dis. 2005; 64(11):1542-9; Raychaudhuri S.P. et al., Prog. Brain. Res. 2004;146: 433-7, Tokuoka S. et al., Br. J. Pharmacol. 2001, 134: 1580-1586).

P75^{NTR} est également décrit pour jouer un rôle important dans la douleur inflammatoire. En effet, la lésion du nerf augmenterait de manière sélective l'expression et le transport axonal de p75^{NTR}, impliqué dans l"induction de la douleur neuropathique. De plus, l'utilisation d'anticorps spécifique de p75^{NTR} ou d'antisens oligodeoxynucleotide capable de bloquer l'activité du récepteur *in vivo* serait capable de réverser la douleur neuropathique (hyperalgésie par la chaleur et le froid et allodynie mécanique) induite chez le rat après lésion du nerf spinal L5 (Obata K. et al., J. Neurosci. 2006 ; 26: 11974-11986). Un anticorps neutralisant anti-p75^{NTR} réduit de manière considérable la douleur inflammatoire induite par l'injection d'adjuvant dans la voute plantaire chez la souris, ainsi que dans un modèle de crush du nerf sciatique chez la souris (Watanabe T. et al., J. Neurosci. Res. 2008 ; 86: 3566-357 ; Fukui Y. et al.. J Orthop Res. 2010; 28(3): 279-83).

L'expression de p75^{NTR} est aussi décrite dans la pancréatite chronique, avec une implication dans le processus apoptotique du pancréas exocrine et endocrine. (Zhu Z. et al., Dig. Dis. Sci. 2003; 48 (4): 717-25).

D'autres rapports ont également décrit l'importance de p75^{NTR} dans le développement de la fibrose hépatique (Kendall T. J. et al., Hepatology. 2009; 49 (3): 901-10).

P75^{NTR} joue également un rôle critique dans la biologie tumorale.

De nombreux composés sont connus pour interagir avec le système trkA/NGF/p75^{NTR} ou pour posséder une activité de type NGF (facteur de croissance neuronale, de l'anglais nerve growth factor). Ainsi la demande de brevet WO 00/59893 décrit des dérivés de pyrimidines substituées qui présentent une activité de type NGF et/ou qui augmentent l'activité du NGF sur les cellules PC12.

Le document WO00/51984 décrit des dérivés de 2-aryl indole qui présentent une activité antagoniste des tachykinines, en particulier des récepteurs de la neurokinine-1. On connait également les composés 3-chloro-1-(5,6,7,8-tetrahydro-1,6-naphthyridin-6-yl)propan-1-one et 3-chloro-1-(5,6,7,8-tetrahydro-1,6-naphthyridin-6-yl)ethan-1-one.

La présente invention a pour objet les composés répondant à la formule (I) : 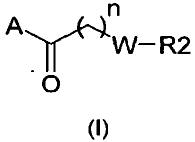 dans laquelle :
- A représente un groupe : 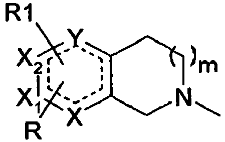
- n représente 1 ou 2 ;
- m représente 0 ou 1 ;
- Y représente un atome de carbone, d'azote, de souffre ou d'oxygène ou une liaison simple ou double ;
- X, X₁ et X₂ représentent un atome de carbone, d'azote, de souffre ou d'oxygène, étant entendu qu'au moins un des X, X₁, X₂ est différent d'un atome de carbone ;
- R et R1, situés sur l'une quelconque des positions disponibles, représentent indépendamment un atome d'hydrogène, un atome d'halogène, un groupe (C1-C4)alkyle, (C1-C4)alkoxy, un radical perfluoroalkyle, trifluorométhoxy, un cyano, un groupe COOH, COOalkyle, CONR5R6 ou NHCOR5 ;
   ou R1 représente un groupe choisi parmi : 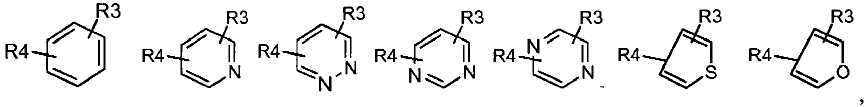 la définition de R restant inchangée;
- R3 et R4, situés sur l'une quelconque des positions disponibles, représentent indépendamment un atome d'hydrogène, un atome d'halogène, un groupe (C1-C4)alkyle, (C1-C4)alkoxy, un radical perfluoroalkyl, trifluorométhoxy, un cyano, un groupe COOH, COOalkyle, CONR5R6 ou NHCOR5 ;
- W- est un hétérocycle azoté choisi parmi: 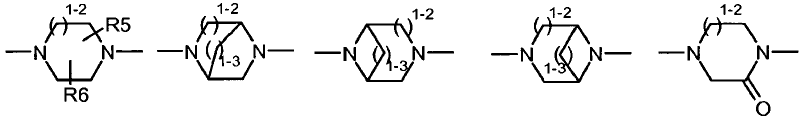
- 1-2 représente 1 ou 2 ;
- 1-3 représente 1, 2 ou 3 ;
- R2 représente un groupe de formule : 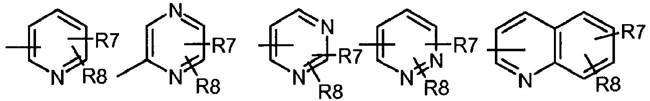
- R7 et R8, situés sur l'une quelconque des positions disponibles, représentent indépendamment un atome d'hydrogène, un atome d'halogène, un groupe (C1-C4)alkyle, (C1-C4)alkoxy, un radical trifluorométhyle, trifluorométhoxy, un cyano, un groupe COOH, COOalkyle, COOcycloalkyle, SOalkyle, SO₂alkyle; CONH2, CONR5R6 ou NHCOR5;
ou un des R7 et R8 représente un hétérocycle choisi parmi : 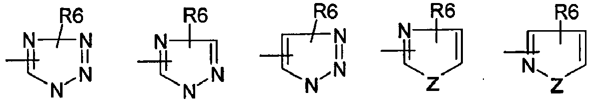 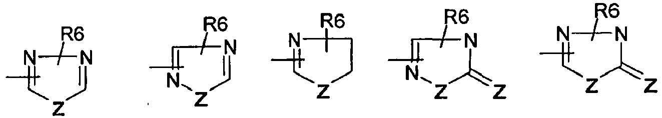
- Z représente un atome d'oxygène ou de souffre ;
- R5 et R6 représentent un hydrogène ou un groupe C1-C6 alkyle.

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.

Ces sels peuvent être préparés avec des acides pharmaceutiquement acceptables, mais les sels d'autres acides utiles, par exemple, pour la purification ou l'isolement des composés de formule (I) font également partie de l'invention.

Dans le cadre de la présente invention, on entend par:
- un atome d'halogène : un fluor, un chlore, un brome ou un iode ;
- un groupe alkyle : un groupe aliphatique saturé, linéaire, ramifié ou cyclique. A titre d'exemples, on peut citer un groupe C1-4 alkyle pouvant représenter un méthyle, éthyle, propyle, isopropyle, butyle, isobutyle, tertbutyle, cyclopropyle ou cyclobutyle ;
- un groupe fluoroalkyle : un groupe alkyle dont un ou plusieurs atomes d'hydrogène ont été substitués par un atome de fluor ;
- un groupe perfluoroalkyle : un groupe alkyle dont tous les atomes d'hydrogène ont été substitués par un atome de fluor, par exemple un groupe trifluoroalkyle comme le trifluorométhyle;
- un groupe alkoxy : un radical -O-alkyle où le groupe alkyle est tel que précédemment défini ;
- un groupe perfluoroalkoxy : un groupe alkoxy dont tous les atomes d'hydrogène ont été substitués par un atome de fluor, par exemple un groupe trifluoroalkoxy comme le trifluorométhoxy;
- un groupe cycloalkyle : un groupe alkyle cyclique. A titre d'exemples, on peut citer les groupes cyclopropyle, méthylcyclopropyle, cyclobutyle, cyclopentyle, cyclohexyle, etc.

Parmi les composés de formule (I) objets de l'invention, un autre groupe de composés est constitué par les composés de formule (I) dans laquelle :
- n représente 1; et/ou
- m représente 0 ou 1 ; et/ou
- Y représente un atome d'azote, ou une liaison simple ou double ; et/ou
- X, X₁ et X₂ représentent un atome de carbone, d'azote ou de souffre, étant entendu qu'au moins un des X, X₁, X₂ est différent d'un atome de carbone ;et/ou
- R et R1, situés sur l'une quelconque des positions disponibles, représentent indépendamment un atome d'hydrogène, un atome d'halogène ou un groupe (C1-C4) alkyle;
ou bien
R1 représente un groupe: 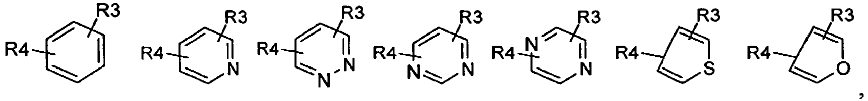 et R est un atome d'hydrogène; et/ou
- R3 et R4, situés sur l'une quelconque des positions disponibles, représentent un atome d'hydrogène, un atome d'halogène, un groupe (C1-C4) alkoxy, un radical perfluoroalkyle; et/ou
- -W- représente : 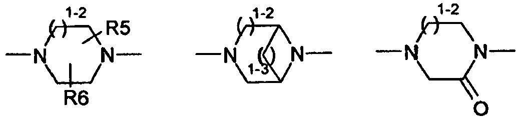
Ou bien 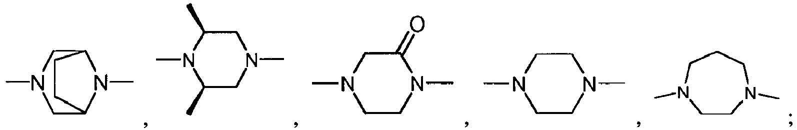 et/ou
- R2 représente : 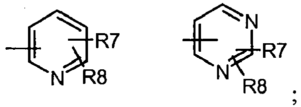 et/ou
- R7 et R8, situés sur l'une quelconque des positions disponibles, représentent indépendamment un atome d'hydrogène, un atome d'halogène, un groupe (C1-C4)alkyle, un radical trifluorométhyle, un groupe COOH, COOalkyle, SOalkyle, SO₂alkyle ;
ou bien
un des R7 et R8 représente un hétérocycle choisi parmi : 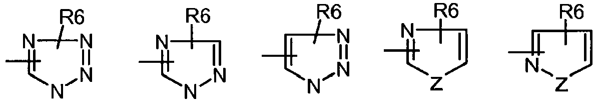 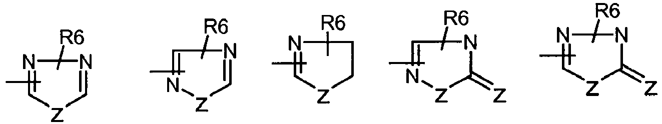 et/ou;
- R5 et R6 représentent un hydrogène ou un groupe méthyle ;
à l'état de base ou de sel d'addition à un acide.

Parmi les composés de formule (I) objets de l'invention, on peut notamment citer les composés suivants :
- Composé n°1 : 1-(6,7-Dihydro-4H-thieno[3,2-c]pyridin-5-yl)-2-[8-(5-fluoro-pyrimidin-2-yl)-3,8-diaza-bicyclo[3.2.1]oct-3-yl]-éthanone ;
- Composé n°2: 2-[8-(5-Fluoro-pyrimidin-2-yl)-3,8-diaza-bicyclo[3.2.1]oct-3-yl]-1-(2-phenyl-6,7-dihydro-4H-thiazolo[5,4-c]pyridin-5-yl)-éthanone ;
- Composé n°3 : 1-(2-Chloro-7,8-dihydro-5H-[1,6]naphthyridin-6-yl)-2-[8-(5-fluoro-pyrimidin-2-yl)-3,8-diaza-bicyclo[3.2.1]oct-3-yl]-éthanone ;
- Composé n°4: 2-[8-(5-Fluoro-pyrimidin-2-yl)-3,8-diaza-bicyclo[3.2.1]oct-3-yl]-1-(2-phényl-2,4,6,7-tétrahydro-pyrazolo[4,3-c]pyridin-5-yl)-éthanone ;
- Composé n°5: Methyl ester de l'acide 6-{3-[2-Oxo-2-(2-phényl-6,7-dihydro-4H-thiazolo[5,4-c]pyridin-5-yl)-ethyl]-3,8-diaza-bicyclo[3.2.1]oct-8-yl}-nicotinique ;
- Composé n°6 :Acide 6-{(3S,5R)-3,5-Dimethyl-4-[2-oxo-2-(2-phenyl-6,7-dihydro-4H-thiazolo[5,4-c]pyridin-5-yl)-éthyl]-piperazin-1-yl}-nicotinique;
- Composé n°7 :Acide 6-{3-[2-Oxo-2-(2-phényl-6,7-dihydro-4H-thiazolo[5,4-c]pyridin-5-yl)-éthyl]-3,8-diaza-bicyclo[3.2.1]oct-8-yl}-nicotinique;
- Composé n°8 :Acide 6-{(3S,5R)-3,5-Dimethyl-4-[2-oxo-2-(2-phényl-6,7-dihydro-4H-thiazolo[5,4-c]pyridin-5-yl)-éthyl]-pipérazin-1-yl}-nicotinique;
- Composé n°9: 2-[(2S,6R)-2,6-Dimethyl-4-(5-trifluoromethyl-pyridin-2-yl)-pipérazin-1-yl]-1-(2-phényl-6,7-dihydro-4H-thiazolo[5,4-c]pyridin-5-yl)-éthanone;
- Composé n°10: 2-[(2S,6R)-2,6-Dimethyl-4-(5-trifluoromethyl-pyridin-2-yl)-pipérazin -1-yl]-1-(2-phényl-2,4,6,7-tétrahydro-pyrazolo[4,3-c]pyridin-5-yl)-éthanone;
- Composé n°11: 4-[2-Oxo-2-(2-phényl-6,7-dihydro-4H-thiazolo[5,4-c]pyridin-5-yl)-éthyl]-1-(5-trifluorométhyl-pyridin-2-yl)-pipérazin-2-one;
- Composé n°12: 1-(2-Phenyl-6,7-dihydro-4H-thiazolo[5,4-c]pyridin-5-yl)-2-[4-(5-trifluorométhyl-pyridin-2-yl)-pipérazin-1-yl]-éthanone;
- Composé n°13: 1-(2-Phényl-2,4,6,7-tétrahydro-pyrazolo[4,3-c]pyridin-5-yl)-2-[4-(5-trifluorométhyl-pyridin-2-yl)-pipérazin-1-yl]-éthanone ;
- Composé n°14: 4-[2-Oxo-2-(2-phényl-2,4,6,7-tétrahydro-pyrazolo[4,3-c]pyridin-5-yl)-éthyl]-1-(5-trifluorométhyl-pyridin-2-yl)-pipérazin-2-one;
- Composé n°15 : 2-[(2S,6R)-4-(5-Fluoro-pyrimidin-2-yl)-2,6-diméthyl-pipérazin-1-yl]-1-(2-phényl-6,7-dihydro-4H-thiazolo[5,4-c]pyridin-5-yl)-éthanone;
- Composé n°16: 2-((2S,6R)-2,6-Diméthyl-4-pyrimidin-5-yl-pipérazin-1-yl)-1-(2-phényl-2,4,6,7-tétrahydro-pyrazolo[4,3-c]pyridin-5-yl)-éthanone;
- Composé n°17 : 2-[(2S,6R)-4-(5-Fluoro-pyrimidin-2-yl)-2,6-diméthyl-pipérazin-1-yl]-1-(2-phényl-2,4,6,7-tetrahydro-pyrazolo[4,3-c]pyridin-5-yl)-éthanone;
- Composé n°18 : 2-[(2S,6R)-2,6-Dimethyl-4-(6-trifluorométhyl-pyridin-3-yl)-pipérazin -1-yl]-1-(2-phényl-6,7-dihydro-4H-thiazolo[5,4-c]pyridin-5-yl)-éthanone;
- Composé n°19 : Methyl ester de l'acide 6-{3-[2-Oxo-2-(2-phényl-2,4,6,7-tétrahydro-pyrazolo[4,3-c]pyridin-5-yl)-éthyl]-3,8-diaza-bicyclo[3.2.1]oct-8-yl}-nicotinique ;
- Composé n°20 : 2-[(2S,6R)-2,6-Diméthyl-4-(6-trifluoromethyl-pyridin-3-yl)-pipérazin -1-yl]-1-(2-phenyl-2,4,6,7-tétrahydro-pyrazolo[4,3-c]pyridin-5-yl)-éthanone;
- Composé n°21 : Acide 6-{(3S,5R)-3,5-Diméthyl-4-[2-oxo-2-(2-phényl-2,4,6,7-tétrahydro-pyrazolo[4,3-c]pyridin-5-yl)-éthyl]-pipérazin-1-yl}-nicotinique;
- Composé n°22: 2-((2S,6R)-2,6-Dimethyl-4-pyrimidin-5-yl-pipérazin-1-yl)-1-(2-phényl-6,7-dihydro-4H-thiazolo[5,4-c]pyridin-5-yl)-éthanone;
- Composé n°23 :Methyl ester de l'acide 6-{(3S,5R)-3,5-Diméthyl-4-[2-oxo-2-(2-phényl-2,4,6,7-tétrahydro-pyrazolo[4,3-c]pyridin-5-yl)-ethyl]-pipérazin-1-yl}-nicotinique;
- Composé n°24: Acide 6-{3-[2-Oxo-2-(2-phenyl-2,4,6,7-tetrahydro-pyrazolo[4,3-c]pyridin-5-yl)-ethyl]-3,8-diaza-bicyclo[3.2.1]oct-8-yl}nicotinique;
- Composé n°25 :Methyl ester de l'acide 6-{2-Oxo-4-[2-oxo-2-(2-phenyl-6,7-dihydro-4H-thiazolo[5,4-c]pyridin-5-yl)-ethyl]-piperazin-1-yl}-nicotinique;
- Composé n°26 2-[8-(5-Fluoro-pyrimidin-2-yl)-3,8-diaza-bicyclo[3.2.1]oct-3-yl]-1-(1-phényl-1,4,6,7-tétrahydro-pyrazolo[4,3-c]pyridin-5-yl)-éthanone ;
- Composé n°27: 1-(2-Phényl-2,4,6,7-tétrahydro-pyrazolo[4,3-c]pyridin-5-yl)-2-(4-pyridin-3-yl-[1,4]diazepan-1-yl)-éthanone ;
- Composé n°28: 1-(2-Phényl-2,4,6,7-tétrahydro-pyrazolo[4,3-c]pyridin-5-yl)-2-(8-pyridin-3-yl-3,8-diaza-bicyclo[3.2.1]oct-3-yl)-éthanone ;
- Composé n°29: 1-(2-Phényl-2,4,6,7-tétrahydro-pyrazolo[4,3-c]pyridin-5-yl)-2-[8-(5-trifluorométhyl-pyridin-2-yl)-3,8-diaza-bicyclo[3.2.1]oct-3-yl]-éthanone ;
- Composé n°30: 1-(2-Phényl-6,7-dihydro-4H-thiazolo[5,4-c]pyridin-5-yl)-2-(4-pyridin -3-yl-[1,4]diazepan-1-yl)-éthanone ;
- Composé n°31 : 1-(2-Phényl-2,4,6,7-tétrahydro-pyrazolo[4,3-c]pyridin-5-yl)-2-(8-pyrimidin-5-yl-3,8-diaza-bicyclo[3.2.1]oct-3-yl)-éthanone ;
- Composé n°32 : 2-[8-(5-Fluoro-pyrimidin-2-yl)-3,8-diaza-bicyclo[3.2.1]oct-3-yl]-1-(2-méthyl-2,4,6,7-tétrahydro-pyrazolo[4,3-c]pyridin-5-yl)-éthanone ;
- Composé n°33 : Méthyl ester de l'acide 6-{3-[2-Oxo-2-(2-phényl-4,6-dihydro-pyrrolo[3,4-d]thiazol-5-yl)-éthyl]-3,8-diaza-bicyclo[3.2.1]oct-8-yl}-nicotinique ;
- Composé n°34: 4-{2-[2-(4-Méthoxy-phényl)-2,4,6,7-tétrahydro-pyrazolo[4,3-c]pyridin-5-yl]-2-oxo-éthyl}-1-(5-trifluorométhyl-pyridin-2-yl)-pipérazin-2-one ;
- Composé n°35 : 4-{2-[2-(4-Fluoro-phényl)-2,4,6,7-tétrahydro-pyrazolo[4,3-c]pyridin-5-yl]-2-oxo-éthyl}-1-(5-trifluorométhyl-pyridin-2-yl)-pipérazin-2-one ;
- Composé n°36 : 2-[8-(5-Fluoro-pyrimidin-2-yl)-3,8-diaza-bicyclo[3.2.1]oct-3-yl]-1-(2-méthyl-6,7-dihydro-4H-thiazolo[5,4-c]pyridin-5-yl)-éthanone ;
- Composé n°37 : Méthyl ester de l'acide 6-(3-{2-[2-(4-Méthoxy-phényl)-6,7-dihydro-4H-thiazolo[5,4-c]pyridin-5-yl]-2-oxo-éthyl}-3,8-diaza-bicyclo[3.2.1]oct-8-yl)-nicotinique ;
- Composé n°38 : Méthyl ester de l'acide 6-(3-{2-[2-(4-Fluoro-phényi)-6,7-dihydro-4H -thiazolo[5,4-c]pyridin-5-yl]-2-oxo-éthyl}-3,8-diaza-bicyclo[3.2.1]oct-8-yl)-nicotinique ;
- Composé n°39 : Méthyl ester de l'acide 6-(3-{2-Oxo-2-[2-(5-trifluorométhyl-pyridin-2-yl)-2,4,6,7-tétrahydro-pyrazolo[4,3-c]pyridin-5-yl]-éthyl}-3,8-diaza-bicyclo[3.2.1] oct -8-yl)-nicotinique ;
- Composé n°40: 2-[8-(5-Fluoro-pyrimidin-2-yl)-3,8-diaza-bicyclo[3.2.1]oct-3-yl]-1-[2-(5-trifluorométhyl-pyridin-2-yl)-2,4,6,7-tétrahydro-pyrazolo[4,3-c]pyridin-5-yl]-éthanone ;
- Composé n°41 : 4-[2-Oxo-2-(2-phényl-7,8-dihydro-5H-pyrido[4,3-d]pyrimidin-6-yl)-éthyl]-1-(5-trifluorométhyl-pyridin-2-yl)-pipérazin-2-one ;
- Composé n°42: 4-[2-Oxo-2-(2-thiophén-3-yl-6,7-dihydro-4H-thiazolo[5,4-c]pyridin-5-yl)-éthyl]-1-(5-trifluorométhyl-pyridin-2-yl)-pipérazin-2-one ;
- Composé n°43: 2-[8-(5-Fluoro-pyrimidin-2-yl)-3,8-diaza-bicyclo[3.2.1]oct-3-yl]-1-[2-(4-methoxy-phenyl)-2,4,6,7-tetrahydro-pyrazolo[4,3-c]pyridin-5-yl]-ethanone;
- Composé n°44: acide 6-{3-[2-Oxo-2-(2-thiophen-3-yl-6,7-dihydro-4H-thiazolo[5,4-c]pyridin-5-yl)-ethyl]-3,8-diaza-bicyclo[3.2.1]oct-8-yl}-nicotinique;
- Composé n°45: 2-[8-(5-Fluoro-pyrimidin-2-yl)-3,8-diaza-bicyclo[3.2.1]oct-3-yl]-1-[1-(2,2,2-trifluoro-ethyl)-4,5,6,7-tetrahydro-1 H-indazol-5-yl]-ethanone;
- Composé n°46: 2-[8-(5-Fluoro-pyrimidin-2-yl)-3,8-diaza-bicyclo[3.2.1]oct-3-yl]-1-[1-(4-methoxy-phenyl)-1,4,6,7-tetrahydro-pyrazolo[4,3-c]pyridin-5-yl]-ethanone;
- Composé n°47: 2-[8-(5-Fluoro-pyrimidin-2-yl)-3,8-diaza-bicyclo[3.2.1]oct-3-yl]-1-(2,4,6,7-tetrahydro-pyrazolo[4,3-c]pyridin-5-yl)-ethanone;
- Composé n°48: méthyl ester de l'acide 6-{3-[2-Oxo-2-(1-phenyl-1,4,6,7-tetrahydro-pyrazolo[4,3-c]pyridin-5-yl)-ethyl]-3,8-diaza-bicyclo[3.2.1]oct-8-yl}-nicotinique;
- Composé n°49: acide 6-{3-[2-Oxo-2-(1-phenyl-1,4,6,7-tetrahydro-pyrazolo[4,3-c]pyridin-5-yl)-ethyl]-3,8-diaza-bicyclo[3.2.1]oct-8-yl}-nicotinique;
- Composé n°50: 1-(1-tert-Butyl-1,4,6,7-tetrahydro-pyrazolo[4,3-c]pyridin-5-yl)-2-[8-(5-fluoro-pyrimidin-2-yl)-3,8-diaza-bicyclo[3.2.1]oct-3-yl]-ethanone;
- Composé n°51: 1-[1-(4-Fluoro-phenyl)-1,4,6,7-tetrahydro-pyrazolo[4,3-c]pyridin-5-yl]-2-[8-(5-fluoro-pyrimidin-2-yl)-3,8-diaza-bicyclo[3.2.1]oct-3-yl]-ethanone;
- Composé n°52: 6-{(2R,5S)-2,5-Dimethyl-4-[2-oxo-2-(2-phenyl-2,4,6,7-tetrahydro-pyrazolo[4,3-c]pyridin-5-yl)-ethyl]-piperazin-1-yl}-nicotinonitrile;
- Composé n°53: acide 6-{(2R,5S)-2,5-Dimethyl-4-[2-oxo-2-(2-phenyl-2,4,6,7-tetrahydro-pyrazolo[4,3-c]pyridin-5-yl)-ethyl]-piperazin-1-yl}-nicotinique;
- Composé n°54: 2-[8-(5-Fluoro-pyrimidin-2-yl)-3,8-diaza-bicyclo[3.2.1]oct-3-yl]-1-(2-phenyl-4,7-dihydro-5H-furo[2,3-c]pyridin-6-yl)-ethanone;
- Composé n°55: 2-[8-(5-Fluoro-pyrimidin-2-yl)-3,8-diaza-bicyclo[3.2.1]oct-3-yl]-1-(2-phenyl-1,4,5,7-tetrahydro-pyrrolo[2,3-c]pyridin-6-yl)-ethanone;
- Composé n°56: méthyl ester de l'acide 6-{8-[2-Oxo-2-(2-phenyl-6,7-dihydro-4H-thiazolo[5,4-c]pyridin-5-yl)-ethyl]-3,8-diaza-bicyclo[3.2.1]oct-3-yl}-nicotinique;
- Composé n°57: méthyl ester de l'acide 6-{8-[2-Oxo-2-(2-phenyl-2,4,6,7-tetrahydro-pyrazolo[4,3-c]pyridin-5-yl)-ethyl]-3,8-diaza-bicyclo[3.2.1]oct-3-yl}-nicotinique;
- Composé n°58: 2-[8-(5-Fluoro-pyrimidin-2-yl)-3,8-diaza-bicyclo[3.2.1]oct-3-yl]-1-(2-phenyl-6,7-dihydro-4H-oxazolo[4,5-c]pyridin-5-yl)-ethanone;
- Composé n°59: 6-{(2R,5S)-2,5-Dimethyl-4-[2-oxo-2-(2-phenyl-6,7-dihydro-4H-thiazolo[5,4-c]pyridin-5-yl)-ethyl]-piperazin-1-ylj-nicotinonitrile;
- Composé n°60: 2-{8-[5-(5-Methyl-[1,2,4]oxadiazol-3-yl)-pyridin-2-yl]-3,8-diaza-bicyclo[3.2.1]oct-3-yl}-1-(2-phenyl-6,7-dihydro-4H-thiazolo[5,4-c]pyridin-5-yl)-ethanone;
- Composé n°61: acide 6-{8-[2-Oxo-2-(2-phenyl-2,4,6,7-tetrahydro-pyrazolo[4,3-c] pyridin-5-yl)-ethyl]-3,8-diaza-bicyclo[3.2.1]oct-3-yl}-nicotinique;
- Composé n°62: acide 6-{8-[2-Oxo-2-(2-phenyl-6,7-dihydro-4H-thiazolo[5,4-c] pyridin-5-yl)-ethyl]-3,8-diaza-bicyclo[3.2.1]oct-3-yl}-nicotinique;
- Composé n°63: acide 6-{(2R,5S)-2,5-Dimethyl-4-[2-oxo-2-(2-phenyl-6,7-dihydro-4H-thiazolo[5,4-c]pyridin-5-yl)-ethyl]-piperazin-1-yl}-nicotinique;
- Composé n°64: 2-[8-(5-Fluoro-pyrimidin-2-yl)-3,8-diaza-bicyclo[3.2.1]oct-3-yl]-1-(2-pyridin-4-yl-6,7-dihydro-4H-thiazolo[5,4-c]pyridin-5-yl)-ethanone;
- Composé n°65: 4-{2-Oxo-2-[2-(5-trifluoromethyl-pyridin-2-yl)-2,4,6,7-tetrahydro-pyrazolo[4,3-c]pyridin-5-yl]-ethyl}-1-(5-trifluoromethyl-pyridin-2-yl)-piperazin-2-one;
- Composé n°66: 4-{2-Oxo-2-[1-(2,2,2-trifluoro-ethyl)-1,4,6,7-tetrahydro-pyrazolo [4,3-c]pyridin-5-yl]-ethyl}-1-(5-trifluoromethyl-pyridin-2-yl)-piperazin-2-one;
- Composé n°67: 2-[8-(5-Fluoro-pyrimidin-2-yl)-3,8-diaza-bicyclo[3.2.1]oct-3-yl]-1-(2-phenyl-7,8-dihydro-5H-pyrido[4,3-d]pyrimidin-6-yl)-ethanone;
- Composé n°68: 3-(6-{3-[2-Oxo-2-(2-phenyl-2,4,6,7-tetrahydro-pyrazolo[4,3-c] pyridin-5-yl)-ethyl]-3,8-diaza-bicyclo[3.2.1]oct-8-yl}-pyridin-3-yl)-4H-[1,2,4] oxadiazol -5-one;
- Composé n°69: 2-[8-(5-Fluoro-pyrimidin-2-yl)-3,8-diaza-bicyclo[3.2.1]oct-3-yl]-1-(2-phenyl-3,4,6,7-tetrahydro-imidazo[4,5-c]pyridin-5-yl)-ethanone;
- Composé n°70: 3-(6-{3-[2-Oxo-2-(2-phenyl-6,7-dihydro-4H-thiazolo[5,4-c]pyridin-5-yl)-ethyl]-3,8-diaza-bicyclo[3.2.1]oct-8-yl}-pyridin-3-yl)-4H-[1,2,4]oxadiazol-5-one;
- Composé n°71: 2-[8-(5-Fluoro-pyrimidin-2-yl)-3,8-diaza-bicyclo[3.2.1]oct-3-yl]-1-(2-phenyl-6,7-dihydro-4H-thiazolo[4,5-c]pyridin-5-yl)-ethanone;
- Composé n°72: acide 2-{(3S,5R)-3,5-Dimethyl-4-[2-oxo-2-(2-phenyl-6,7-dihydro-4H-thiazolo[5,4-c]pyridin-5-yl)-ethyl]-piperazin-1-yl}-pyrimidine-5-carboxylique;
- Composé n°73: 2-[8-(5-Fluoro-pyrimidin-2-yl)-3,8-diaza-bicyclo[3.2.1]oct-3-yl]-1-(2-pyridin-3-yl-6,7-dihydro-4H-thiazolo[5,4-c]pyridin-5-yl)-ethanone;
- Composé n°74: 1-(2-Phenyl-6,7-dihydro-4H-thiazolo[5,4-c]pyridin-5-yl)-2-{8-[5-(1H-tetrazol-5-yl)-pyridin-2-yl]-3,8-diaza-bicyclo[3.2.1]oct-3-yl}-ethanone;
- Composé n°75: acide 2-{(3S,5R)-3,5-Dimethyl-4-[2-oxo-2-(2-phenyl-2,4,6,7-tetrahydro-pyrazolo[4,3-c]pyridin-5-yl)-ethyl]-piperazin-1-yl}-pyrimidine-5-carboxylique;
- Composé n°76: 6-(3-{2-[2-(4-Fluoro-phenyl)-6,7-dihydro-4H-thiazolo[5,4-c]pyridin-5-yl]-2-oxo-ethyl}-3,8-diaza-bicyclo[3.2.1]oct-8-yl)-nicotinonitrile;
- Composé n°77: 6-{3-[2-Oxo-2-(2-phenyl-2,4,6,7-tetrahydro-pyrazolo[4,3-c]pyridin-5-yl)-ethyl]-3,8-diaza-bicyclo[3.2.1]oct-8-yl}-nicotinonitrile;
- Composé n°78: 4-[2-Oxo-2-(2-phenyl-3,4,6,7-tetrahydro-imidazo[4,5-c]pyridin-5-yl)-ethyl]-1-(5-trifluoromethyl-pyridin-2-yl)-piperazin-2-one;
- Composé n°79: éthyl ester de l'acide 6-{(3S,5R)-3,5-Dimethyl-4-[2-oxo-2-(2-phenyl-2,4,6,7-tetrahydro-pyrazolo[4,3-c]pyridin-5-yl)-ethyl]-piperazin-1-yl}-nicotinique;
- Composé n°80: 1-[2-(4-Fluoro-phenyl)-6,7-dihydro-4H-thiazolo[5,4-c]pyridin-5-yl]-2-[8-(5-fluoro-pyrimidin-2-yl)-3,8-diaza-bicyclo[3.2.1]oct-3-yl]-ethanone;
- Composé n°81: 2-[8-(5-Fluoro-pyrimidin-2-yl)-3,8-diaza-bicyclo[3.2.1]oct-3-yl]-1-[2-(4-methoxy-phenyl)-6,7-dihydro-4H-thiazolo[5,4-c]pyridin-5-yl]-ethanone;
- Composé n°82: 4-[2-Oxo-2-(2-phenyl-6,7-dihydro-4H-thiazolo[4,5-c]pyridin-5-yl)-ethyl]-1-(5-trifluoromethyl-pyridin-2-yl)-piperazin-2-one;
- Composé n°83: 4-[2-Oxo-2-(2-phenyl-6,7-dihydro-4H-oxazolo[4,5-c]pyridin-5-yl)-ethyl]-1-(5-trifluoromethyl-pyridin-2-yl)-piperazin-2-one;
- Composé n°84: 4-[2-Oxo-2-(2-pyridin-3-yl-6,7-dihydro-4H-thiazolo[5,4-c]pyridin-5-yl)-ethyl]-1-(5-trifluoromethyl-pyridin-2-yl)-piperazin-2-one;
- Composé n°85: 2-{8-[5-(2-Methyl-2H-tetrazol-5-yl)-pyridin-2-yl]-3,8-diaza-bicyclo [3.2.1]oct-3-yl}-1-(2-phenyl-2,4,6,7-tetrahydro-pyrazolo[4,3-c]pyridin-5-yl)-ethanone;
- Composé n°86: 2-{(2S,6R)-2,6-Dimethyl-4-[5-(1-methyl-1H-tetrazol-5-yl)-pyridin-2-yl]-piperazin-1-yl}-1-(2-phenyl-2,4,6,7-tetrahydro-pyrazolo[4,3-c]pyridin-5-yl)-ethanone;
- Composé n°87: 2-{(2S,6R)-2,6-Dimethyl-4-[5-(2-methyl-2H-tetrazol-5-yl)-pyridin-2-yl]-piperazin-1-yl}-1-(2-phenyl-2,4,6,7-tetrahydro-pyrazolo[4,3-c]pyridin-5-yl)-ethanone;
- Composé n°88: 2-[8-(5-Fluoro-pyrimidin-2-yl)-3,8-diaza-bicyclo[3.2.1]oct-3-yl]-1-[1-(4-trifluoromethyl-phenyl)-1,4,6,7-tetrahydro-pyrazolo[4,3-c]pyridin-5-yl]-ethanone;
- Composé n°89: 2-{(2S,6R)-2,6-Dimethyl-4-[5-(5-methyl-[1,2,4]oxadiazol-3-yl)-pyridin-2-yl]-piperazin-1-yl}-1-(2-phenyl-2,4,6,7-tetrahydro-pyrazolo[4,3-c]pyridin-5-yl)-ethanone;
- Composé n°90: 2-[(2S,6R)-2,6-Dimethyl-4-(5-[1,3,4]oxadiazol-2-yl-pyridin-2-yl)-piperazin-1-yl]-1-(2-phenyl-2,4,6,7-tetrahydro-pyrazolo[4,3-c]pyridin-5-yl)-ethanone;
- Composé n°91: 2-{(2S,6R)-2,6-Dimethyl-4-[5-(3-methyl-[1,2,4]oxadiazol-5-yl)-pyridin-2-yl]-piperazin-1-yl}-1-(2-phenyl-2,4,6,7-tetrahydro-pyrazolo[4,3-c]pyridin-5-yl)-ethanone;
- Composé n°92: 6-{(3S,5R)-4-[2-(1-tert-Butyl-1,4,6,7-tetrahydro-pyrazolo[4,3-c]pyridin-5-yl)-2-oxo-ethyl]-3,5-dimethyl-piperazin-1-yl}-nicotinic acid methyl ester;
- Composé n°93: acide 6-{(3S,5R)-4-[2-(1-tert-Butyl-1,4,6,7-tetrahydro-pyrazolo[4,3-c]pyridin-5-yl)-2-oxo-ethyl]-3,5-dimethyl-piperazin-1-yl}-nicotinique;
- Composé n°94: acide 6-{3-[2-(1-tert-Butyl-1,4,6,7-tetrahydro-pyrazolo[4,3-c]pyridin-5-yl)-2-oxo-ethyl]-3,8-diaza-bicyclo[3.2.1]oct-8-yl}-nicotinique;
- Composé n°95: 2-[8-(5-Fluoro-pyrimidin-2-yl)-3,8-diaza-bicyclo[3.2.1]oct-3-yl]-1-(2-phenyl-6,7-dihydro-4H-oxazolo[5,4-c]pyridin-5-yl)-ethanone;
- Composé n°96: 4-[2-Oxo-2-(2-phenyl-6,7-dihydro-4H-oxazolo[5,4-c]pyridin-5-yl)-ethyl]-1-(5-trifluoromethyl-pyridin-2-yl)-piperazin-2-one;
- Composé n°97: 2-[(2S,6R)-2,6-Dimethyl-4-(5-trifluoromethyl-pyridin-2-yl)-piperazin-1-yl]-1-(2-phenyl-4,7-dihydro-5H-furo[2,3-c]pyridin-6-yl)-ethanone;
- Composé n°98: isopropyl ester de l'acide 6-{(3S,5R)-3,5-Dimethyl-4-[2-oxo-2-(2-phenyl-2,4,6,7-tetrahydro-pyrazolo[4,3-c]pyridin-5-yl)-ethyl]-piperazin-1-yl}-nicotinique;
- Composé n°99: 1-(2-Methyl-2,4,6,7-tetrahydro-pyrazolo[4,3-c]pyridin-5-yl)-2-[8-(5-trifluoromethyl-pyridin-2-yl)-3,8-diaza-bicyclo[3.2.1]oct-3-yl]-ethanone;
- Composé n°100: 2-[(2S,6R)-2,6-Dimethyl-4-(5-trifluoromethyl-pyridin-2-yl)-piperazin-1-yl]-1-(2-methyl-2,4,6,7-tetrahydro-pyrazolo[4,3-c]pyridin-5-yl)-ethanone;
- Composé n°101: 1-(2-Phenyl-4,7-dihydro-5H-furo[2,3-c]pyridin-6-yl)-2-[8-(5-trifluoromethyl-pyridin-2-yl)-3,8-diaza-bicyclo[3.2.1]oct-3-yl]-ethanone;
- Composé n°102: acide 6-(3-{2-[2-(4-Fluoro-phenyl)-6,7-dihydro-4H-thiazolo[5,4-c] pyridin-5-yl]-2-oxo-ethyl}-3,8-diaza-bicyclo[3.2.1]oct-8-yl)-nicotinique;
- Composé n°103: 2-[(2S,6R)-2,6-Dimethyl-4-(5-trifluoromethyl-pyridin-2-yl)-piperazin-1-yl]-1-[2-(5-trifluoromethyl-pyridin-2-yl)-2,4,6,7-tetrahydro-pyrazolo[4,3-c] pyridin-5-yl]-ethanone;
- Composé n°104: 2-[8-(5-Trifluoromethyl-pyridin-2-yl)-3,8-diaza-bicyclo[3.2.1]oct-3-yl]-1-[2-(5-trifluoromethyl-pyridin-2-yl)-2,4,6,7-tetrahydro-pyrazolo[4,3-c]pyridin-5-yl]-ethanone;
- Composé n°105: acide 6-{(3S,5R)-4-[2-(2-tert-Butyl-2,4,6,7-tetrahydro-pyrazolo [4,3-c]pyridin-5-yl)-2-oxo-ethyl]-3,5-dimethyl-piperazin-1-yl}-nicotinique;
- Composé n°106: 2-(8-Pyridin-3-yl-3,8-diaza-bicyclo[3.2.1]oct-3-yl)-1-[2-(5-trifluoromethyl-pyridin-2-yl)-2,4,6,7-tetrahydro-pyrazolo[4,3-c]pyridin-5-yl]-ethanone;
- Composé n°107: 2-[5-(6-Trifluoromethyl-pyridazin-3-yl)-2,5-diaza-bicyclo[2.2.1] hept-2-yl]-1-[2-(5-trifluoromethyl-pyridin-2-yl)-2,4,6,7-tetrahydro-pyrazolo[4,3-c] pyridin-5-yl]-ethanone;
- Composé n°108: 2-(6'-Chloro-2,3,5,6-tetrahydro-[1,2']bipyrazinyl-4-yl)-1-[2-(5-trifluoromethyl-pyridin-2-yl)-2,4,6,7-tetrahydro-pyrazolo[4,3-c]pyridin-5-yl]-ethanone;
- Composé n°109: 1-(2-Phenyl-3,4,6,7-tetrahydro-imidazo[4,5-c]pyridin-5-yl)-2-[8-(5-trifluoromethyl-pyridin-2-yl)-3,8-diaza-bicyclo[3.2.1]oct-3-yl]-ethanone;
- Composé n°110: 2-[(2S,6R)-2,6-Dimethyl-4-(5-trifluoromethyl-pyridin-2-yl)-piperazin-1-yl]-1-(2-phenyl-3,4,6,7-tetrahydro-imidazo[4,5-c]pyridin-5-yl)-ethanone;
- Composé n°111: 2-((3S,5R)-3,5-Dimethyl-2,3,5,6-tetrahydro-[1,2']bipyrazinyl-4-yl)-1-[2-(5-trifluoromethyl-pyridin-2-yl)-2,4,6,7-tetrahydro-pyrazolo[4,3-c]pyridin-5-yl]-ethanone;
- Composé n°112: 2-[(2S,6R)-4-(5-Chloro-pyridin-2-yl)-2,6-dimethyl-piperazin-1-yl]-1-[2-(5-trifluoromethyl-pyridin-2-yl)-2,4,6,7-tetrahydro-pyrazolo[4,3-c]pyridin-5-yl]-ethanone;
- Composé n°113: 2-[4-(7-Chloro-quinolin-4-yl)-piperazin-1-yl]-1-(2-phenyl-2,4,6,7-tetrahydro-pyrazolo[4,3-c]pyridin-5-yl)-ethanone;
- Composé n°114: 2-[4-(6-Chloro-pyridin-2-yl)-piperazin-1-yl]-1-(2-phenyl-2,4,6,7-tetrahydro-pyrazolo[4,3-c]pyridin-5-yl)-ethanone;
- Composé n°115: 1-(2-Pyridin-4-yl-6,7-dihydro-4H-thiazolo[5,4-c]pyridin-5-yl)-2-[8-(5-trifluoromethyl-pyridin-2-yl)-3,8-diaza-bicyclo[3.2.1]oct-3-yl]-ethanone;
- Composé n°116: acide 6-{(3S,5R)-3,5-Dimethyl-4-[2-oxo-2-(2-phenyl-3,4,6,7-tetrahydro-imidazo[4,5-c]pyridin-5-yl)-ethyl]-piperazin-1-yl}-nicotinique;
- Composé n 117: 1-(2-Pyridin-2-yl-2,4,6,7-tetrahydro-pyrazolo[4,3-c]pyridin-5-yl)-2-[8-(5-trifluoromethyl-pyridin-2-yl)-3,8-diaza-bicyclo[3.2.1]oct-3-yl]-ethanone;
- Composé n°118: 2-[(2S,6R)-2,6-Dimethyl-4-(5-trifluoromethyl-pyridin-2-yl)-piperazin-1-yl]-1-(2-pyridin-2-yl-2,4,6,7-tetrahydro-pyrazolo[4,3-c]pyridin-5-yl)-ethanone;
- Composé n°119: 4-[2-Oxo-2-(2-pyridin-2-yl-2,4,6,7-tetrahydro-pyrazolo[4,3-c] pyridin-5-yl)-ethyl]-1-(5-trifluoromethyl-pyridin-2-yl)-piperazin-2-one;
- Composé n°120: 4-{2-[2-(4-Fluoro-phenyl)-3,4,6,7-tetrahydro-imidazo[4,5-c]pyridin-5-yl]-2-oxo-ethyl}-1-(5-trifluoromethyl-pyridin-2-yl)-piperazin-2-one;
- Composé n°121: 2-(8-Pyridin-3-yl-3,8-diaza-bicyclo[3.2.1]oct-3-yl)-1-(2-pyridin-2-yl-2,4,6,7-tetrahydro-pyrazolo[4,3-c]pyridin-5-yl)-ethanone;
- Composé n° 122: 1-[2-(2,2,2-Trifluoro-ethyl)-2,4,6,7-tetrahydro-pyrazolo[4,3-c]pyridin-5-yl]-2-[8-(5-trifluoromethyl-pyridin-2-yl)-3,8-diaza-bicyclo[3.2.1]oct-3-yl]-ethanone;
- Composé n° 123: 1-[2-(4-Fluoro-phenyl)-3,4,6,7-tetrahydro-imidazo[4,5-c]pyridin-5-yl]-2-[8-(5-trifluoromethyl-pyridin-2-yl)-3,8-diaza-bicyclo[3.2.1]oct-3-yl]-ethanone;
- Composé n°124: 2-[(2S,6R)-2,6-Dimethyl-4-(5-trifluoromethyl-pyridin-2-yl)-piperazin-1-yl]-1-[2-(4-fluoro-phenyl)-3,4,6,7-tetrahydro-imidazo[4,5-c]pyridin-5-yl]-ethanone;
- Composé n°125: 4-{2-Oxo-2-[2-(2,2,2-trifluoro-ethyl)-2,4,6,7-tetrahydro-pyrazolo [4,3-c]pyridin-5-yl]-ethyl}-1-(5-trifluoromethyl-pyridin-2-yl)-piperazin-2-one;
- Composé n°126: 2-[(2S,6R)-2,6-Dimethyl-4-(5-trifluoromethyl-pyridin-2-yl)-piperazin-1-yl]-1-[2-(2,2,2-trifluoro-ethyl)-2,4,6,7-tetrahydro-pyrazolo[4,3-c]pyridin-5-yl]-ethanone;
- Composé n°127: 1-(2-Phenyl-2,4,6,7-tetrahydro-pyrazolo[4,3-c]pyridin-5-yl)-2-[5-(5-trifluoromethyl-pyridin-2-yl)-2,5-diaza-bicyclo[2.2.1]hept-2-yl]-ethanone;
- Composé n°128: cyclobutyl ester de l'acide 6-{3-[2-Oxo-2-(2-phenyl-2,4,6,7-tetrahydro-pyrazolo[4,3-c]pyridin-5-yl)-ethyl]-3,8-diaza-bicyclo[3.2.1]oct-8-yl}-nicotinique;
- Composé n°129: 2-((2S,6R)-2,6-Dimethyl-4-quinolin-2-yl-piperazin-1-yl)-1-(2-phenyl-2,4,6,7-tetrahydro-pyrazolo[4,3-c]pyridin-5-yl)-ethanone;
- Composé n°130: éthyl ester de l'acide 6-{(3S,5R)-3,5-Diméthyl-4-[2-oxo-2-(2-phényl-2,4,6,7-tétrahydro-pyrazolo[4,3-c]pyridin-5-yl)-ethyl]-pipérazin-1-yl}-nicotinique;
- Composé n°131: 2-[(2S,6R)-4-(5-Methanesulfonyl-pyridin-2-yl)-2,6-dimethyl-piperazin-1-yl]-1-(2-phenyl-2,4,6,7-tetrahydro-pyrazolo[4,3-c]pyridin-5-yl)-ethanone;
- Composé n°132: 2-[(2S,6R)-4-(5-Fluoro-pyrimidin-2-yl)-2,6-dimethyl-piperazin-1-yl]-1-[2-(4-methoxy-phenyl)-6,7-dihydro-4H-thiazolo[5,4-c]pyridin-5-yl]-ethanone;
- Composé n°133: 1-[2-(4-Methoxy-phenyl)-6,7-dihydro-4H-thiazolo[5,4-c]pyridin-5-yl]-2-[5-(5-trifluoromethyl-pyridin-2-yl)-2,5-diaza-bicyclo[2.2.1]hept-2-yl]-ethanone;
- Composé n°134: 1-(2-Phenyl-6,7-dihydro-4H-thiazolo[5,4-c]pyridin-5-yl)-2-{4-[5-(2H-pyrazol-3-yl)-pyridin-2-yl]-piperazin-1-yl}-ethanone;
- Composé n°135: 2-[(2S,6R)-2,6-Dimethyl-4-(5-thiazol-2-yl-pyridin-2-yl)-piperazin-1-yl]-1-(2-phenyl-2,4,6,7-tetrahydro-pyrazolo[4,3-c]pyridin-5-yl)-ethanone;
- Composé n°136: 1-(2-Phenyl-6,7-dihydro-4H-thiazolo[5,4-c]pyridin-5-yl)-2-[8-(5-thiazol-2-yl-pyridin-2-yl)-3,8-diaza-bicyclo[3.2.1]oct-3-yl]-ethanone;
- Composé n°137: 2-[8-(5-[1,2,4]Oxadiazol-3-yl-pyridin-2-yl)-3,8-diaza-bicyclo [3.2.1]oct-3-yl]-1-(2-phenyl-2,4,6,7-tetrahydro-pyrazolo[4,3-c]pyridin-5-yl)-ethanone;
- Composé n°138: 1-(2-Ethyl-2,4,6,7-tetrahydro-pyrazolo[4,3-c]pyridin-5-yl)-2-[8-(5-trifluoromethyl-pyridin-2-yl)-3,8-diaza-bicyclo[3.2.1]oct-3-yl]-ethanone;
- Composé n°139: 2-[(2S,6R)-2,6-Dimethyl-4-(5-[1,2,4]oxadiazol-5-yl-pyridin-2-yl)-piperazin-1-yl]-1-(2-phenyl-2,4,6,7-tetrahydro-pyrazolo[4,3-c]pyridin-5-yl)-ethanone;
- Composé n°140: acide 6-{(3S,5R)-3,5-Dimethyl-4-[2-oxo-2-(2-pyridin-2-yl-2,4,6,7-tetrahydro-pyrazolo[4,3-c]pyridin-5-yl)-ethyl]-piperazin-1-yl}-nicotinique;
- Composé n°141: 2-((2S,6R)-2,6-Dimethyl-4-pyridin-3-yl-piperazin-1-yl)-1-(2-pyridin-4-yl-2,4,6,7-tetrahydro-pyrazolo[4,3-c]pyridin-5-yl)-ethanone;
- Composé n°142: 2-((2S,6R)-2,6-Dimethyl-4-pyridin-3-yl-piperazin-1-yl)-1-[2-(5-fluoro-pyridin-2-yl)-2,4,6,7-tetrahydro-pyrazolo[4,3-c]pyridin-5-yl]-ethanone;
- Composé n°143: 2-((2S,6R)-2,6-Dimethyl-4-pyridin-3-yl-piperazin-1-yl)-1-[2-(5-trifluoromethyl-pyridin-2-yl)-2,4,6,7-tetrahydro-pyrazolo[4,3-c]pyridin-5-yl]-ethanone;
- Composé n°144: methyl ester de l'acide 6-{(3S,5R)-3,5-Dimethyl-4-[2-oxo-2-(2-pyridin-2-yl-2,4,6,7-tetrahydro-pyrazolo[4,3-c]pyridin-5-yl)-ethyl]-piperazin-1-yl}-nicotinique;
- Composé n°145: 2-((2S,6R)-2,6-Dimethyl-4-pyridin-3-yl-piperazin-1-yl)-1-(2-phenyl-3,4,6,7-tetrahydro-imidazo[4,5-c]pyridin-5-yl)-ethanone;
- Composé n°146: 2-[(2S,6R)-2,6-Dimethyl-4-(5-trifluoromethyl-pyridin-2-yl)-piperazin-1-yl]-1-(2-pyridazin-3-yl-2,4,6,7-tetrahydro-pyrazolo[4,3-c]pyridin-5-yl)-ethanone;
- Composé n°147: 2-[(2S,6R)-2,6-Dimethyl-4-(5-trifluoromethyl-pyridin-2-yl)-piperazin-1-yl]-1-(2-ethyl-2,4,6,7-tetrahydro-pyrazolo[4,3-c]pyridin-5-yl)-ethanone;
- Composé n°148: 4-[2-(2-Ethyl-2,4,6,7-tetrahydro-pyrazolo[4,3-c]pyridin-5-yl)-2-oxo-ethyl]-1-(5-trifluoromethyl-pyridin-2-yl)-piperazin-2-one;
- Composé n°149: acide 6-{(3S,5R)-3,5-Dimethyl-4-[2-oxo-2-(2-phenyl-2,4,6,7-tetrahydro-pyrazolo[4,3-c]pyridin-5-yl)-ethyl]-piperazin-1-yl}-nicotinique;
- Composé n°150: acide 6-{(3S,5R)-3,5-Dimethyl-4-[2-oxo-2-(2-phenyl-2,4,6,7-tetrahydro-pyrazolo[4,3-c]pyridin-5-yl)-ethyl]-piperazin-1-yl}-nicotinique ;
à l'état de base ou de sel d'addition à un acide.

Dans ce qui suit, on entend par "groupe protecteur Pg" un groupe qui permet, d'une part, de protéger une fonction réactive telle qu'un hydroxy ou une amine pendant une synthèse et, d'autre part, de régénérer la fonction réactive intacte en fin de synthèse. Des exemples de groupes protecteurs ainsi que des méthodes de protection et de déprotection sont données dans Protective Groups in Organic Synthesis, Green et al., 2nd Edition (John Wiley & Sons, Inc., New York).

Conformément à l'invention, on peut préparer les composés de formule générale (I) selon le procédé qui suit.

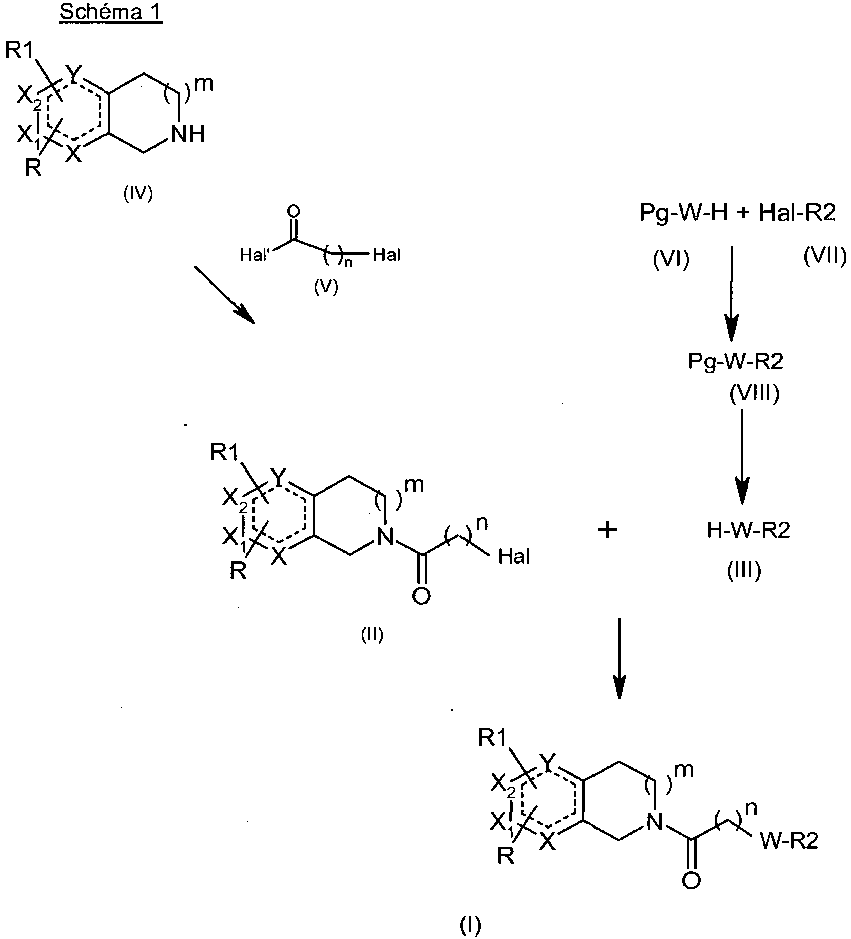

Plus précisément, le procédé de préparation des composés de formule générale (I) dans laquelle R, R1, X, X1, X2, Y, W, R2, m et n sont tels que définis précédemment comprend la réaction d'un composé de formule (II) : 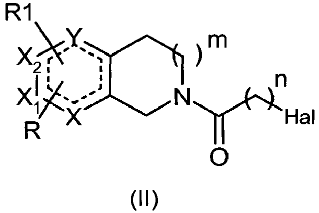 dans laquelle R, R1, X, X1, X2, Y, m et n sont définis comme en formule générale (I) et Hal représente un atome d'halogène, par exemple le chlore ; et d'un composé de formule générale (III) :

H-W-R2 (III)

dans laquelle W et R2 sont définis comme en formule générale (I), selon des méthodes connues de l'homme du métier, par exemple en présence d'une base, dans un solvant tel que décrit dans WO 03/104225. Ainsi, à titre de base, on peut citer les bases organiques telles que la triéthylamine, la N,N-diisopropylamine, la Diisopropyl-éthylamine (DPEA) ou la N-méthyl-morpholine ou les carbonates ou bicarbonates de métal alcalin tel que le carbonate de potassium, le carbonate de sodium ou le bicarbonate de sodium et en l'absence ou en présence d'un iodure de métal alcalin tel que l'iodure de potassium ou l'iodure de sodium. La réaction s'effectue dans un solvant tel que l'acétonitrile, le N,N-diméthylformamide (DMF), l'N-méthyl-pyrrodinone le toluène ou le propan-2-ol et à une température comprise entre la température ambiante et la température de reflux du solvant. Par température ambiante, on entend une température comprise entre 5 et 25°C. A titre d'exemple la réaction peut s'effectuer en présence de bicarbonate de sodium, d'iodure de sodium dans un solvant tel que le DMF. Ces réactions peuvent aussi être conduites dans un réacteur à micro-ondes.

Dans les composés de formule générale (I) ainsi obtenus, R, R1, R3, R4, R5, R6, R7 et R8 peuvent être modifiés par des traitements communément utilisés par l'homme du métier, comme par exemple par hydrolyse d'un groupe ester pour donner un groupe carboxylique ou d'un cyano pour obtenir un groupe tétrazole.

Généralement, les sels d'addition acide des composés de formule générale (I) peuvent être obtenus par addition de l'acide approprié, tel que l'acide chlorhydrique, l'acide bromhydrique, l'acide oxalique.

Les composés de formule (III), éventuellement sous forme de sels peuvent être préparés à partir des composés correspondants de formule (VIII) :

Pg-W-R2 (VIII)

dans laquelle W et R2 sont tels que définis en formule (I) et Pg représente un groupe protecteur d'un atome d'azote de W. De préférence, Pg est un groupe benzyle et la déprotection s'effectue selon des méthodes classiques, bien connues de l'homme du métier, par exemple par hydrogénation catalytique sur Pd/C ou par traitement avec des chloroformiates puis hydrolyse en milieu acide.

Les composés de formule (VIII) peuvent être préparés à partir des composés de formule (VI) :

Pg-W-H (VI)

et (VII) :

Hal-R2 (VII)

dans lesquelles Pg, W et R2 sont définis comme précédemment et Hal représente un atome d'halogène, de préférence le chlore. Cette réaction s'effectue généralement dans les mêmes conditions que la réaction de préparation des composés de formule (I) à partir des composés de formule (II) et (III).

Alternativement, les composés de formule (VIII) peuvent être préparés par la méthode de couplage de Buchwald en présence d'un catalysateur au Palladium et d'une phosphine opportunément choisie, en utilisant comme solvant des solvants inertes tels que le toluène ou le xylène, à une température comprise entre la température ambiante et 110°C.

Dans les composés de formule générale (VIII) ainsi obtenus, R7 et R8, peuvent être modifié par des traitements communément utilisés par l'homme du métier, comme par exemple la synthèse d'un groupe oxadiazole à partir d'un groupe cyano, ou bien par des couplages de Suzuki comme décrit dans le schéma ci-desous.

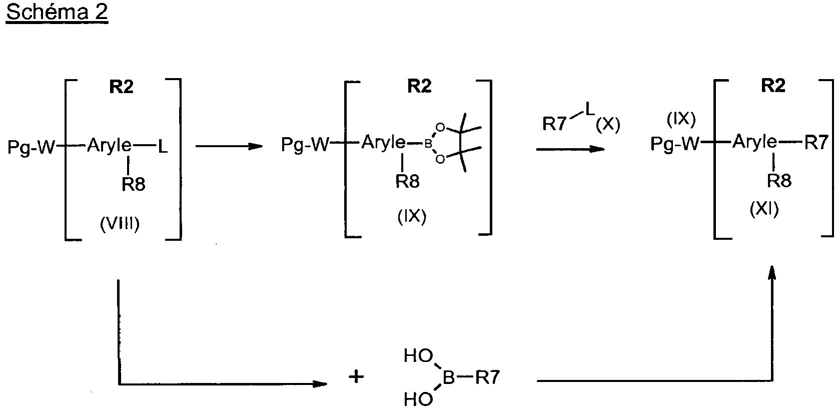

Dans le schéma 2 ci-dessus, L représente un groupe partant comme l'iodo, le bromo ou le trifluorométhanesulphonate ; R7 représente un hétérocycle comme décrit dans la formule générale (I), R8 est tel que défini dans la formule génèrale (I) et B représente l'atome de Bore.

Des exemples des telles réactions sont décrits dans la partie expérimentale.

Les composés de formule (III), éventuellement sous forme de sels, quant W représente une Oxo-pipérazine, sont disponibles dans le commerce ou décrits dans la littérature, ou bien peuvent être préparés, à partir des composés correspondants de formule (VII), selon des méthodes qui sont décrites ou connues de l'homme du métier.

Des exemples des telles préparations sont décrits dans la partie expérimentale.

Les composés de formule (II) peuvent être obtenus par réaction d'un composé correspondant de formule (IV) :

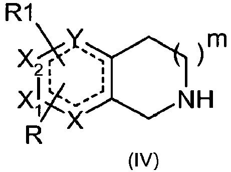

Dans laquelle R, R1, X, X1, X2, Y et m sont définis comme en formule générale (I) ; éventuellement sous forme de sel d'addition acide, et d'un composé de formule (V) : 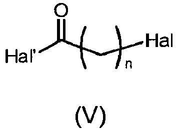 dans laquelle Hal et n sont tels que définis en formule (II) et Hal' représente un atome d'halogène, identique ou différent de Hal. De préférence, Hal' représente un atome de chlore.

Cette réaction s'effectue généralement en présence d'une base, telle que la triéthylamine, la N,N-diisopropyléthylamine ou la N-méthylmorpholine, dans un solvant tel que le dichlorométhane, le chloroforme, le tétrahydrofurane, le dioxane ou un mélange de ces solvants et à une température comprise entre 0°C et la température ambiante. Les composés de formule (V) sont généralement disponibles commercialement.

Eventuellement, le procédé selon l'invention comprend l'étape ultérieure consistant à isoler le produit désiré obtenu.

Les produits de formules (IV) (V), (VI), (VII), et les réactifs, quand leur mode de préparation n'est pas décrit, sont disponibles dans le commerce ou décrits dans la littérature, ou bien peuvent être préparés selon des méthodes qui sont décrites ou connues de l'homme du métier.

De façon alternative, les composés de formule (I) peuvent être préparés selon le schéma 3 qui suit :

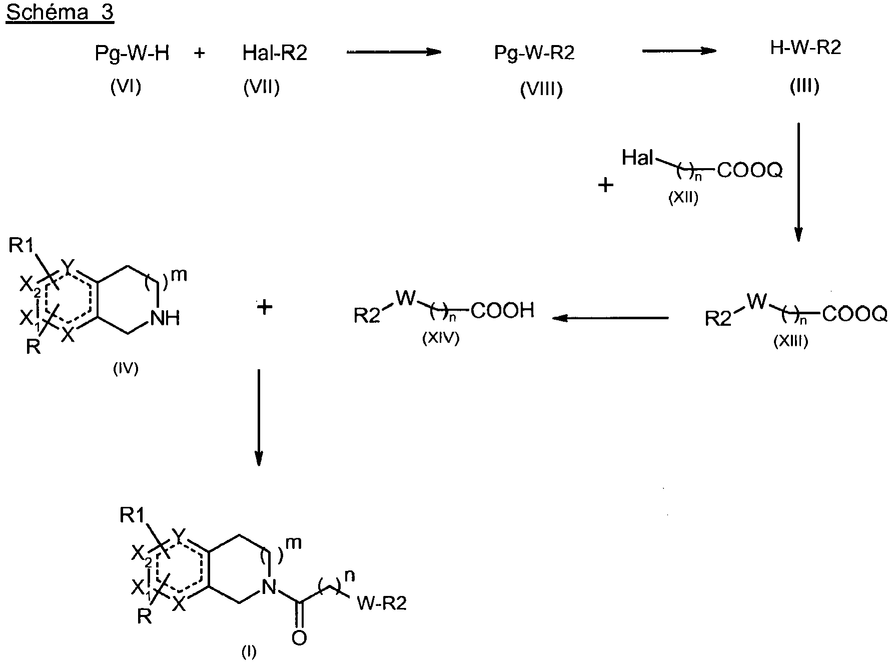

Plus précisément, le procédé de préparation des composés de formule générale (I) dans lequel R, R1, X, X1, X2, Y, W, R2, m et n sont tels que définis précédemment et Q représente un résidu apte à former un ester, tel que le méthyl, l'éthyl ou le benzyl, comprend la réaction d'un composé de formule (XIV) : 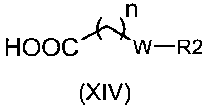 dans laquelle R2 , W et n sont définis comme en formule générale (I) et d'un composé de formule générale (IV) 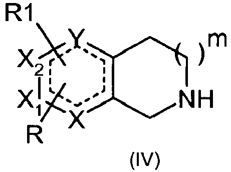 dans laquelle R, R1, X, X1, X2, Y et m sont définis comme en formule générale (I),
selon des méthodes connues de l'homme du métier, par exemple, dans un solvant tel que le dichlorométhane, le DMF ou le THF, en présence d'une base tel que la pyridine, la triéthylamine, la N,N-diisopropylamine, la Diisopropyl-éthylamine (DPEA) et d'une agent de condensation tel que le BOP, le DBU ou la DCC. La réaction s'effectue à une température comprise entre la température ambiante et la température de reflux du solvant. Par température ambiante, on entend une température comprise entre 5 et 25°C. A titre d'exemple, la réaction peut s'effectuer en présence de bicarbonate de sodium, d'iodure de sodium dans un solvant tel que le DMF. Ces réactions peuvent aussi être conduites dans un réacteur à micro-ondes.

Dans les composés de formule générale (I) ainsi obtenus, R, R1, R3, R4, R5, R6, R7 et R8 peuvent être modifiés par des traitements communément utilisés par l'homme du métier, comme par exemple par hydrolyse d'un groupe ester pour donner un groupe carboxylique ou d'un cyano pour donner un groupe tétrazole.

Généralement, les sels d'addition acide des composés de formule générale (I) peuvent être obtenus par addition de l'acide approprié, tel que l'acide chlorhydrique, l'acide bromhydrique, l'acide oxalique.

Les composés de formule (XIV) peuvent être obtenus à partir de composés de formule (XIII) 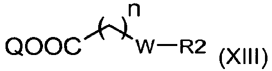 dans laquelle R2, W et n sont définis comme en formule générale (I) et Q représente un résidu adpte à former un ester, tel que le méthyl, l'éthyl ou le benzyl, par hydrolyse de la liaison ester selon des méthodes connues de l'homme du métier, par exemple par un traitement en milieu acqueux acide ou basique, ou bien par réduction dans un solvant polaire tel qu'un alcool ou le THF, sous flux d' hydrogène.

Les composés de formule (XIII) peuvent être obtenus à partir de composés de formule (III)

H-W-R2 (III)

dans laquelle R2 et W sont définis comme en formule générale (I) ; éventuellement sous forme de sel d'addition acide, et d'un composé de formule (XII) : 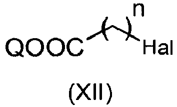 dans laquelle Q représente un résidu apte à former un ester tel que le méthyl, l'éthyl ou le benzyl. Hal représente un atome d'halogène, de préférence un atome de chlore, n est tel que défini dans la formule générale (I).

Cette réaction s'effectue généralement en présence d'une base, telle que la triéthylamine, la N,N-diisopropyléthylamine ou la N-méthylmorpholine, dans un solvant tel que le dichlorométhane, le chloroforme, le tétrahydrofuranne, le dioxane ou un mélange de ces solvants et à une température comprise entre 0°C et la température ambiante. Les composés de formule (XII) sont généralement disponibles commercialement.

Les composés de formule (III), éventuellement sous forme de sels peuvent être préparés à partir des composés correspondants de formule (VIII) :

Pg-W-R2 (VIII)

dans laquelle W et R2 sont tels que définis en formule (I) et Pg représente un groupe protecteur d'un atome d'azote de W. De préférence, Pg est un groupe benzyle et la déprotection s'effectue selon des méthodes classiques, bien connues de l'homme du métier, par exemple par hydrogénation catalytique sur Pd/C ou par traitement avec des chloroformiates puis hydrolyse en milieu acide.

Les composés de formule (VIII) peuvent être préparés à partir des composés de formule (VI) :

Pg-W-H (VI)

et (VII) :

Hal-R2 (VII)

dans lesquelles Pg, W et R2 sont définis comme précédemment et Hal représente un atome d'halogène, de préférence le chlore. Cette réaction s'effectue généralement dans les mêmes conditions que la réaction de préparation des composés de formule (I) à partir des composés de formule (IV) et (XIV).

Alternativement, les composés de formule (VIII) peuvent être préparés par la méthode de couplage de Buchwald en présence d'un catalysateur au Palladium et d'une phosphine opportunément choisie, en utilisant comme solvant des solvants inertes tels que le toluène ou le xylène, à une température comprise entre la température ambiante et 110°C.

Dans les composés de formule générale (VIII) ainsi obtenus, R7 et R8, peuvent être modifié par des traitements communément utilisés par l'homme du métier, comme par exemple la synthèse d'un groupe oxadiazole à partir d'un groupe cyano, ou bien par des couplages de Suzuki comme déjà décrit dans le schéma 2 déjà exposé ci-dessus.

Les composés de formule (III), éventuellement sous forme de sels, quant W représente une Oxo-pipérazine, sont disponibles dans le commerce ou décrits dans la littérature, ou bien peuvent être préparés, à partir des composés correspondants de formule (VII), selon des méthodes qui sont décrites ou connues de l'homme du métier.

Des exemples des telles préparations sont décrits dans la partie expérimentale.

Eventuellement, le procédé selon l'invention comprend l'étape ultérieure consistant à isoler le produit désiré obtenu.

Les produits de formules (IV), (VI), (VII), et les réactifs, quand leur mode de préparation n'est pas décrit, sont disponibles dans le commerce ou décrits dans la littérature, ou bien peuvent être préparés selon des méthodes qui sont décrites ou connues de l'homme du métier.

Des exemples des telles préparations sont décrits dans la partie expérimentale.

Selon un autre de ses aspects, l'invention a également pour objet des composés de formule (II) 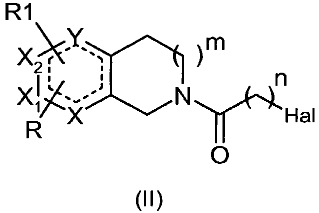 dans laquelle R1, R, X, X1, X2, Y, m, n et Hal sont définis comme précédemment ; à l'état de base ou de sel d'addition à un acide. Ces composés sont utiles comme intermédiaires de synthèse des composés de formule (I).

Les exemples suivants décrivent la préparation de certains composés conformes à l'invention. Ces exemples ne sont pas limitatifs et ne font qu'illustrer la présente invention. Les numéros des composés exemplifiés renvoient à ceux donnés dans le tableau ci-après, qui illustre les structures chimiques et les propriétés physiques de quelques composés selon l'invention.

Les mesures physico-chimiques ont été effectuées de la façon suivante :
Les points de fusion ont été mesurés avec un appareil Buchi B540.

Les spectres de résonnance magnétique nucléaire du proton (RMN 1 H) ont été enregistrés dans les conditions suivantes:
a) à 500 MHz sur un appareil Bruker équipé avec une console Avance III ;
b) à 400MHz sur un appareil Bruker équipé avec une console Avance I.

Les déplacements chimiques sont rapportés en ppm par rapport à la fréquence TMS.

Les spectres ont étés enregistrés dans les conditions de température suivantes :
Temp. A : 40°C
Temp. B : 30°C

Les abréviations utilisées pour caractériser les signaux sont les suivantes: s = singulet, bs =singulet large, m = multiplet, bm= multiplet large, d = doublet, bd= doublet large, t = triplet, q = quadruplet.
* = non intégrable à cause de l'interférence avec un pic large du à l'eau.
** = non intégrable à cause de l'interférence avec un pic du au solvant RMN.
2Xs= deux singulet partiellement superposés.
2Xbs= deux singulet large partiellement superposés.
2Xm= deux multiplets partiellement superposés.

L'HPLC a été effectuée au moyen d'un système ThermoElectron LCQ Deca XP Max équipé avec un détecteur à spectrométrie de masse à trappe d'ions ainsi qu'un détecteur à barre de diodes.

Les conditions d'analyse par chromatographie liquide couplée à la spectrométrie de masse (LC/UV/MS) sont les suivantes:

### - système chromatographique A

- Eluant A = H₂O + 0,01 % TFA
- Eluant B = CH₃CN
- Gradient de 98% de A à 95% de B en 10 minutes, puis élution par 95% de B pendant 5 minutes.
- Débit 0,5 ml/minute; température 40°C
- Injection de 2 µL de solution à 0,1 mg/ml dans un mélange CH₃CN : H₂O = 9 :1

### - système chromatographique B

- Eluant A = H₂O + 0,05% TFA
- Eluant B = CH₃CN + 0.035 TFA
- Gradient de 98% de A à 95% de B en 12 minutes, puis élution par 95% de B pendant 3 minutes
- Débit 0,7 ml/minute; température 40°C
- Injection de 2 µL de solution à 0,1mg/ml dans un mélange CH₃CN : H₂O = 9 :1

### - système chromatographique C

- Eluant A = Tampon acétate d'ammonium 5mM pH 6.5
- Eluant B = CH₃CN
- Gradient de 98% de A à 95% de B en 10 minutes, puis élution par 95% de B pendant 5 minutes.
- Débit 0,5 ml/minute; température 40°C
- Injection de 2 µL de solution à 0,1mg/ml dans un mélange CH₃CN : H₂O = 9 :1

Les produits sont détectés en UV à 220 nm.

Les colonnes utilisées sont des C18 avec une granulométrie entre 2 et 5 µm de préférence de 3,5 µm.

Pour la partie spectrométrie de masse :
- Mode d'ionisation: électrospray positif (ESI+)
- Balayage de 100 à 1200 uma

La chromatographie en couche mince a été effectuée sur des plaques CCM de gel de silice Merck Silica gel 60. Le gel de silice pour la chromatographie sur colonne flash est commercialisé par Biotage ou Supelco.

Tous les solvants utilisés sont de pureté "reagent grade" ou "HPLC grade".

### Préparation 1

### (3R,5S)-3,5-Diméthyl-1-(5-trifluorométhyl-pyridin-2-yl)-pipérazine

On charge 0,8 g de 2-chloro-5(trifluorométhyl)pyridine (composé de formule (VII)), 0,5g de cis-2-6 diméthylamino pipérazine (composé de formule (VI)), 0,67 g de carbonate de potassium et 0,3 g de Nal dans 8 mL de DMF. La réaction est conduite dans un initiateur à micro-ondes CEMdiscover pendant 30 mn à 160°C. Puis, on verse dans une solution aqueuse saturée en chlorure de sodium et on extrait avec de l'acétate d'éthyle. La phase organique est séchée sur Na₂SO₄, filtrée et évaporée sous vide. On isole 1,1 g d'une matière huileuse correspondant au produit du titre.

### Préparation 2

### Chlorhydrate de 2-[8-(5-fluoro-pyrimidin-2-yl)-3,8-diaza-bicyclo[3.2.1]octane

On charge 1,44 g de 2-chloro-5-fluoropyrimidine (composé de formule (VII)), 2,2g de 1-benzyl-3,8-diaza-bicyclo[3.2.1]octane (composé de formule (VI)), 1,7 g de carbonate de potassium et 0,73 g de Nal dans 27 mL de N-méthylpyrrolidone. On chauffe à 110°C pendant 5 heures. Puis on verse dans une solution aqueuse saturée en chlorure de sodium et on extrait avec de l'acétate d'éthyle. La phase organique est séchée sur Na₂SO₄, filtrée et évaporée sous vide. On isole 3,2 g d'une matière huileuse que l'on purifie par flash chromatographie sur colonne Biotage®, au moyen de l'éluant cyclohexane 95/acétate d'éthyle 5. On isole 1,4 g de solide blanc que l'on dissous dans 35 ml de 1,2-dichloroéthane. On ajoute à 0°C 0,72 ml de 1-chloroéthylchloroformate et laisse agiter sous flux d'azote pendant 10 minutes à 0°C et ensuite 3 heures à 85°C. On évapore le solvant et on ajoute 35 ml de méthanol. On chauffe pendant 30 minutes à la température de reflux. On évapore le solvant et on traite le résidu avec de l'isopropanol. On obtient un solide blanc que l'on filtre et on isole 900 mg de produit du titre. Pf 236-239°C

### Préparation 3

### (3R,5S)-3,5-Diméthyl-1-(6-trifluorométhyl-pyridin-2-yl)-pipérazine

On charge 2,2 g de 2-trifluorométhyl-5-bromo-pyridine (composé de formule (VII)), 1,1g de cis-2-6 diméthyl pipérazine (composé de formule (VI)) 0,22 g de palladium acétate, 0,28 g de sodium t-butoxyde et 1,3 g de tri-t-butyl phosphine dans 16 mL de o-xilène. On chauffe à 120°C pendant 6 heures. On filtre sur célyte et on évapore le solvant. On isole 1,8 g d'une matière huileuse correspondent au produit du titre.

### Préparation 4

### Chlorhydrate du méthyl ester de l'acide 3,8-diaza-bicyclo[3.2.1]oct-8-yl-nicotinique

On charge 0,42 g de 6-chloronicotinate de méthyl (composé de formule (VII)), 0,5g de 1-benzyl-3,8-diaza-bicyclo[3.2.1]octane (composé de formule (VI)), 0,4 g de carbonate de potassium et 0,17 g de Nal dans 7 mL de N-méthylpyrrolidone. On chauffe pendant 7 heures à 110°C. Puis on verse dans une solution aqueuse saturée en chlorure de sodium et on extrait avec de l'acétate d'éthyle. La phase organique est séchée sur Na₂SO₄, filtrée et évaporée sous vide. On isole 1,1 g d'une matière huileuse que l'on purifie par flash chromatographie sur colonne Biotage®, au moyen de l'éluant cyclohexane 8/acétate d'éthyle 2. On isole 520 mg d'une huile claire. On hydrogène à 40°C sous pression atmosphérique pendant 2 heures le produit obtenu à l'étape précédente, dans 20 mL d'éthanol, 2 mL d'isopropanol.HCl, en présence de 0,22 g de Pd/C à 10%. On filtre, on évapore sous vide et on isole 440 mg du produit du titre, sous forme de solide blanc.

### Préparation 5

### Chlohydrate de Méthyl ester de l'acide 6-(2-Oxo-pipérazin-1-yl)-nicotinique

### Etape a) Méthyl ester de l'acide 6-(2-Benzylamino-éthylamino)-nicotinique):

On chauffe à 135° C pendant 6 heures dans un ballon, 4,6 g de 6-chloronicotinate de méthyle et 40,5 ml de N-benzyléthylèndiamine.On verse dans l'eau et on extrait avec de l'acétate d'éthyle. On sèche et évapore sous vide ; le brut ainsi obtenu est purifié par flash chromatographie.

### Etape b) Méthyl ester de l'acide 6-(4-Benzyl-2-oxo-pipérazin-1-yl)-nicotinique) :

Le produit de l'étape a), 2,2 g, est solubilisé dans 35 ml d'une solution 2N de HCl. On ajoute 5 g de dihydrate de glyoxal trimérique et on laisse agiter à température ambiante pendant 120 heures. On extrait avec de l'acétate d'éthyle. On sèche et évapore sous vide; le brut ainsi obtenu est purifié par flash chromatographie.

### Etape c) (Chlorhydrate de Méthyl ester de l'acide 6-(2-Oxo-pipérazin-1-yl)-nicotinique) :

Le produit isolé de 1,4 g est solubilisé dans 150 ml d'éthanol, puis on ajoute 4 ml d'une solution d'isopropanol saturé avec HCl et 0,6 g de Pd/C à 10%. On laisse réagir sous flux d'hydrogène pendant 4 heures à la température de 40°C. On filtre et on évapore sous vide et on obtient 0,52 g du composé du titre.

### Préparation 6

### 2-Chloro-1-(2-phényl-6,7-dihydro-4H-thiazolo[4,5-c]pyridin-5-yl)-éthanone

### Etape a) (2-Bromo-4-pipéridone) :

On dissous 10 g de 1-Boc-4-pipéridone dans 280 ml de dichlorométhane. On ajoute lentement 8 g de brome et on laisse sous agitation à température ambiante pendant 2 heures. On évapore sous vide et on obtient un solide. On le traite avec de l'éther isopropylique pour obtenir une solide blanc que l'on filtre.

### Etape b) (2-Phényl-4,5,6,7-tétrahydro-thiazolo[5,4-c]pyridine) :

On charge 4,5 g du produit de l'étape a) dans un ballon avec 34 ml de DMF et 2,6 g de Thiobenzamide. On chauffe à la température de 60° C pendant 6 heures. On ajoute une solution d'ammoniaque jusqu'à pH basique et on évapore sous vide.

On purifie sur colonne par flash chromatographie au moyen d'une colonne Biotage® que l'on élue avec de l'éthyle acétate et ensuite avec du méthanol. On isole 4 g d'un solide marron que l'on crystalise avec de l'isopropanol. On filtre et on obtient 2,8 g d'un solide beige.

### Etape c) (2-Chloro-1-(2-phényl-6,7-dihydro-4H-thiazolo[4,5-c]pyridin-5-yl)-éthanone):

Dans un ballon muni d'un agitateur magnétique, on met en suspension 2,8 g du ce produit dans 50 mL de dichlorométhane. On ajoute 2,8 mL de triéthylamine et on porte à 0°C. A 0°C, on coule goutte à goutte 1,5 mL de chloroacétylchlorure, c'est-à-dire le composé de formule générale (V) dans laquelle Hal=Hal'=Cl et n=1. On laisse réagir pendant 1 heure et demie et on verse dans de l'eau. On extrait au dichlorométhane. La phase organique est séchée sur Na₂SO₄, filtrée et évaporée sous vide. On isole 4,1 g d'une graisse huileuse et sombre que l'on triture puis que l'on laisse reposer au froid. On décante et on évapore le surnageant sous vide. On isole 1,1 mg du produit du titre sous forme d'une huile claire.

### Préparations 7 -I et 7-II

### 2-Chloro-1-(2-phényl-2,4,6,7-tétrahydro-pyrazolo[4,3-c]pyridin-5-yl)-éthanone (7-I)

### et 2-Chloro-1-(1-phényl-1,4,6,7-tétrahydro-pyrazolo[4,3-c]pyridin-5-yl)-éthanone (7-II)

### Etape a)Tert-butyl ester de l'acide (3-[1-Diméthylamino-méth-(Z)-ylidene]-4-oxo-pipéridine-1-carboxylique) :

On chauffe à la température de reflux dans un ballon 10 g de 1-Boc-4-pipéridone et 7,2 g de N,N-diméthylformamide-diméthylacétate. On purifie sur colonne par flash chromatographie le brut ainsi obtenu en isolant 2,4 g d'une matière huileuse.

### Etape b) (Tert-butyl ester de l'acide 2-Phényl-2,4,6,7-tétrahydro-pyrazolo[4,3-c]pyridine-5-carboxylique (b-I) et Tert-butyl ester de l'acide 1-Phényl-1,4,6,7-tétrahydro-pyrazolo[4,3-c]pyridine-5-carboxylique (b-II)) :

On dissous dans 27 ml de méthanol le produit de l'étape a) (2,4 g). On ajoute 1,24 g de phénylhydrazine et on chauffe 3 heures à la température de reflux. On évapore sous vide et on purifie sur colonne par flash chromatographie au moyen d'une colonne Biotage® que l'on élue avec un mélange d'éthyl acétate et cyclohexane. On isole 1,8 g d'une matière huileuse.

### Etape c) Chlorhydrate de (2-Phényl-2,4,6,7-tétrahydro-pyrazolo[4,3-c]pyridine- (c-l) et Chlorhydrate de 1-Phényl-1,4,6,7-tétrahydro-pyrazolo[4,3-c]pyridine-5-(c-II))

Le mélange des produits de l'étape b) (b-I et b-II, 2,4 g) est dissous lentement dans 60 ml d'acide trifluoroacétique à 0°C. On laisse ensuite sous agitation pendant 2 heures à température ambiante. On évapore sous vide l'acide trifluoroacétique, on ajoute de l'acide chloridrique 37% et on évapore sous vide à sec. On crystalise avec de l'isopropanol. On obtient 1 g de solide beige.

### Etape d) 2-Chloro-1-(2-phényl-2,4,6,7-tétrahydro-pyrazolo[4,3-c]pyridin-5-yl)-éthanone (7-I) et 2-Chloro-1-(1-phényl-1,4,6,7-tétrahydro-pyrazolo[4,3-c]pyridin-5-yl)-éthanone (7-II)

Le mélange des produits de l'étape c) (c-I et c-II, 1 g) est mis en suspension dans un ballon muni d'un agitateur magnétique, dans 26 mL de dichlorométhane. On ajoute 1,23 mL de triéthylamine et on porte à 0°C. A 0°C, on coule goutte à goutte 0,5 mL de chloroacétylchlorure, c'est-à-dire le composé de formule générale (V) dans laquelle Hal=Hal'=Cl et n=1. On laisse réagir pendant 1 heure et demie et on verse dans de l'eau. On extrait au dichlorométhane. La phase organique est séchée sur Na₂SO₄, filtrée et évaporée sous vide. Le résidu est purifié par flash chromatographie sur colonne Biotage®, au moyen de l'éluant cyclohexane 9/acétate d'éthyle 1.On isole 0,15 g du produit 7-I du titre (produit plus polaire) sous forme d'huile claire et 0,15g du produit 7-II du titre (produit moins polaire) sous forme d'huile claire.

### Préparation 8

### 2-Chloro-1-[1-(2,2,2-trifluoro-ethyl)-1,4,6,7-tetrahydro-pyrazolo[4,3-c]pyridin-5-yl]-ethanone

En opérant comme décrit dans la préparation 7, mais en utilisant la (2,2,2-Trifluoro-éthyl)-hydrazine au lieu de la phénylhydrazine on obtient le composé du titre sous forme d'un huile claire.

### Préparation 9

### 2-Chloro-1-(2-pyridin-2-yl-2,4,6,7-tetrahydro-pyrazolo[4,3-c]pyridin-5-yl)-éthanone

### Etape a) Tert--butylester de l'acide 4-(Pyridin-2-yl-hydrazono)-piperidine-1-carboxylique:

Dans un ballon muni d'un agitateur magnétique, on charge : 5 g de N-Boc-piperidone, 2,45 g de Pyridin-2-yl-hydrazine, 100 ml de méthanol et 95 ml d'une solution de méthanol saturée en HCl On laisse réagir sous flux d'azote pendant 2 h à température de reflux. On évapore le solvant sous vide, on dissous avec du dichlorométhane et on lave avec une solution acqueuse saturée en NaHCO3. On sèche la phase organique sur Na₂SO₄ et on évapore le solvant et on isole 4,0 g du produit du titre sous forme d'huile rouge.

### Etape b) Tert-butyl ester de l'acide 2-Pyridin-2-yl-2,4,6,7-tetrahydro-pyrazolo[4,3-c]pyridine-5-carboxylique:

On charge dans un ballon 33 ml de DMF et on ajoute lentement à 0°C 3,7 ml de POCI3. On laisse sous agitation à 0°C pendant 30 min puis on ajoute 33 ml de pyridine , 4,0 g du produit de l'étape a) et 5 ml de DMF. On laisse ensuite sous agitation pendant 4 heures à la température de 80°C. On ajoute 270 ml d'eau et on extrait avec de l'acétate d'éthyle. On sèche la phase organique sur Na₂SO₄ et on évapore le solvant. On isole 4,0 g du produit du titre sous forme d'huile noire que l'on purifie par flash chromatographie sur colonne Biotage®, au moyen de l'éluant hexane 8/acétate d'éthyle 2.On isole 0,78 g du produit sous forme d'un solide jaune.

### Etape c) Chlorhydrate de la 2-Pyridin-2-yl-4,5,6,7-tetrahydro-2H-pyrazolo[4,3-c]pyridine :

0,78 g du produit de l'étape b) est dissous à 0°C dans un ballon avec 21 ml d'acide trifluoroacétique. On laisse ensuite sous agitation pendant 2 heures à température ambiante.

### On évapore sous vide l'acide trifluoroacétique, on ajoute de l'acide chlorhydrique 37% et on évapore sous vide à sec. On crystalise avec de l'isopropanol. On obtient 0,6 g de solide beige.

### Etape d) 2-Chloro-1-[1-(2,2,2-trifluoro-ethyl)-1,4,6,7-tetrahydro-pyrazolo[4,3-c]pyridin-5-yl]-ethanone

0.3 g du produit de l'étape c) est mis en suspension dans un ballon muni d'un agitateur magnétique, dans 4 mL de dichlorométhane. On ajoute 0,36 mL de triéthylamine et on porte à 0°C. A 0°C, on coule goutte à goutte 0,12 mL de chloroacétylchlorure, c'est-à-dire le composé de formule générale (V) dans laquelle Hal=Hal'=Cl et n=1. On laisse réagir pendant 1 heure et demie et on verse dans de l'eau. On extrait au dichlorométhane. La phase organique est séchée sur Na₂SO₄, filtrée et évaporée sous vide. Le résidu est purifié par flash chromatographie sur colonne Biotage®, au moyen de l'éluant cyclohexane 9/acétate d'éthyle 1.On isole 0,19 g du produit du titre sous forme d'huile claire.

### Préparation 10

### Chlorhydrate de la 2-(2,2,2-Trifluoro-ethyl)-4,5,6,7-tetrahydro-2H-pyrazolo[4,3-c]pyridine:

En opérant comme décrit dans la préparation 9 jusqu'à l'étape c, mais en utilisant la (2,2,2-Trifluoro-ethyl)-hydrazine au lieu de la pyrid-2-yl-hydrazine on obtient le composé du titre sous forme d'un solide jaune pâle.

### Préparation 11

### Ethyl ester de l'acide 6-((3R,5S)-4-Carboxymethyl-3,5-dimethyl-piperazin-1-yl)-nicotinique

### Etape a) Ethyl ester de l'acide 6-((3R,5S)-3,5-Dimethyl-piperazin-1-yl)-nicotinique:

En opérant comme décrit dans la préparation 3 mais en utilisant l'éthyl ester de l'acide nicotinique au lieu de la 2-trifluorométhyl-5-bromo-pyridine, on obtient le composé du titre sous forme d'un huile claire.

### Etape b) Ethyl ester de l'acide 6-((3R,5S)-4-Benzyloxycarbonylmethyl-3,5-dimethyl-piperazin-1-yl)-nicotinique

Dans un ballon muni d'un agitateur magnétique, on charge : 3,6 g du produit de l'étape précédente, 132 ml de THF, 2,6 ml de benzyl-bromoacétate, 4,4 ml de triéthylamine. On laisse réagir sous flux d'azote pendant une nuit à température ambiante. On évapore le solvant et on purifie par flash chromatographie sur colonne Biotage®, au moyen de l'éluant hexane 7/acétate d'éthyle 3. On isole 2,9 g du produit du titre sous forme d'huile claire.

### Etape c) Ethyl ester de l'acide 6-((3R,5S)-4-Carboxymethyl-3,5-dimethyl-piperazin-1-yl)-nicotinique

2,9 g du produit de l'étape précédente est solubilisé dans 290 ml d'éthanol, puis on ajoute 1,74 g de Pd/C à 10%. On laisse réagir sous flux d'hydrogène pendant 4 heures à la température de 40°C. On filtre et on évapore sous vide et on obtient 2,2 g du composé du titre sous forme d'un solide blanc.

### Préparation 12

### Acide [(2R,6S)-2,6-Dimethyl-4-(5-trifluoromethyl-pyridin-2-yl)-piperazin-1-yl]-acetique

### Etape a) Ethyl ester de l'acide [(2R,6S)-2,6-Dimethyl-4-(5-trifluoromethyl-pyridin-2-yl)-piperazin-1-yl]-acétique

En opérant comme décrit dans l'étape b) de la préparation 11, mais en utilisant le composé de la préparation 3 au lieu du composé de l'étape a) de la préparation 11 et l'éthyl ester de l'acide bromoacétique au lieu du benzyl ester de l'acide bromoacétique, on obtient le composé du titre sous forme solide.

### Etabe b) Acide [(2R,6S)-2,6-Diméthyl-4-(5-trifluoromethyl-pyridin-2-yl)-piperazin-1-yl]-acetique :

2,5 g du produit de l'étape précédente sont solubilisés dans 22 ml d'éthanol, puis on ajoute 5 ml d'une solution acqueuse à 40% de NaOH. On laisse réagir pendant 3 heures à la température de 70°C. On règle le pH à 6 à l'aide d'une solution 1 N de HCl. On extrait à l'acétate d'éthyle. La phase organique est séchée sur Na₂SO₄, filtrée et évaporée sous vide.On isole 1,6 g du produit du titre sous forme d'un solide blanc.

### Préparation 13

### Méthyl ester de l'acide 6-((3R,5S)-3,5-Diméthyl-piperazin-1-yl)-nicotinique

En opérant comme décrit dans la préparation 1, mais en utilisant le méthyl ester de l'acide 6-Chloro-nicotinique au lieu du 2-chloro-5(trifluorométhyl)pyridine on obtient le composé du titre sous forme d'huile.

### Préparation 14

### 2-Chloro-1-[2-(5-trifluoromethyl-pyridin-2-yl)-2,4,6,7-tetrahydro-pyrazolo[4,3-c]pyridin-5-yl]-ethanone

1 g du chlorhydrate de la 2-(5-Trifluoromethyl-pyridin-2-yl)-4,5,6,7-tetrahydro-2H-pyrazolo[4,3-c]pyridine est mis en suspension dans un ballon muni d'un agitateur magnétique, dans 12 mL de dichlorométhane. On ajoute 0,95 mL de triéthylamine et on porte à 0°C. A 0°C, on coule goutte à goutte 0,33 mL de chloroacétylchlorure, c'est-à-dire le composé de formule générale (V) dans laquelle Hal=Hal'=Cl et n=1. On laisse réagir pendant 1 heure et demie et on verse dans de l'eau. On extrait au dichlorométhane. La phase organique est séchée sur Na₂SO₄, filtrée et évaporée sous vide. Le résidu est purifié par flash chromatographie sur colonne Biotage®, au moyen de l'éluant cyclohexane 9/ éthyle acétate 1. On obtient 1,08 g du composé du titre sous forme de solide jaune.

### Préparation 15

### Chlorydrate du 8-Pyridin-3-yl-3,8-diaza-bicyclo[3.2.1]octane

### Etape a) 3-Benzyl-8-pyridin-3-yl-3,8-diaza-bicyclo[3.2.1]octane :

En opérant comme décrit dans la préparation 3, mais en utilisant le 3-bromo-pyridine au lieu 2-trifluorométhyl-5-bromo-pyridine et le 1-benzyl-3,8-diaza-bicyclo[3.2.1]octane au lieu de la cis-2-6 diméthyl pipérazine, on obtient le composé du titre sous forme d'huile.

### Etape b) 8-Pyridin-3-yl-3,8-diaza-bicyclo[3.2.1]octane

Le produit isolé de 1,1 g est solubilisé dans 70 ml d'éthanol, puis on ajoute 4 ml d'une solution d'isopropanol saturé avec HCl et 0,6 g de Pd/C à 10%. On laisse réagir sous flux d'hydrogène pendant 4 heures à la température de 40°C. On filtre et on évapore sous vide et on obtient 0,84g du composé du titre.

### Préparation 16

### (3R,5S)-3,5-Dimethyl-1-(5-[1,2,4]oxadiazol-3-yl-pyridin-2-yl)-piperazine

### Etape a) 6-((3R,5S)-3,5-Dimethyl-piperazin-1-yl)-nicotinonitrile

En opérant comme décrit dans la préparation 1, mais en utilisant le 2-chloro-5-cyano-pyridine au lieu 2-chloro-5(trifluorométhyl)pyridine, on obtient le composé du titre sous forme d'huile.

### Etape b) (3R,5S)-3,5-Dimethyl-1-(5-[1,2,4]oxadiazol-3-yl-pyridin-2-yl)-piperazine

Dans un ballon muni d'un agitateur magnétique, on charge : 1 g du produit de l'étape précédente, 15 ml d'éthanol, une solution acqueuse de chlorhydrate d' hydroxilamine (2 équivalents), une solution de 0,98 g de Na₂SO₄ dans 7,2 ml d'eau. On laisse réagir sous flux d'azote pendant 4 h à la température de 90°C. On filtre le précipitat qui se forme et on évapore sous vide 0,5g du brut ainsi obtenu (1,7g). On le charge dans un ballon où 20 ml d'anhydride acétique sont ajoutés à 0°C.

On laisse réagir sous flux d'azote pendant 2,5 heures à la température de reflux. On évapore sous vide et on reprend le résidu avec de l'étyle acétate et une solution acqueuse saturée en K2CO3. La phase organique est séchée sur Na₂SO₄, filtrée et évaporée sous vide.

On dissous 0,27 g du brut ainsi obtenu dans 8 ml de HCl 6N. On laisse réagir sous flux d'azote pendant 2 h à la température de reflux. On règle le pH à 9 avec de la soude et on extrait avec de l'éthyl acétate. La phase organique est séchée sur Na₂SO₄, filtrée et évaporée sous vide. Le résidu est purifié par flash chromatographie sur colonne Biotage®, au moyen de l'éluant éthyle acétate 8 /méthanol 2. On isole 0,1 g du produit du titre sous forme d'huile qui a tendance à se solidifier.

### Préparation 17

### 1-[5-(2H-Pyrazol-3-yl)-pyridin-2-yl]-piperazine

### Etape a) 4-(5-lodo-pyridin-2-yl)-piperazine-1-carboxylic acid tert-butyl ester

En opérant comme décrit dans la préparation 1, mais en utilisant le 2-fluoro-5-iodo-pyridine au lieu 2-chloro-5(trifluorométhyl)-pyridine et la N-Boc pipérazine au lieu de la cis-2-6 diméthyl pipérazine, on obtient le composé du titre sous forme d'huile

### Etape b) Tert-butyl ester de l'acide 4-[5-(2H-Pyrazol-3-yl)-pyridin-2-yl]-piperazine-1-carboxylique

On charge 0,2 g de composé de l'étape a), 0,069 g de d'acide 1H-pyrazole-5-boronique, 0,03 g de palladium tetrakis, 0,087 g de sodium bicarbonate, 15 mL de DME et 2 ml d'eau. On chauffe à la température de reflux pendant 7 heures. On filtre sur célyte et on évapore le solvant. On isole 0,23 g de brut. Le résidu est purifié par flash chromatographie sur colonne Biotage®, au moyen de l'éluant Hexane 7/éthyle acétate 3. On isole 0,11 g du composè du titre sous forme d'un solide jaune pâle.

### Etape c) Trifluoroacétate de la 1-[5-(2H-Pyrazol-3-yl)-pyridin-2-yl]-piperazine

Le composé de l'étape b) (0,11 g) est dissous lentement dans 3,5 ml d'acide trifluoroacétique à 0°C. On laisse ensuite sous agitation pendant 2 heures à température ambiante. On évapore sous vide l'acide trifluoroacétique et on obtient 0,065 g du composé du titre sous forme d'un solide blanc.

### Préparation 18

### (3S,5R)-3,5-Dimethyl-1-(5-thiazol-2-yl-pyridin-2-yl)-piperazine

### Etape a) (3S,5R)-3,5-Dimethyl-1-(5-lodo-2-yl-pyridin-2-yl)-piperazine

En opérant comme décrit dans la préparation 1, mais en utilisant le 2-fluoro-5-iodo-pyridine au lieu 2-chloro-5(trifluorométhyl)-pyridine, on obtient le composé du titre sous forme d'huile.

### Etape b) Tert-butyl ester de l'acide (2S,6R)-2,6-Dimethyl-4-(5-iodo-2-yl-pyridin-2-yl)-piperazine-1-carboxylique

On charge sous flux d'azote à 0°C : 0,35 g de composé de l'étape a), 0,26 g de (Boc)₂O 0,46 mL de tryéthylamine et 5 ml de DMF. On chauffe à 140°C pendant 4 heures. On évapore le solvant. On isole 0,49 g de brut. Le résidu est purifié par flash chromatographie sur colonne Biotage®, au moyen de l'éluant éthyle acétate. On isole 0,43 g du composé du titre sous forme d'une huile jaune pâle.

### Etape c) Tert-butyl ester de l'acide (2S,6R)-2,6-Dimethyl-4-[5-(4,4,5,5-tetramethyl-[1,3,2]dioxaborolan-2-yl)-pyridin-2-yl]-piperazine-1-carboxylique

On charge dans un ballon sous flux d'azote 0,43 g composé de l'étape b), 0,29 g de bis(pinacole)diboron, 0,026 g de PalladiumCl₂ (dppf)₂·CH₂Cl₂, 0,31 g de potassium acétate, 10 ml de DMSO. On chauffe à 85°C pendant 2 heures. On verse dans une solution acqueuse saturée en NaCl et on extrait avec de l'acétate d'éthyle. La phase organique est séchée sur Na₂SO₄, filtrée et évaporée sous vide. On isole 0,32 g d'une matiere huileuse que l'on purifie par flash chromatographie sur colonne Biotage®, au moyen de l'éluant cyclohexane 9/acétate d'éthyle 1. On isole 0,28 g de solide jaunâtre.

### Etape d) tert-butyl ester de l'acide 2S,6R)-2,6-Dimethyl-4-(5-thiazol-2-yl-pyridin-2-yl)-piperazine-1-carboxylique

On charge dans un ballon sous flux d'azote 0,28 g de de l'étape c) 0,092 g de 2-bromothyazole, 0,032 g de PalladiumTetrakis (PdP(Ph3)4), 0,094 g de bicarbonate de sodium 20 ml de DME et 3 ml d'eau. On chauffe à la température de reflux pendant 7 heures. On verse dans une solution acqueuse saturée en NaCl et on extrait avec de l'acétate d'éthyle. La phase organique est séchée sur Na₂SO₄, filtrée et évaporée sous vide. On isole 0,36 g d'une matière huileuse que l'on purifie par flash chromatographie sur colonne Biotage®, au moyen de l'éluant cyclohexane 9/acétate d'éthyle 1. On isole 0,2 g d'une huile jaunâtre.

### Etape e) Trifluoroacétate de la (3S,5R)-3,5-Dimethyl-1-(5-thiazol-2-yl-pyridin-2-yl)-piperazine

Le composé de l'étape d) (0,2 g) est dissous lentement dans 5 ml d'acide trifluoroacétique à 0°C. On laisse ensuite sous agitation pendant 2 heures à température ambiante. On évapore sous vide l'acide trifluoroacétique et on obtient le 0,15 g du composé du titre sous forme d'un solide beige.

### EXEMPLE 1

### Composé n°9 :

### 2-[(2S,6R)-2,6-Diméthyl-4-(5-trifluorométhyl-pyridin-2-yl)-pipérazin-1-yl]-1(2-phényl-6,7-dihydro-4H-thiazolo[5,4-c]pyridin-5-yl)-éthanone

On fait réagir 0,25 g du composé obtenu à la préparation 6 (composé de formule (II)), 0,22 g du composé obtenu à la préparation 1 (composé de formule (III)), 0,13 g de carbonate de potassium et 0,06 g de Nal dans 4 mL de DMF. La réaction est conduite au moyen d'un initiateur CEMdiscover à micro-ondes pendant 30 mn à 160°C. On verse dans l'eau et on extrait à l'acétate d'éthyle. La phase organique est séchée sur Na₂SO₄, filtrée et évaporée sous vide. On isole 500 mg d'une matière huileuse. On purifie sur colonne par flash chromatographie au moyen d'une colonne Biotage® que l'on élue avec un mélange cyclohexane 8/acétate d'éthyle 2. On isole 400 mg d'un solide jaune pâle que l'on cristallise avec de l'éther éthylique. On filtre et on obtient 0,25 g du produit du titre sous forme d'un solide blanc.
Pf: (169-170)°C
RMN (Appareil b) : δ (ppm, dmso-d6): 1,04 (m, 6H); 2,65 - 2,76 (m, 2H); 2,82 + 2,94 (2 x m, 2H); 3,15 (m, 2H), 3,64 - 3,91 (m, 4H); 4,11 - 4,25 (m, 2H); 4,73 + 4,89 (2 x s, 2H); 6,94 (d, 1 H, J= 9Hz); 7,44 - 7,54 (m, 3H); 7,76 (dd, 1 H, J= 9 et 2Hz); 7,89 (m, 2H); 8,38 (bs, 1 H).

### EXEMPLE 2

### Composé n°18 :

### 2-[(2S,6R)-2,6-Diméthyl-4-(6-trifluorométhyl-pyridin-2-yl)-pipérazin-1-yl]-1-(2-phényl-6,7-dihydro-4H-thiazolo[5,4-c]pyridin-5-yl)-éthanone

En opérant comme décrit dans l'exemple 1, mais en utilisant le composé de la préparation 3 au lieu du composé de la préparation 1 on obtient le composé du titre sous forme d'un solide blanc.
Pf : (168-169)°C
RMN: (Appareil a). δ (ppm, dmso-d6): 1,06 (m, 6H); 2,57 - 2,65 (m, 2H); 2,76 - 3,05 (m, 2H); 3,25 (m, *); 3,65 - 3,92 (m, 6H); 4,75 + 4,90 (2 x bs, 2H); 7,40 (dd, J= 8,9 et 2,4 Hz, 1 H); 7,45 - 7,53 (m, 3H); 7,60 (d, J= 8,8Hz, 1 H); 7,86 - 7,92 (m, 2H); 8,39 (d, J= 2,7Hz, 1 H).

### EXEMPLE 3

### Composé n°19 :

### Méthyl ester de l'acide 6-{3-[2-Oxo-2-(2-phényl-2,4,6,7-tétrahydro-pyrazolo[4,3-c]pyridin-5-yl)-éthyl]-3,8-diaza-bicyclo[3.2.1]oct-8-yl}-nicotinique

On met à réagir 0,45 g du composé obtenu à la préparation 7-I (composé de formule (II)), 0,45 g du composé obtenu à la préparation 4 (composé de formule (III)), 0,6 ml diisopropyl-éthyl-amine et 44 mL de DMF. On chauffe pendant 2 heures à 100°C. On verse dans l'eau et on extrait à l'acétate d'éthyle. La phase organique est séchée sur Na₂SO₄, filtrée et évaporée sous vide. On isole 700 g d'une matière solide. On purifie sur colonne par flash chromatographie au moyen d'une colonne que l'on élue avec un mélange hexane 7/acétate d'éthyle 3. On isole 0,5 g du produit du titre. On traite avec du diéthyl éther, on filtre et on obtient 0,45 d'un solide blanc.
Pf : (204-205)°C
RMN: (Appareil b). δ (ppm, dmso-d6): 1,73 (m, 2H); 1,81 - 2,03 (m, 2H); 2,29 - 2,43 (m, 2H); 2,58 - 2,65 (m, 1 H); 2,66 - 2,76 (m, 2H); 2,89 (m, 1 H); 3,17 (s, 1 H); 3,22 (s, *); 3,94 (m, 4H); 3,86 (m, 1 H); 4,53 - 4,70 (m, 3H); 4,77 (bs, 1 H); 6,77 (m, 1 H); 7,27 (m, 1 H); 7,44 -7,52 (m, 2H); 7,73 -7,79 (m, 2H); 7,92 (m, 1 H); 8,28 + 8,33 (2 x s, 1 H); 8,63 (m, 1 H).

### EXEMPLE 4

### Composé n°26 :

### 2-[8-(5-Fluoro-pyrimidin-2-yl)-3,8-diaza-bicyclo[3.2.1]oct-3-yl]-1-(1-phényl-1,4,6,7-tétrahydro-pyrazolo[4,3-c]pyridin-5-yl)-éthanone

En opérant comme décrit dans l'exemple 3, mais en utilisant le composé de la préparation 7-II au lieu du composé de la préparation 7-I et le composé préparation 2 au lieu du composé de la préparation 4 on obtient le composé du titre sous forme d'un solide blanc.
Pf : (148-150)°C
RMN: (Appareil b). δ (ppm, dmso-d6): 1,74 (m, 2H); 1,79 - 1,99 (m, 2H); 2,31 - 2,45 (m, 2H); 2,61 - 2,75 (m, 2H); 2,84 (m, 1H); 2,98 (m, 1H); 3,18 + 3,22 (2 x s, 2H); 3,74 + 3,83 (2 x m, 2H); 4,47 - 4,65 (m, 3H); 4,71 (s, 1 H); 7,33 - 7,42 (m, 1 H), 7,45 - 7,66 (m, 5H); 8,44 (m, 1 H).

### EXEMPLE 5

### Composé n°24 :

### Acide 6-{3-[2-Oxo-2-(2-phényl-2,4,6,7-tétrahydro-pyrazolo[4,3-c]pyridin-5-yl)-éthyl]-3,8-diaza-bicyclo[3.2.1]oct-8-yl}-nicotinique

On dissous 0,4 g du composé de l'exemple 3 dans 8 ml de solution aqueuse de NaOH à 20% et 8 ml de méthanol. On chauffe à la température de reflux pendant 3 heures. On évapore le méthanol et on lave avec de l'éther éthylique. On ajuste le pH à 6 avec une solution de HCl 1N et on extrait avec de l'acétate d'éthyle. On sèche et on évapore la phase organique et on obtient 200 mg d'une matière hulieuse. On traite avec du diéthyl éther, on filtre et on obtient 0,45g d'un solide blanc correspondant au produit du titre.
Pf : (202-206)°C
RMN: (Appareil a). δ (ppm, dmso-d6): 1,73 (m, 2H); 1,83 - 2,01 (m, 2H); 2,28 - 2,44 (m, 2H); 2,57 - 2,64 (m, 1 H); 2,66 - 2,77 (m, 2H); 2,89 (m, 1 H); 3,16 (s, *); 3,79 + 3,87 (2 x m, 2H); 4,53 - 4,69 (m, 3H); 4,77 (s, 1 H); 6,74 (m, 1 H); 7,27 (m, 1 H); 7,44 - 7,51 (m, 2H); 7,73 - 7,79 (m, 2H); 7,90 (m, 1 H); 8,28 + 8,32 (2 x s, 1 H); 8,62 (m, 1 H); 11,92 - 12,54 (bs, 1 H).

### EXEMPLE 6

### Composé n°11:

### 4-[2-Oxo-2-(2-phényl-6,7-dihydro-4H-thiazolo[5,4-c]pyridin-5-yl)-éthyl]-1-(5-trifluorométhyl-pyridin-2-yl)-pipérazin-2-one;

En opérant comme décrit dans l'exemple 3, mais en utilisant le composé de la préparation 6 au lieu du composé de la préparation 7-I et le composé préparation 5 au lieu du composé de la préparation 4 on obtient le composé du titre sous forme d'un solide blanc.
Pf : (165-166)°C
RMN: (Appareil a). δ (ppm, dmso-d6): 2,80 - 3,03 (m, 4H); 3,40 - 3,59 (m, 4H); 3,72 - 4,05 (m, 7H); 4,79 + 4,88 (2 x s, 2H); 7,39 - 7,55 (m, 3H); 7,74 - 7,94 (m, 2H); 8,05 - 8,35 (m, 2H); 8,82 (s, 0.4H); 8,95 (s, 0.6H).

### EXEMPLE 7

### Composé n°23:

### Chorohydrate du Méthyl ester de l'acide 6-{(3S,5R)-3,5-Diméthyl-4-[2-oxo-2-(2-phényl-2,4,6,7-tétrahydro-pyrazolo[4,3-c]pyridin-5-yl)-éthyl]-pipérazin-1-yl}-nicotinique;

En opérant comme décrit dans l'exemple 1, mais en utilisant le composé de la préparation 7-I au lieu du composé de la préparation 6 et le composé préparation 13 au lieu du composé de la préparation 1, on obtient le composé du titre (base libre). On le dissous dans de l'isopropanol puis on ajoute une solution d'acide chlorydrique dans l'isopropanol. On obtient le produit du titre sous forme de solide blanc.
P.f 210-212
RMN(Appareil a, Temp.A). δ (ppm, dmso-d6): 1.25 + 1.32 (2Xm, 6H), 2.75 - 3.00 (m, 2H), 3.10 - 3.63 (m, *), 3.66 - 4.01 (m, 6H), 4.32 - 4.81 (m, 6H), 7.07 (m, 1 H), 7.29 (m, 1H), 7.49 (m, 2H), 7.78 (m, 2H), 8.06 (m, 1H), 8.27 - 8.40 (m, 1H), 8.71 (m, 1H), 9.19 (bs, 0.5H), 9.44 (bs, 0.5H).

### EXEMPLE 8

### Composé n°21:

### Chlorohydrate de l'acide 6-{(3S,5R)-3,5-Diméthyl-4-[2-oxo-2-(2-phényl-2,4,6,7-tétrahydro-pyrazolo[4,3-c]pyridin-5-yl)-éthyl]-pipérazin-1-yl}-nicotinique

En opérant comme décrit dans l'exemple 5, mais en utilisant le composé de l'exemple 7 au lieu du composé de l'exemple 3, on obtient le composé du titre (base libre)). On le dissous dans de l'isopropanol puis on ajoute une solution d'acide chlorydrique dans l'isopropanol. On obtient le produit du titre sous forme de solide blanc.
P.f 209-211
RMN: (Temp. B). δ (ppm, dmso-d6): 1.24 (d, J= 6.5Hz, 2.7H), 1.32 (d, J= 6.5Hz, 3.3H), 2.80 (m, 0.8H), 2.94 (m, 1.2H), 3.14 - 3.30 (m, 1 H), 3.44 (m, 1 H), 3.49 - 4.26 (m, *), 4.35 - 4.80 (m, 6H), 7.01 - 7.11 (m, 1 H), 7.29 (m, 1 H), 7.49 (m, 2H), 7.73 - 7.84 (m, 2H), 8.05 (m, 1 H), 8.33 (m, 0.8H), 8.39 (m, 0.2H), 8.66 - 8.72 (m, 1 H), 9.28 + 9.44 (2Xbs, 1H), 12.1 - 13.4 (bs, 1H).

### EXEMPLE 9

### Composé n°106:

### 2-(8-Pyridin-3-yl-3,8-diaza-bicyclo[3.2.1]oct-3-yl)-1-[2-(5-trifluoromethylpyridin-2-yl)-2,4,6,7-tetrahydro-pyrazolo[4,3-c]pyridin-5-yl]-ethanone

En opérant comme décrit dans l'exemple 3, mais en utilisant le composé de la préparation 14 au lieu du composé de la préparation 7-I et le composé préparation 15 au lieu du composé de la préparation 4, on obtient le composé du titre sous forme d'un solide blanc.
Pf : (150-151)°C
RMN:(Temp. B). δ (ppm, dmso-d6): 1.69 (m, 2H), 1.83 - 1.97 (m, 2H), 2.34 - 2.66 (m, **), 2.75 (m, 1 H), 2.93 (m, 1 H), 3.11 (s, 1 H), 3.17 (s, 1 H), 3.80 (m, 1 H), 3.88 (m, 1 H), 4.26 (m, 1 H), 4.33 (m, 1 H), 4.59 (s, 1 H), 4.81 (s, 1 H), 7.11 - 7.23 (m, 2H), 7.86 (m, 1 H), 8.04 (m, 1 H), 8.18 (m, 1 H), 8.34 (m, 1 H), 8.53 (s, 0.5H), 8.57 (s, 0.5H), 8.86 (m, 1 H).

### EXEMPLE 10

### Composé n°89:

### 2-{(2S,6R)-2,6-Dimethyl-4-[5-(5-methyl-[1,2,4]oxadiazol-3-yl)-pyridin-2-yl]-piperazin-1-yl}-1-(2-phenyl-2,4,6,7-tetrahydro-pyrazolo[4,3-c]pyridin-5-yl)-ethanone;

En opérant comme décrit dans l'exemple 1, mais en utilisant le composé de la préparation 16 au lieu du composé de la préparation 1 et le composé préparation 7-I au lieu du composé de la préparation 6 on obtient le composé du titre sous forme d'un solide blanc.
Pf : (163-164)°C
RMN:(Temp. B). δ (ppm, dmso-d6): 1.05 (m, 6H), 2.63 (s, 3H), 2.65 - 2.75 (m, 2.7H), 2.84 (m, 1.3H), 3.17 (m, 2H), 3.67 - 3.84 (m, 4H), 4.20 (m, 2H), 4.56 + 4.68 (2Xs, 2H), 6.96 (d, J= 9.0Hz, 1H), 7.27 (m, 1H), 7.47 (m, 2H), 7.76 (m, 2H), 7.99 (dd, J= 9.0 et 2.3Hz, 1 H), 8.29 (s,1 H), 8.67 (d, J= 2.3Hz, 1 H).

### EXEMPLE 11

### Composé n°45:

### Oxalate du 2-[8-(5-Fluoro-pyrimidin-2-yl)-3,8-diaza-bicyclo[3.2.1]oct-3-yl]-1-[1-(2,2,2-trifluoro-ethyl)-4,5,6,7-tetrahydro-1H-indazol-5-yl]-ethanone

En opérant comme décrit dans l'exemple 3, mais en utilisant le composé de la préparation 8 au lieu du composé de la préparation 7-1 et le composé de la préparation 2 au lieu du composé de la préparation 4, on obtient le composé du titre (base libre). On le dissous dans de l'acétone puis on ajoute une solution d'acide oxalique dans l'acétone. On obtient le produit du titre sous forme de solide blanc.
Pf : (160-162)°C
RMN:(Temp. A). δ (ppm, dmso-d6): 1.61 - 1.78 (m, 2H), 1.81 - 1.98 (m, 2H), 2.34 - 2.49 (m, 2H), 2.59 - 2.90 (m, 4H), 3.23 + 3.32 (2Xs, 2H), 3.78 (m, 2H), 4.40 - 4.67 (m, 4H), 5.02 (m, 2H), 7.44 + 7.46 (2Xs, 1 H), 8.44 (m, 2H).

### EXEMPLE 12

### Composé n°130:

### Ethyl ester de l'acide 6-{(3S,5R)-3,5-Diméthyl-4-[2-oxo-2-(2-phényl-2,4,6,7-tétrahydro-pyrazolo[4,3-c]pyridin-5-yl)-éthyl]-pipérazin-1-yl}-nicotinique;

1,88 g du chlorhydrate de la 2-Phenyl-4,5,6,7-tetrahydro-2H-pyrazolo[4,3-c]pyridine est mis en suspension dans un ballon muni d'un agitateur magnétique, dans 123 mL de dichlorométhane. On ajoute 2,5g du composé de la préparation 11, 4,4mL de triéthylamine et 3,5g de BOP. On laisse réagir pendant 1 heure à la température de 20-25°C. Puis, on verse dans l'eau et on extrait avec du dichlorométhane. La phase organique est séchée sur Na₂SO₄, filtrée et évaporée sous vide. On isole 6,07 g d'une matière hulieuse. On la purifie sur colonne par flash chromatographie au moyen d'une colonne Biotage® automatique que l'on élue avec d'éthyl acétate. On isole 1,8 g d'un solide blanc.
RMN : (Appareil b, Temp.B). □ (ppm, dmso-d6): 1.04 (m, 6H), 1.29 (m, 3H), 2.70 (m, 2H), 2.83 + 2.94 (2Xm, 2H), 3.16 (m, 2H), 3.63 - 3.85 (m, 4H), 4.13 - 4.33 (m, 4H), 4.48 - 4.77 (m, 2H), 6.87 (m, 1H), 7.27 (m, 1H), 7.47 (m, 2H), 7.76 (m, 2H), 7.92 (m, 1H), 8.29 (bs, 1H), 8.63 (bs, 1 H).

### EXEMPLE 13

### Composé n°126:

### Oxalate du 2-[(2S,6R)-2,6-Dimethyl-4-(5-trifluoromethyl-pyridin-2-yl)-piperazin-1-yl]-1-[2-(2,2,2-trifluoro-ethyl)-2,4,6,7-tetrahydro-pyrazolo[4,3-c]pyridin-5-yl]-ethanone

En opérant comme décrit dans l'exemple 12, mais en utilisant le composé de la préparation 12 au lieu du composé de la préparation 11 et le composé préparation 10 au lieu du chlorhydrate de la 2-Phenyl-4,5,6,7-tetrahydro-2H-pyrazolo[4,3-c]pyridine, on obtient le composé du titre (base libre). On le dissous dans de l'acétone puis on ajoute une solution d'acide oxalique dans l'acétone. On obtient le produit du titre sous forme de solide blanc.
RMN : (Appareil b, Temp.B). δ (ppm, dmso-d6): 1.11 (m, 6H), 2.57 - 3.07 (m, 4H), 3.10 - 4.73 (m, *), 5.04 (m, 2H), 7.02 (m, 1 H), 7.65 (m, 1 H), 7.82 (m, 1 H), 8.42 (m, 1 H).

### EXEMPLE 14

### Composé n°134

### 1-(2-Phenyl-6,7-dihydro-4H-thiazolo[5,4-c]pyridin-5-yl)-2-{4-[5-(2H-pyrazol-3-yl)-pyridin-2-yl]-piperazin-1-yl}-ethanone

En opérant comme décrit dans l'exemple 3, mais en utilisant le composé préparation 17 au lieu du composé de la préparation 4 et le composé préparation 6 au lieu du composé de la préparation 7-I, on obtient le composé du titre sous forme d'un solide blanc.
Pf : (231-237)°C
RMN : (Appareil a, Temp. B). δ (ppm, dmso-d6): 2.34 - 2.69 (m, *), 2.85 + 3.00 (2xm, 2H), 3.16 - 3.68 (m, **), 3.89 (m, 2H), 4.67 - 5.05 (m, 2H), 6.62 (m, 1 H), 6.89 (m, 1H), 7.49 (m, 3H), 7.74 (m, 1H), 7.83 - 8.01 (m, 3H), 8.56 (m, 1H), 12.68 - 13.26 (m, 1 H).

### EXEMPLE 15

### Composé n°135

### 2-[(2S,6R)-2,6-Dimethyl-4-(5-thiazol-2-yl-pyridin-2-yl)-piperazin-1-yl]-1-(2-phenyl-2,4,6,7-tetrahydro-pyrazolo[4,3-c]pyridin-5-yl)-ethanone

En opérant comme décrit dans l'exemple 1, mais en utilisant le composé de la préparation 7-I au lieu du composé de la préparation 6 et le composé préparation 18 au lieu du composé de la préparation 1 on obtient le composé du titre sous forme de base libre.
Pf (173-174)°C
RMN : (Appareil a, Temp. B). δ (ppm, dmso-d6): 1.05 (m, 6H), 2.67 (m, 3H), 2.84 (m, 1H), 3.17 (m, 2H), 3.63 - 3.87 (m, 4H), 4.18 (m, 2H), 4.50 - 4.74 (m, 2H), 6.93 (d, J= 9.0Hz, 1H), 7.27 (m, 1H), 7.47 (m, 2H), 7.64 (d, J= 3.3Hz, 1 H), 7.76 (m, 2H), 7.83 (d, J= 3.3Hz, 1 H), 8.00 (dd, J= 9.0 et 2.4Hz, 1 H), 8.29 (s, 1 H), 8.66 (d, J= 2.4Hz, 1 H).

Le tableau suivant décrit les exemples obtenus par application et/ou adaptation de méthodes décrites au moyen des réactifs et produits de départ appropriés :

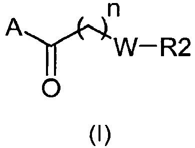

| **N** | **A** | **W** | **R2** | **n** | **Sel** | **PF** | **LCMS** |
|---|---|---|---|---|---|---|---|
| **1** | 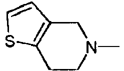 | 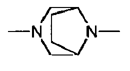 | 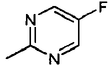 | 1 | -- | 178-180 | MH+ 388 r.t. 4,3' Méthode A |
| **2** | 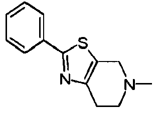 | 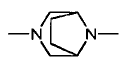 | 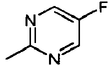 | 1 | -- | 180-181 | MH+ 465 r.t. 5,2' Méthode A |
| **3** | 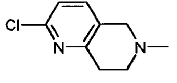 | 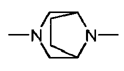 | 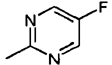 | 1 | -- | 210-212 | M+ = 417 r.t. 4,3' Méthode A |
| **4** | 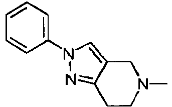 | 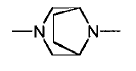 | 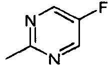 | 1 | -- | 200-201 | M+ = 448 r.t. 4,7' Méthode A |
| **5** | 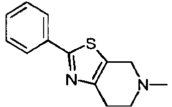 | 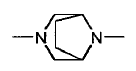 | 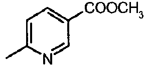 | 1 | -- | 160-162 | M+ = 504 r.t. 5,1' Méthode A |
| **6** | 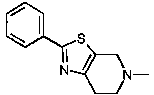 | 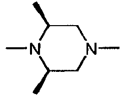 | 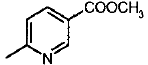 | 1 | HCl | 192-195 | M+ = 506 r.t. 5,2' Méthode A |
| **7** | 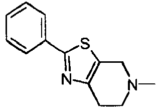 | 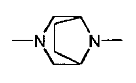 | 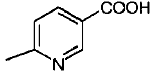 | 1 | -- | 156-157 | M+ = 490 r.t. 4,4' Méthode A |
| **8** | 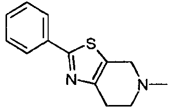 | 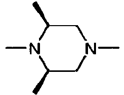 | 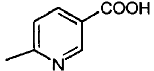 | 1 | HCl | 222-225 | M+ = 492 r.t. 4,7' Méthode A |
| **9** | 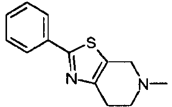 | 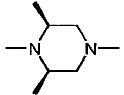 | 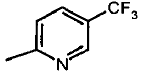 | 1 | -- | 169-170 | M+ = 516 r.t. 5,5' Méthode A |
| **10** | 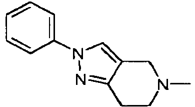 | 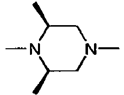 | 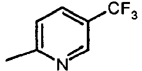 | 1 | -- | 175-177 | M+ = 489 r.t. 5,1' Méthode A |
| **11** | 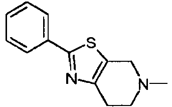 | 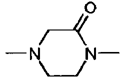 | 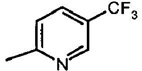 | 1 | -- | 165-166 | M+ = 502 r.t. 7,2' Méthode A |
| **12** | 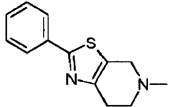 | 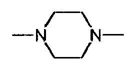 | 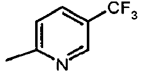 | 1 | -- | 164-165 | M+ = 488 r.t.5,3' Méthode A |
| **13** | 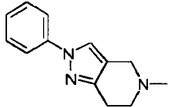 | 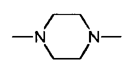 | 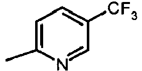 | 1 | -- | 133-134 | M+ = 471 r.t. 5, 0' Méthode A |
| **14** | 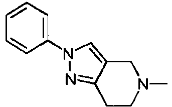 | 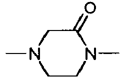 | 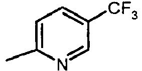 | 1 | -- | 141-142 | M+ = 485 r.t. 6,6' Méthode A |
| **15** | 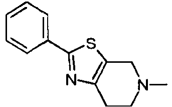 | 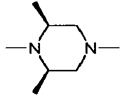 | 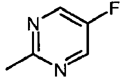 | 1 | -- | 182-184 | M+ = 467 r.t. 4,9' Méthode A |
| **16** | 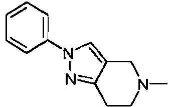 | 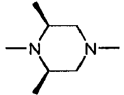 | 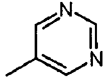 | 1 | HCl | 166-167 | M+ = 432 r.t. 3,9' Méthode A |
| **17** | 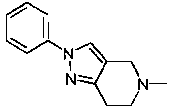 | 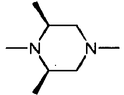 | 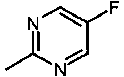 | 1 | -- | 174-175 | M+ = 450 r.t. 4,4' Méthode A |
| **18** | 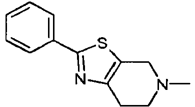 | 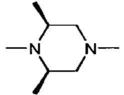 | 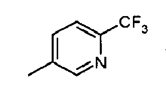 | 1 | -- | 168-169 | M+ = 516 r.t. 5,1' Méthode A |
| **19** | 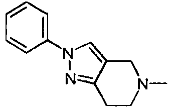 | 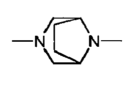 | 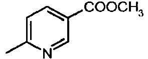 | 1 | -- | 204-205 | M+ = 487 r.t -5, 3' Méthode A |
| **20** | 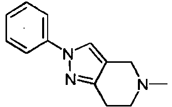 | 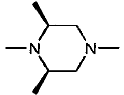 | 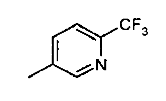 | 1 | -- | 174-175 | M+ = 499 r.t. 4,9' Méthode A |
| **21** | 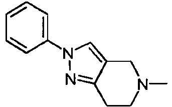 | 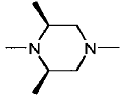 | 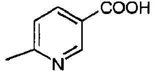 | 1 | HCl | 209-211 | M+ = 475 r.t. 4,3' Méthode A |
| **22** | 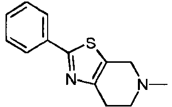 | 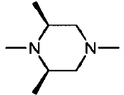 | 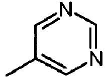 | 1 | -- | 215-216 | M+ = 449 r.t. 3,7' Méthode A |
| **23** | 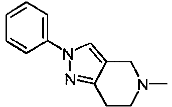 | 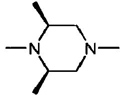 | 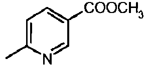 | 1 | HCl | 210-212 | M+ = 489 r.t. 4,8' Méthode A |
| **24** | 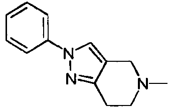 | 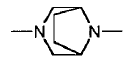 | 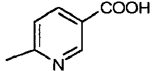 | 1 | -- | 202-206 | M+ = 473 r.t. 4,0' Méthode A |
| **25** | 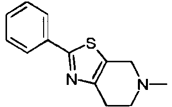 | 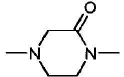 | 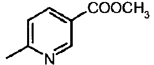 | 1 | -- | 191-192 | M+ = 492 r.t. 5,6' Méthode A |
| **26** | 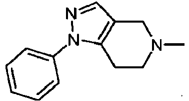 | 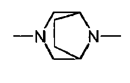 | 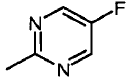 | 1 | -- | 148-150 | M+ = 448 r.t. 4,3' Méthode A |
| **27** | 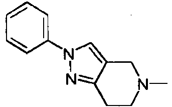 | 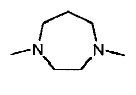 | 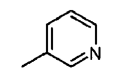 | 1 | -- | -- | M+ = 417 r.t. 3,0' Méthode A |
| **28** | 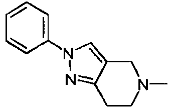 | 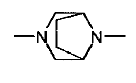 | 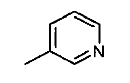 | 1 | -- | 165-168 | M+ = 429 r.t. 3,6' Méthode A |
| **29** | 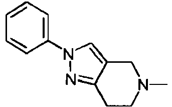 | 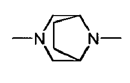 | 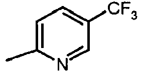 | 1 | -- | 212-213 | M+ = 497 r.t. 5,4' Méthode A |
| **30** | 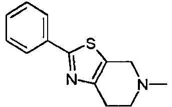 | 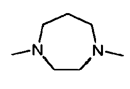 | 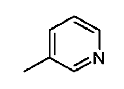 | 1 | HCl | 142-145 | M+ = 434 r.t. 3,2' Méthode A |
| **31** | 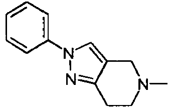 | 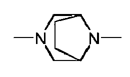 | 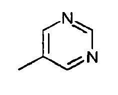 | 1 | -- | 197-198 | M+ = 430 r.t. 3,7' Méthode A |
| **32** | 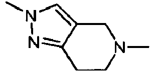 | 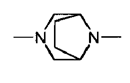 | 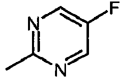 | 1 | -- | 147-149 | M+ = 386 r.t. 3,3' Méthode A |
| **33** | 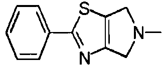 | 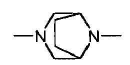 | 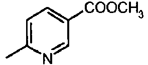 | 1 | -- | 252-253 | M+ = 490 r.t. 4,9' Méthode A |
| **34** | 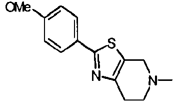 | 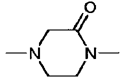 | 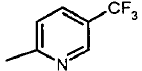 | 1 | -- | 191-193 | M+ = 532 r.t. 6,2' Méthode A |
| **35** | 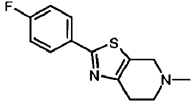 | 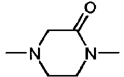 | 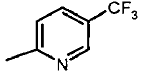 | 1 | -- | 173-175 | M+ = 532 r.t. 6, 3' Méthode A |
| **36** | 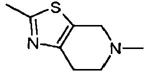 | 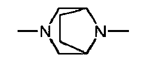 | 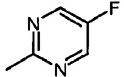 | 1 | -- | 137-138 | M+ = 403 r.t. 3,6' Méthode A |
| **37** | 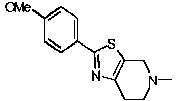 | 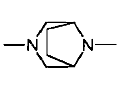 | 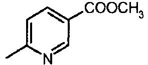 | 1 | -- | 159-160 | M+ = 534 r.t. 5,1' Méthode A |
| **38** | 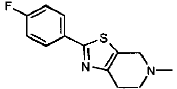 | 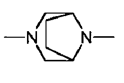 | 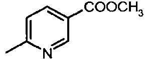 | 1 | -- | 192-193 | M+ = 523 r.t. 5,3' Méthode A |
| **39** | 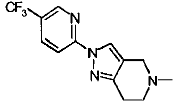 | 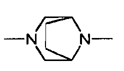 | 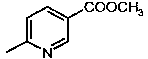 | 1 | -- | 217-218 | M+ = 556 r.t. 5,4' Méthode A |
| **40** | 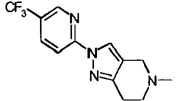 | 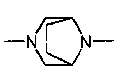 | 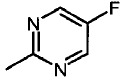 | 1 | -- | 208-209 | M+ = 517 r.t. 5,1' Méthode A |
| **41** | 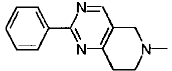 | 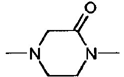 | 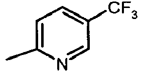 | 1 | -- | | M+ = 497 r.t. 6,0' Méthode A |
| **42** | 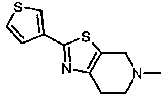 | 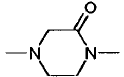 | 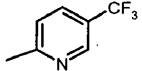 | 1 | -- | 179-180 | M+ = 508 r.t. 6,0' Méthode A |
| **43** | 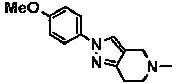 | 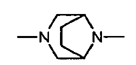 | 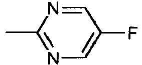 | 1 | -- | 186-188 | M+ = 478 r.t. 1,48' Méthode A |
| **44** | 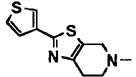 | 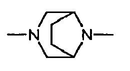 | 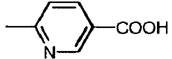 | 1 | -- | 262-264 | M+ = 496 r.t. 4,19' Méthode A |
| **45** | 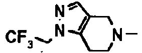 | 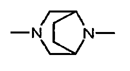 | 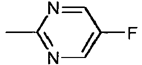 | 1 | oxalate | 160-162 | M+ = 454 r.t. 3,81' Méthode A |
| **46** | 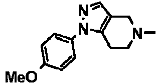 | 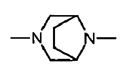 | 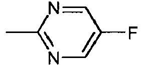 | 1 | -- | 194-195 | M+ = 478 r.t. 4,3' Méthode A |
| **47** | 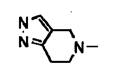 | 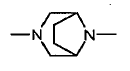 | 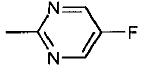 | 1 | -- | 122-123 | M+ = 372 r.t. 3,17' Méthode A |
| **48** | 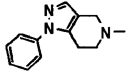 | 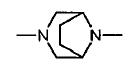 | 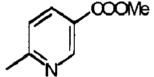 | 1 | -- | -- | M+ = 487 r.t. 4,65' Méthode A |
| **49** | 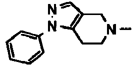 | 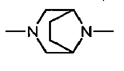 | 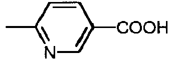 | 1 | -- | -- | M+ = 473 r.t. 3,95 Méthode A |
| **50** | 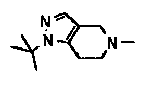 | 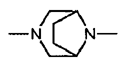 | 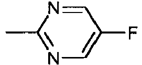 | 1 | -- | 142-143 | M+ = 428 r.t. 4,05' Méthode A |
| **51** | 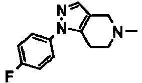 | 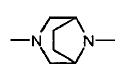 | 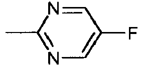 | 1. | -- | 151-152 | M+ = 466 r.t. 4,29' Méthode A |
| **52** | 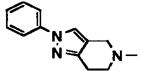 | 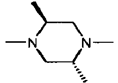 | 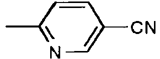 | 1 | -- | 156-159 | M+ = 456 r.t. 4,61' Méthode A |
| **53** | 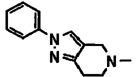 | 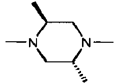 | 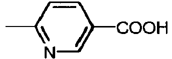 | 1 | -- | 165-167 | M+ = 475 r.t. 4,05' Méthode A |
| **54** | 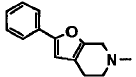 | 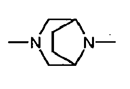 | 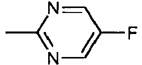 | 1 | -- | 165-167 | M+ = 448 r.t. 5,54 Méthode A |
| **55** | 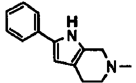 | 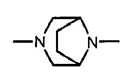 | 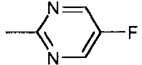 | 1 | -- | 240-242 | M+ = 447 r.t. 5,11 Méthode A |
| **56** | 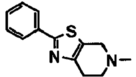 | 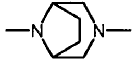 | 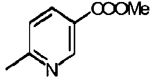 | 1 | -- | 162-163 | M+ = 504 r.t. 4,69' Méthode A |
| **57** | 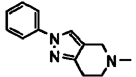 | 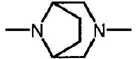 | 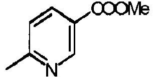 | 1 | -- | 157-158 | M+ = 487 r.t. 4,39' Méthode A |
| **58** | 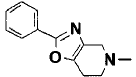 | 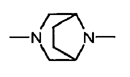 | 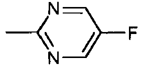 | 1 | -- | 88-89 | M+ = 449 r.t. 4,67' Méthode A |
| **59** | 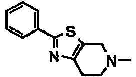 | 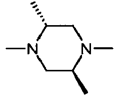 | 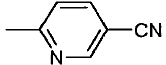 | 1 | -- | 159-160 | M+ = 473 r.t. 4,8' Méthode A |
| **60** | 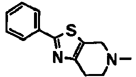 | 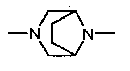 | 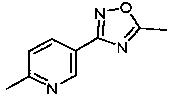 | 1 | -- | 190-192 | M+ = 528 r.t. 5,1' Méthode A |
| **61** | 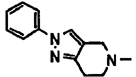 | 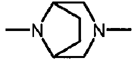 | 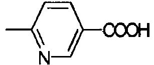 | 1 | -- | 207-208 | M+ = 473 r.t. 3,82' Méthode A |
| **62** | 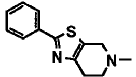 | 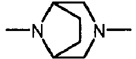 | 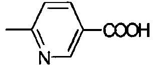 | 1 | -- | 250-251 | M+ = 490 r.t. 4,09' Méthode A |
| **63** | 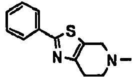 | 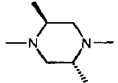 | 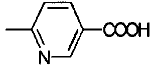 | 1 | -- | 220-221 | M+ = 490 r.t. 4,27' Méthode A |
| **64** | 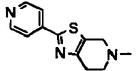 | 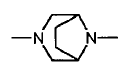 | 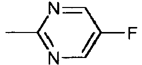 | 1 | -- | 193-194 | M+ = 466 r.t. 3,48 Méthode A |
| **65** | 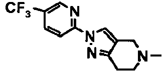 | 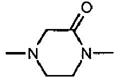 | 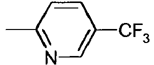 | 1 | -- | 200-201 | M+ = 554 r.t. 6,88' Méthode A |
| **66** | 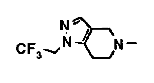 | 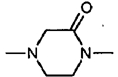 | 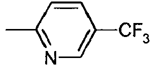 | 1 | oxalate | 82-83 | M+ = 491 r.t. 5,31' Méthode A |
| **67** | 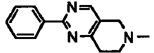 | 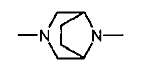 | 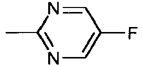 | 1 | -- | 212-213 | M+ = 460 r.t. 4,75' Méthode A |
| **68** | 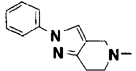 | 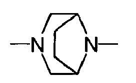 | 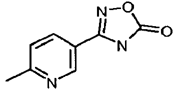 | 1 | -- | 176-179 | M+ = 513 r.t. 4,38' Méthode A |
| **69** | 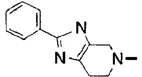 | 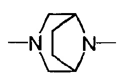 | 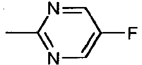 | 1 | -- | 130-132 | M+ = 448 r.t. 3,26' Méthode A |
| **70** | 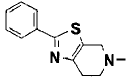 | 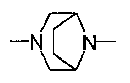 | 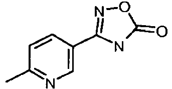 | 1 | -- | 215-218 | M+ = 530 r.t. 4,65' Méthode A |
| **71** | 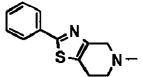 | 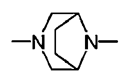 | 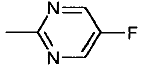 | 1 | -- | 180-184 | M+ = 465 r.t. 5,02' Méthode A |
| **72** | 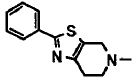 | 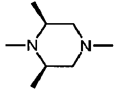 | 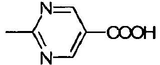 | 1 | sodium salt | 269-272 | M+ = 493 r.t. 4,24' Méthode A |
| **73** | 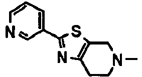 | 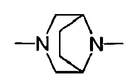 | 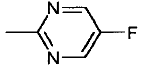 | 1 | -- | 200-202 | M+ = 466 r.t. 3,76' Méthode A |
| **74** | 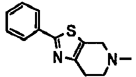 | 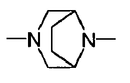 | 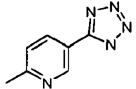 | 1 | -- | 201-202 | M+ = 514 r.t. 4,4' Méthode A |
| **75** | 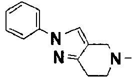 | 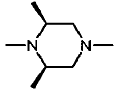 | 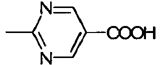 | 1 | -- | 94-97 | M+ = 476 r.t. 3,9' Méthode A |
| **76** | 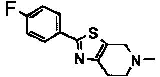 | 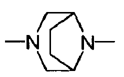 | 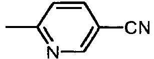 | 1 | -- | 212-213 | M+ = 489 r.t. 5,21' Méthode A |
| **77** | 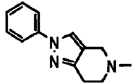 | 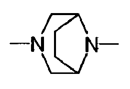 | 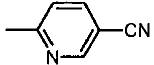 | 1 | -- | 198-200 | M+ = 454 r.t. 4,53' Méthode A |
| **78** | 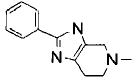 | 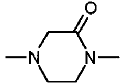 | 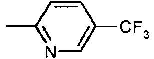 | 1 | -- | 198-200 | M+ = 485 r.t. 3,94' Méthode A |
| **79** | 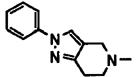 | 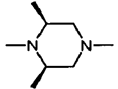 | 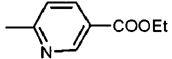 | 1 | HCl | 235-236 | M+ = 503 r.t. 4,6' Méthode A |
| **80** | 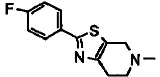 | 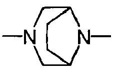 | 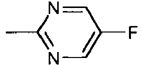 | 1 | -- | 196-198 | M+ = 483 r.t. 4,9' Méthode A |
| **81** | 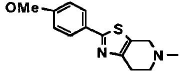 | 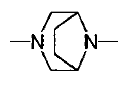 | 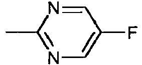 | 1 | -- | 203-207 | M+ = 495 r.t. 4,82' Méthode A |
| **82** | 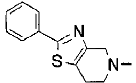 | 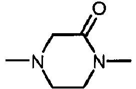 | 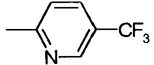 | 1 | -- | 140-142 | M+ = 502 r.t. 6,26 Méthode A |
| **83** | 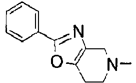 | 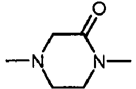 | 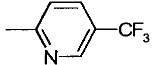 | 1 | -- | 140-142 | M+ = 486 r.t. 6,33' Méthode A |
| **84** | 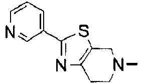 | 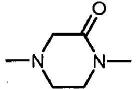 | 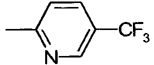 | 1 | -- | 180-183 | M+ = 503 r.t. 5,03' Méthode A |
| **85** | 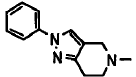 | 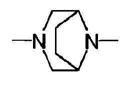 | 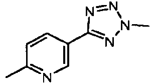 | 1 | -- | 230-231 | M+ = 511 r.t. 4,42' Méthode A |
| **86** | 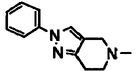 | 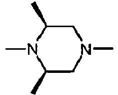 | 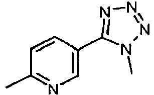 | 1 | -- | 198-199 | M+ = 513 r.t. 4,05 Méthode A |
| **87** | 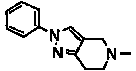 | 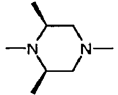 | 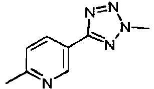 | 1 | -- | 182-183 | M+ = 513 r.t. 4,3' Méthode A |
| **88** | 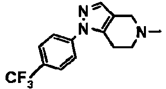 | 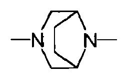 | 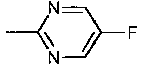 | 1 | -- | 162-164 | M+ = 516 r.t. 5,08' Méthode A |
| **89** | 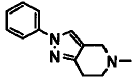 | 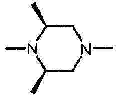 | 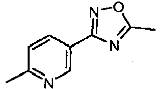 | 1 | -- | 163-164 | M+ = 513 r.t. 4,48' Méthode A |
| **90** | 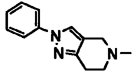 | 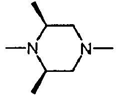 | 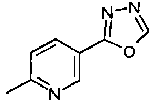 | 1 | -- | 189-190 | M+ = 499 r.t. 4,03' Méthode A |
| **91** | 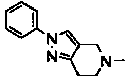 | 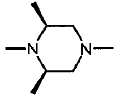 | 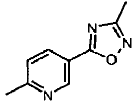 | 1 | -- | 168-171 | M+ = 513 r.t. 4,48 Méthode A |
| **92** | 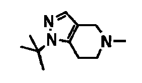 | 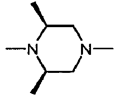 | 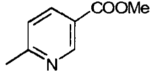 | 1 | -- | 134-135 | M+ = 469 r.t. 4,06' Méthode A |
| **93** | 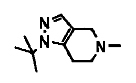 | 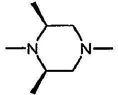 | 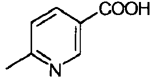 | 1 | -- | 150-151 | M+ = 455 r.t. 3,61' Méthode A |
| **94** | 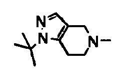 | 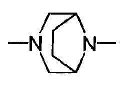 | 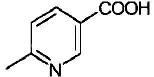 | 1 | -- | -- | M+ = 453 r.t. 3,67' Méthode A |
| **95** | 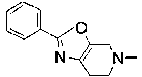 | 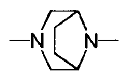 | 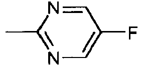 | 1 | -- | 193 | M+ = 449 r.t. 4,62' Méthode A |
| **96** | 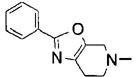 | 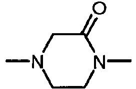 | 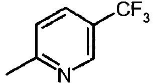 | 1 | -- | 175 | M+ = 486 r.t. 5,96' Méthode A |
| **97** | 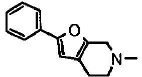 | 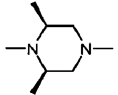 | 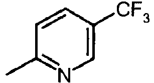 | 1 | -- | 162 | M+ = 499 r.t. 5,66' Méthode A |
| **98** | 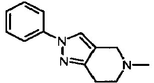 | 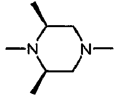 | 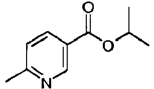 | 1 | -- | 135-136 | M+ = 517 r.t. 4,87' Méthode A . |
| **99** | 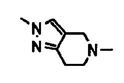 | 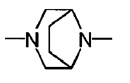 | 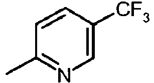 | 1 | -- | 70-71 | M+ = 435 r.t. 4,06 Méthode A |
| **100** | 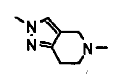 | 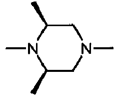 | 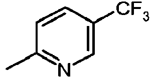 | 1 | HCl | 50-51 | M+ = 437 r.t. 3,93' Méthode A |
| **101** | 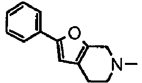 | 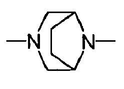 | 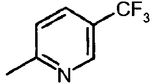 | 1 | -- | 155 | M+ = 497 r.t. 6,21' Méthode A |
| **102** | 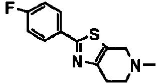 | 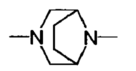 | 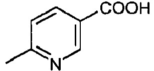 | 1 | -- | 240-244 | M+ = 508 r.t. 4,56 Méthode A |
| **103** | 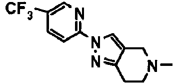 | 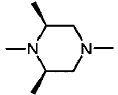 | 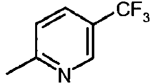 | 1 | -- | 190-191 | M+ = 568 r.t. 5,55' Méthode A |
| **104** | 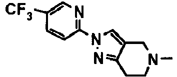 | 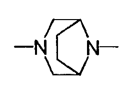 | 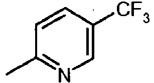 | 1 | -- | 192-193 | M+ = 566 r.t. 5,91' Méthode A |
| **105** | 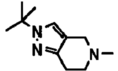 | 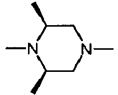 | 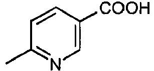 | 1 | -- | 120-121 | M+ = 455 r.t. 3,53' Méthode A |
| **106** | 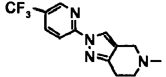 | 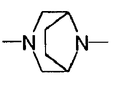 | 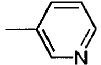 | 1 | -- | 150-151 | M+ = 498 r.t. 3,99' Méthode A |
| **107** | 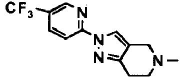 | 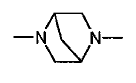 | 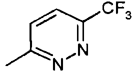 | 1 | -- | 140-141 | M+ = 553 r.t. 4,71' Méthode A |
| **108** | 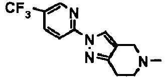 | 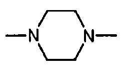 | 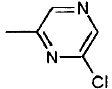 | 1 | -- | 185-186 | M+ = 507 r.t. 4,77' Méthode A |
| **109** | 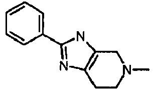 | 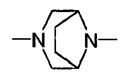 | 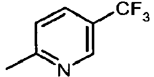 | 1 | -- | 150-152 | M+ = 497 r.t. 3.91' Méthode A |
| **110** | 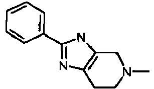 | 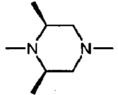 | 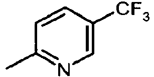 | 1 | -- | 189-192 | M+ = 499 r.t. 3,75' Méthode A |
| **111** | 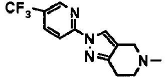 | 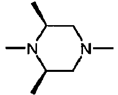 | 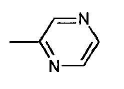 | 1 | -- | 150-151 | M+ = 501 r.t. 4,59' Méthode A |
| **112** | 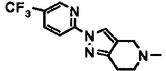 | 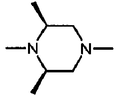 | 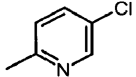 | 1 | -- | 165-166 | M+ = 534 r.t. 5,39' Méthode A |
| **113** | 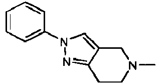 | 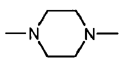 | 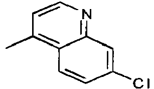 | 1 | -- | 100-101 | M+ = 487 r.t. 3,7' Méthode A |
| **114** | 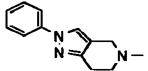 | 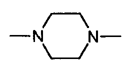 | 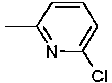 | 1 | -- | 155-156 | M+ = 437 r.t. 4,46' Méthode A |
| **115** | 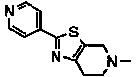 | 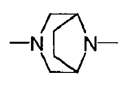 | 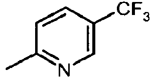 | 1 | -- | 183-185 | M+ = 515 r.t. 4,35' Méthode A |
| **116** | 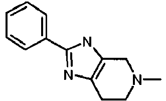 | 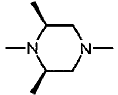 | 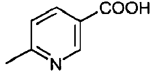 | 1 | -- | 190-192 | M+ = 475 r.t. 2,86' Méthode A |
| **117** | 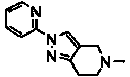 | 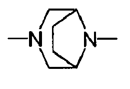 | 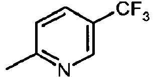 | 1 | -- | 170-171 | M+ = 498 r.t.5' Méthode A |
| **118** | 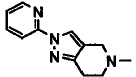 | 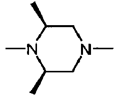 | 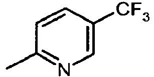 | 1 | -- | 165-166 | M+ = 500 r.t. 4,79' Méthode A |
| **119** | 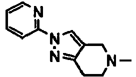 | 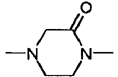 | 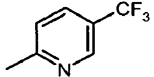 | 1 | -- | 139-140 | M+ = 486 r.t. 5,23' Méthode A |
| **120** | 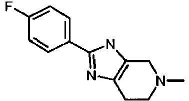 | 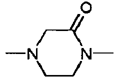 | 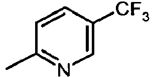 | 1 | -- | 190-192 | M+ = 503 r.t. 4.11' Méthode A |
| **121** | 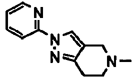 | 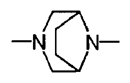 | 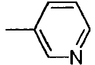 | 1 | -- | 61-62 | M+ = 430 r.t. 3.09' Méthode A |
| **122** | 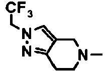 | 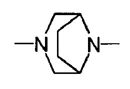 | 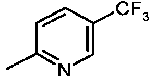 | 1 | -- | -- | M+ = 503 r.t. 4.68' Méthode A |
| **123** | 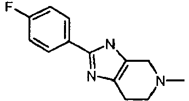 | 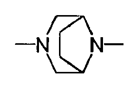 | 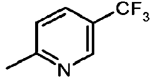 | 1 | -- | 148-150 | M+ = 515 r.t. 4.08' Méthode A |
| **124** | 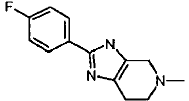 | 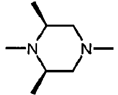 | 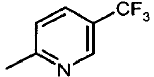 | 1 | -- | 145-147 | M+ = 517 r.t. 3.77' Méthode A |
| **125** | 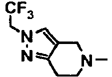 | 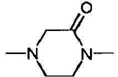 | 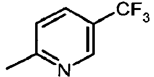 | 1 | -- | -- | M+ = 491 r.t. 4.89' Méthode A |
| **126** | 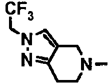 | 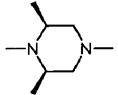 | 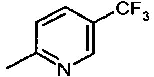 | 1 | oxalate | 151-153 | M+ = 505 r.t. 4.54' Méthode A |
| **127** | 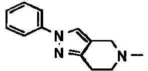 | 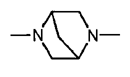 | 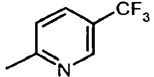 | 1 | -- | 174-175 | M+ = 483 r.t. 4.59' Méthode A |
| **128** | 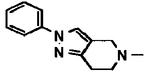 | 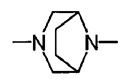 | 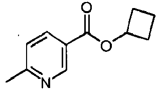 | 1 | -- | 176-177 | M+ = 527 r.t. 5.34' Méthode A |
| **129** | 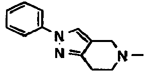 | 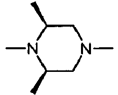 | 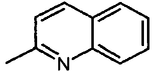 | 1 | -- | 203-204 | M+ = 481 r.t. 4.17' Méthode A |
| **130** | 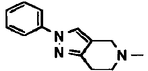 | 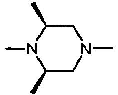 | 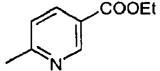 | 1 | -- | -- | M+ = 503 r.t. 4.66' Méthode A |
| **131** | 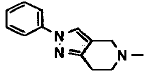 | 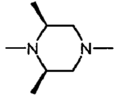 | 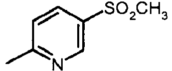 | 1 | -- | 103-105 | M+ = 509 r.t. 4.07' Méthode A |
| **132** | 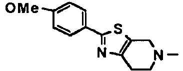 | 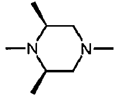 | 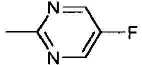 | 1 | -- | 159-161 | M+ = 497 r.t. 4.69' Méthode A |
| **133** | 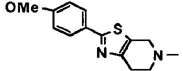 | 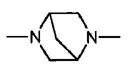 | 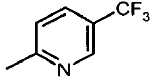 | 1 | -- | 70-73 | M+ = 530 r.t. 4.97' Méthode A |
| **134** | 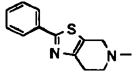 | 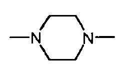 | 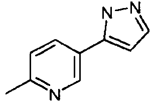 | 1 | -- | 231-237 | M+ = 486 r.t. 4.13' Méthode A |
| **135** | 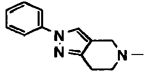 | 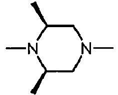 | 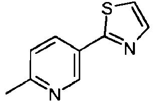 | 1 | -- | 173-174 | M+ = 514 r.t. 4.56' Méthode A |
| **136** | 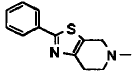 | 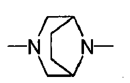 | 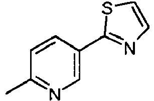 | 1 | -- | | M+ = 529 r.t. 5.04' Méthode A |
| **137** | 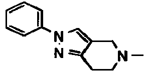 | 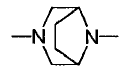 | 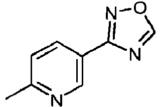 | 1 | -- | 201-202 | M+ = 497 r.t. 4.45' Méthode A |
| **138** | 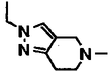 | 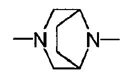 | 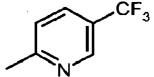 | 1 | -- | 99-100 | M+ = 449 r.t. 4.37' Méthode A |
| **139** | 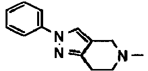 | 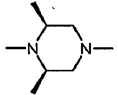 | 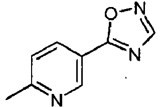 | 1 | -- | -- | M+ = 499 r.t. 4.21' Méthode A |
| **140** | 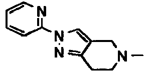 | 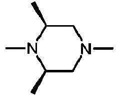 | 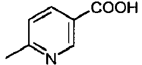 | 1 | -- | 147-150 | M+ = 476 r.t. 4.0' Méthode B |
| **141** | 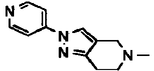 | 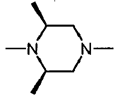 | 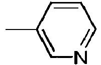 | 1 | -- | 163-164 | M+ = 432 r.t. 4.56' Méthode B |
| **142** | 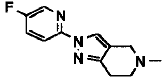 | 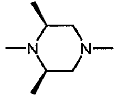 | 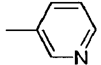 | 1 | -- | 180-181 | M+ = 450 r.t. 3.14' Méthode A |
| **143** | 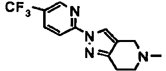 | 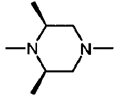 | 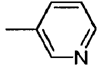 | 1 | -- | 196-197 | M+ = 500 r.t. 6.29' Méthode B |
| **144** | 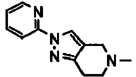 | 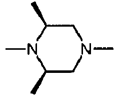 | 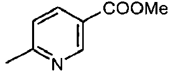 | 1 | -- | -- | M+ = 490 r.t. 4.22' Méthode A |
| **145** | 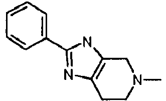 | 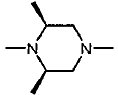 | 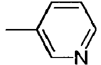 | 1 | -- | 207-209 | M+=431 r.t. 4.69' Méthode B |
| **146** | 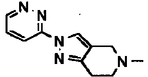 | 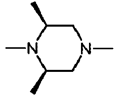 | 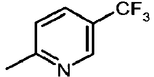 | 1 | -- | -- | M+ = 501 r.t. 4.32' Méthode A |
| **147** | 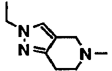 | 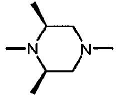 | 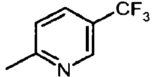 | 1 | -- | -- | M+ = 451 r.t. 4.35' Méthode A |
| **148** | 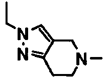 | 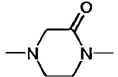 | 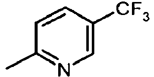 | 1 | -- | 151-152 | M+ = 437 r.t. 4.44' Méthode A |
| **149** | 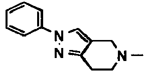 | 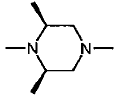 | 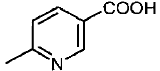 | 1 | -- | 208-209 | M+ = 475 r.t. 3.98' Méthode A |
| **150** | 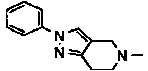 | 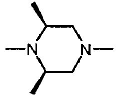 | 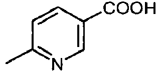 | 1 | 2HCl 2H2O | 252-253 | M+ = 475 r.t. 3.95' Méthode A |

Les composés selon l'invention ont fait l'objet d'études biochimiques.

### Culture cellulaire :

La souche SH-SY-5Y (neuroblastome humain) est cultivée classiquement dans un milieu de culture DMEM (de l'anglais Dulbecco's Modified Eagle's Medium) (Gibco BRL, France) contenant du SVF (5%) (sérum de veau foetal) (Boehringer Mannheim, Allemagne), du pyruvate de sodium (1 mM) et de la glutamine (4 mM) dans des flacons de culture recouvert de collagène (Becton Dickinson, France).

La souche mère SK-N-BE (neuroblastome humain) et le clone Bep 75, exprimant de manière stable la forme entière du récepteur p75^{NTR} humain (SK-N-BE Bep 75) sont cultivés classiquement dans un milieu de culture RPMI contenant du SVF (5%), du pyruvate de sodium (1 mM) et de la glutamine (4 mM). Pour les cellules SK-N-BE Bep 75, on ajoute de l'hygromycine (200 µl/20ml milieu) comme agent de sélection.

### Etude de la dimérisation du récepteur p75^{NTR} indépendamment de son ligand

L'étude de la dimérisation du récepteur p75^{NTR} est réalisée sur une suspension cellulaire de la souche SK-N-BE Bep 75. Les cellules (2,5 10⁴ cellules/puits) sont disposées dans des puits (plaque de 96 puits) pendant 24 h, puis pré-incubées pendant 1h à 37°C en présence ou non des composés selon l'invention. On ajoute ensuite du surnageant, issu de culture de cellules humaines d'origine rénale HEK293 exprimant, après 48h de transfection, et sécrétant une forme soluble du récepteur p75^{NTR} (partie extracellulaire du récepteur) couplée à une phosphatase alcaline, ce à la concentration finale de 10 nM. La quantification de la liaison spécifique du récepteur soluble p75^{NTR} au récepteur présent sur cellules SK-N-BE Bep 75 est déterminée par la mesure de l'activité enzymatique de la phosphatase alcaline après incubation des cellules pendant 1 heure à 37°C en présence du surnageant. Après filtration et transfert des filtres dans des plaques de 24 puits, l'activité de la phosphatase alcaline est déterminée par ajout de substrat chimioluminescent CDP-Star (ready-to-use, Roche). Les concentrations inhibitrices de 50 %(Cl₅₀) de la dimérisation du récepteur p75^{NTR} des composés selon l'invention sont faibles et varient de 10⁻⁶ à 10⁻¹¹M.

Par exemple, les composés n° 9, 11, 19 et 24 ont montré respectivement une Cl₅₀ de 0,73 nM, 1,9 nM, 14 nM et 1,55 nM.

### Mesure de l'apoptose

Les cellules (souches de neuroblastomes humains SH-SY-5Y et SK-N-BE Bep 75) sont installées dans des boîtes de Pétri de 35 mm de diamètre (Biocoat collagenl, (10⁵ cellules/puits)) dans un milieu de culture approprié contenant 5 % de SVF durant 24H. Le milieu de culture est ensuite éliminé, les cellules sont rincées avec du PBS (de l'anglais Dulbecco's Phosphate buffered saline), puis on ajoute soit du milieu frais contenant 5% de SVF, soit du milieu contenant du NGF (à la concentration de 10 ng/ml), soit du peptide beta-amyloïde (Aβ1-40) (à la concentration de 10 µM), ceci en présence ou non des composés selon l'invention. Les taux d'apoptose sont mesurés 48 heures après les traitements dans le cas de la souche SH-SY-5Y, et 24 heures après dans le cas de la souche SK-N-BE Bep 75 par quantification des histones cytoplasmiques associés aux fragments d'ADN (cell death détection ELISA, Boehringer Mannheim, Allemagne). Les taux d'apoptose sont exprimés en quantité d'oligonucléosomes/10⁵ cellules. Chaque valeur correspond à la moyenne de 9 points expérimentaux répartis dans 3 expériences indépendantes.

Les composés de formule (I) présentent une activité inhibitrice de l'apoptose induite par le NGF avec des Cl₅₀ qui varient de de10⁻⁶ à 10⁻¹¹M.

Par exemple, les composés n° 9 et 11 ont montré respectivement une Cl₅₀ de 0,72 nM, et 4,46 nM.

Ainsi, la fixation des composés selon l'invention au récepteur p75^{NTR} se traduit, d'une part, au niveau biochimique par l'inhibition de la dimérisation du récepteur induit par les neurotrophines, ou indépendamment du ligand, et, d'autre part, au niveau cellulaire par l'inhibition de l'effet proapoptotique médié par le récepteur p75^{NTR}.

Ainsi, selon un des objets de la présente invention, les composés de formule (I) présentent une activité très intéressante d'inhibition de la dimérisation du récepteur p75^{NTR} indépendamment de son ligand.

Les composés selon l'invention peuvent donc être utilisés pour la préparation de médicaments, en particulier de médicaments destinés à prévenir ou à traiter toute pathologie où le récepteur p75^{NTR} est impliqué, plus particulièrement celles indiquées ci-après.

Les composés selon l'invention peuvent également être utilisés pour prévenir ou traiter toute pathologie où le récepteur p75^{NTR} est impliqué, plus particulièrement celles indiquées ci-après.

Ainsi, selon un autre de ses aspects, l'invention a pour objet des médicaments qui comprennent un composé de formule (I), ou un sel d'addition de ce dernier à un acide pharmaceutiquement acceptable.

Ainsi, les composés selon l'invention peuvent être utilisés, chez l'homme ou chez l'animal, dans le traitement ou la prévention de différentes affections p75^{NTR} dépendantes telles que les maladies neurodégénératives centrales et périphériques comme la démence sénile, l'épilepsie, la maladie d'Alzheimer, la maladie de Parkinson, la chorée d'Huntington, le syndrome de Down, les maladies à prion, l'amnésie, la schizophrénie, la dépression, le désordre bipolaire; la sclérose latérale amyotrophique, la sclérose en plaques ; les affections cardiovasculaires comme les dommages cardiaques post-ischémiques, les cardiomyopathies, l'infarctus du myocarde, l'insuffisance cardiaque, l'ischémie cardiaque, l'infarctus cérébral ; les neuropathies périphériques (d'origine diabétique, traumatisme ou iatrogène) ; les dommages au nerf optique et à la rétine (dégénérescence du pigment rétinien, glaucome) ; l'ischémie rétinale ; la dégénérescence maculaire ; les traumatismes de la moelle épinière et les traumatismes crâniens ; l'athérosclérose ; les sténoses ; les désordres de la cicatrisation ; l'alopécie.

Les composés selon l'invention peuvent également être utilisés dans le traitement de la pancréatite, et de la fibrose hépatique.

Les composés selon l'invention peuvent également être utilisés dans le traitement des cancers comme celui du poumon, de la thyroïde, du pancréas, de la prostate, de l'intestin grêle et du colon, du sein, dans le traitement des tumeurs, des métastases et des leucémies.

Les composés selon l'invention peuvent également être utilisés dans le traitement des désordres respiratoires comme l'inflammation pulmonaire, l'allergie et l'asthme, la broncho-pneumopathie chronique obstructive.

Les composés selon l'invention peuvent aussi être utilisés dans le traitement de la douleur cutanée (de la peau, des tissus sous-cutanés et organes associés), somatique, viscérale (au niveau du système circulatoire, respiratoire, gastro-intestinal, ou génito-urinaire), et neurologiques.

Les composés selon l'invention peuvent être utilisés dans le traitement des douleurs chroniques neuropathiques et inflammatoires, et dans le traitement des maladies auto-immunes comme la polyarthrite rhumatoïde.

Les composés selon l'invention peuvent aussi être utilisés dans le traitement des maladies comme la spondylarthrite ankylosante, le rhumatisme psoriasique, le psoriasis en plaques.

Les composés selon l'invention peuvent également être utilisés dans le traitement des fractures osseuses, dans le traitement ou la prévention des maladies osseuses comme l'ostéoporose.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de l'Homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel peut être administré sous forme unitaire d'administration, en mélange avec des excipients pharmaceutiques classiques, aux animaux et aux êtres humains pour la prévention ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intraauriculaire, intranasale, par inhalation, les formes d'administration topique, parentérale telle que transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

La dose de principe actif administrée par jour peut atteindre 0,01 à 100 mg/kg, en une ou plusieurs prises, préférentiellement 0,02 à 50 mg/kg. En général, la dose journalière du composé de l'invention sera la dose efficace la plus faible du composé capable de produire un effet thérapeutique.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

La présente invention, selon un autre de ses aspects, concerne également une méthode de traitement des pathologies ci-dessus indiquées qui comprend l'administration, à un patient, d'une dose efficace d'un composé selon l'invention, ou un de ses sels pharmaceutiquement acceptables.

## Revendications

1. Composé répondant à la formule (I) : 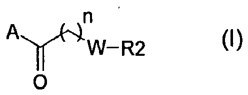 dans laquelle:
- A représente un groupe : 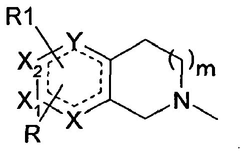
- n représente 1 ou 2 ;
- m représente 0 ou 1 ;
- Y représente un atome de carbone, d'azote, de soufre ou d'oxygène ou une liaison simple ou double ;
- X, X₁ et X₂ représentent un atome de carbone, d'azote, de soufre ou d'oxygène, étant entendu qu'au moins un des X, X₁, X₂ est différent d'un atome de carbone ;
- R et R1, situés sur l'une quelconque des positions disponibles, représentent indépendamment un atome d'hydrogène, un atome d'halogène, un groupe (C1-C4)alkyle, (C1-C4)alkoxy, un radical perfluoroalkyle, trifluorométhoxy, un cyano, un groupe COOH, COOalkyle, CONR5R6 ou NHCOR5 ; ou R1 représente un groupe choisi parmi : 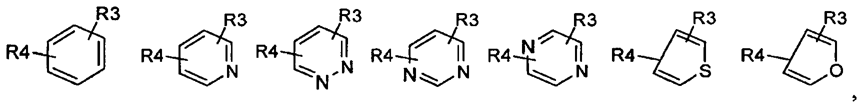 la définition de R restant inchangée;
- R3 et R4, situés sur l'une quelconque des positions disponibles, représentent indépendamment un atome d'hydrogène, un atome d'halogène, un groupe (C1-C4)-alkyle, (C1-C4)alkoxy, un radical perfluoroalkyle, trifluorométhoxy, un cyano, un groupe COOH, COOalkyle, CONR5R6 ou NHCOR5 ;
- - W- est un hétérocycle azoté choisi parmi: 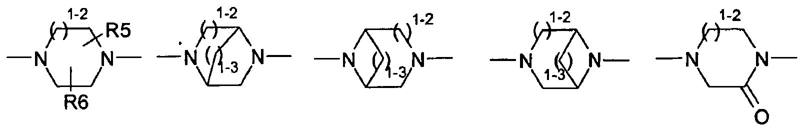
- 1-2 représente 1 ou 2 ;
- 1-3 représente 1, 2 ou 3 ;
- R2 représente un groupe de formule : 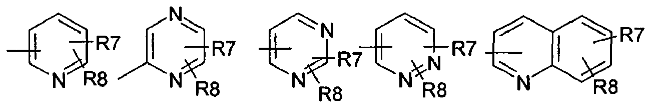
- R7 et R8, situés sur l'une quelconque des positions disponibles, représentent indépendamment un atome d'hydrogène, un atome d'halogène, un groupe (C1-C4) alkyle, (C1-C4)alkoxy, un radical trifluorométhyle, trifluorométhoxy, un cyano, un groupe COOH, COOalkyle, COOcycloalkyle, SOalkyle, SO₂alkyle; CONH2, CONR5R6 ou NHCOR5 ; ou un des R7 et R8 représente un hétérocycle choisi parmi : 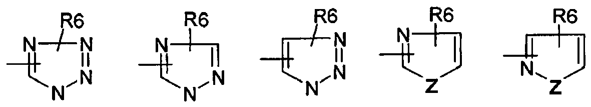 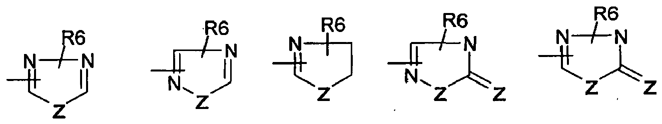
- Z représente un atome d'oxygène ou de soufre;
- R5 et R6 représentent un hydrogène ou un groupe (C1-C6)-alkyle.
à l'état de base ou de de sel d'addition à un acide.

2. Composé selon la revendication 1 tel que :
- W est un groupe de formule choisi parmi: 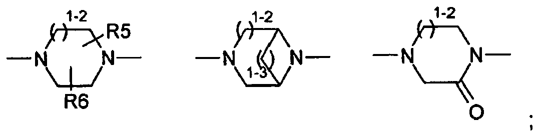
- R5 et R6 représentent un hydrogène ou un groupe méthyle ;
à l'état de base ou de sel d'addition à un acide.

3. Composé selon les revendications 1 ou 2 tel que n représente 1 ; à l'état de base ou de sel d'addition à un acide.

4. Composé selon l'une quelconque des revendications 1 à 3 tel que :
- R2 représente : 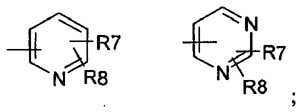
- R7 et R8, situés sur l'une quelconque des positions disponibles, représentent indépendamment un atome d'hydrogène, un atome d'halogène, un groupe (C1-C4)-alkyle, un radical trifluorométhyle, un groupe COOH ou un groupe COOalkyle
Ou un des R7 et R8 représente un hétérocycle choisi parmi : 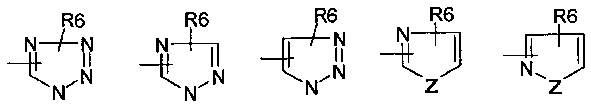 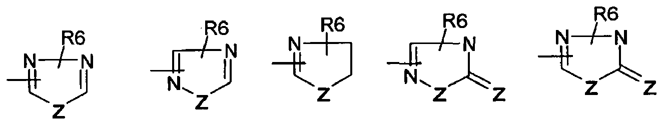 à l'état de de base ou de sel d'addition à un acide.

5. Composé selon l'une quelconque des revendications 1 à 4 tel que Y représente un atome d'azote, ou une liaison simple ou double ; à l'état de de base ou de sel d'addition à un acide.

6. Composé selon l'une quelconque des revendications 1 à 5 tel que X, X₁ et X₂ représentent un atome de carbone, d'azote ou de soufre, étant entendu qu'au moins un des X, X₁, X₂ est différent d'un atome de carbone ; à l'état de de base ou de sel d'addition à un acide.

7. Composé selon l'une quelconque des revendications 1 à 6 tel que :
- R et R1, situés sur l'une quelconque des positions disponibles, représentent indépendamment un atome d'hydrogène ou un atome d'halogène ou un groupe (C1-C4) alkyle;
ou bien R1 représente un groupe: 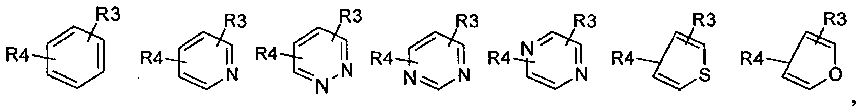 et R est un atome d'hydrogène;
- R3 et R4, situés sur l'une quelconque des positions disponibles, représentent un atome d'hydrogène, un atome d'halogène, un groupe (C1-C4)-alkoxy, un radical perfluoroalkyle; à l'état de de base ou de sel d'addition à un acide.

8. Composé selon l'une -quelconque des revendications précédentes choisi parmi :
- Composé n°1 : 1-(6,7-Dihydro-4H-thieno[3,2-c]pyridin-5-yl)-2-[8-(5-fluoro-pyrimidin-2-yl)-3,8-diaza-bicyclo[3.2.1]oct-3-yl]-éthanone ;
- Composé n°2 : 2-[8-(5-Fluoro-pyrimidin-2-yl)-3,8-diaza-bicyclo[3.2.1]oct-3-yl]-1-(2-phenyl-6,7-dihydro-4H-thiazolo[5,4-c]pyridin-5-yl)-éthanone ;
- Composé n°3 : 1-(2-Chloro-7,8-dihydro-5H-[1,6]naphthyridin-6-yl)-2-[8-(5-fluoropyrimidin-2-yl)-3,8-diaza-bicyclo[3.2.1]oct-3-yl]-éthanone ;
- Composé n°4 : 2-[8-(5-Fluoro-pyrimidin-2-yl)-3,8-diaza-bicyclo[3.2.1]oct-3-yl]-1-(2-phényl-2,4,6,7-tétrahydro-pyrazolo[4,3-c]pyridin-5-yl)-éthanone ;
- Composé n°5 : Methyl ester de l'acide 6-{3-[2-Oxo-2-(2-phényl-6,7-dihydro-4H-thiazolo[5,4-c]pyridin-5-yl)-ethyl]-3,8-diaza-bicyclo[3.2.1]oct-8-yl}-nicotinique ;
- Composé n°6: Acide 6-{(3S,5R)-3,5-Dimethyl-4-[2-oxo-2-(2-phenyl-6,7-dihydro-4H-thiazolo[5,4-c]pyridin-5-yl)-éthyl]-piperazin-1-yl}-nicotinique;
- Composé n°7 : Acide 6-{3-[2-Oxo-2-(2-phényl-6,7-dihydro-4H-thiazolo[5,4-c]pyridin-5-yl)-éthyl]-3,8-diaza-bicyclo[3.2.1]oct-8-yl}-nicotinique;
- Composé n°8 : Acide 6-{(3S,5R)-3,5-Dimethyl-4-[2-oxo-2-(2-phényl-6,7-dihydro-4H-thiazolo[5,4-c]pyridin-5-yl)-éthyl]-pipérazin-1-yl}-nicotinique;
- Composé n°9 : 2-[(2S,6R)-2,6-Dimethyl-4-(5-trifluoromethyl-pyridin-2-yl)-pipérazin-1-yl]-1-(2-phényl-6,7-dihydro-4H-thiazolo[5,4-c]pyridin-5-yl)-éthanone;
- Composé n°10 : 2-[(2S,6R)-2,6-Dimethyl-4-(5-trifluoromethyl-pyridin-2-yl)-pipérazin-1-yl]-1-(2-phényl-2,4,6,7-tétrahydro-pyrazolo[4,3-c]pyridin-5-yl)-éthanone;
- Composé n°11 : 4-[2-Oxo-2-(2-phényl-6,7-dihydro-4H-thiazolo[5,4-c]pyridin-5-yl)-éthyl]-1-(5-trifluorométhyl-pyridin-2-yl)-pipérazin-2-one;
- Composé n°12 : 1-(2-Phenyl-6,7-dihydro-4H-thiazolo[5,4-c]pyridin-5-yl)-2-[4-(5-trifluorométhyl-pyridin-2-yl)-pipérazin-1-yl]-éthanone;
- Composé n°13 : 1-(2-Phényl-2,4,6,7-tétrahydro-pyrazolo[4,3-c]pyridin-5-yl)-2-[4-(5-trifluorométhyl-pyridin-2-yl)-pipérazin-1-yl]-éthanone ;
- Composé n°14 : 4-[2-Oxo-2-(2-phényl-2,4,6,7-tétrahydro-pyrazolo[4,3-c]pyridin-5-yl)-éthyl]-1-(5-trifluorométhyl-pyridin-2-yl)-pipérazin-2-one;
- Composé n°15 : 2-[(2S,6R)-4-(5-Fluoro-pyrimidin-2-yl)-2,6-diméthyl-pipérazin-1-yl]-1 -(2-phényl-6,7-dihydro-4H-thiazolo[5,4-c]pyridin-5-yl)-éthanone;
- Composé n°16 : 2-((2S,6R)-2,6-Diméthyl-4-pyrimidin-5-yl-pipérazin-1-yl)-1-(2-phényl-2,4,6,7-tétrahydro-pyrazolo[4,3-c]pyridin-5-yl)-éthanone;
- Composé n°17 : 2-[(2S,6R)-4-(5-Fluoro-pyrimidin-2-yl)-2,6-diméthyl-pipérazin-1-yl]-1-(2-phényl-2,4,6,7-tetrahydro-pyrazolo[4,3-c]pyridin-5-yl)-éthanone;
- Composé n°18 : 2-[(2S,6R)-2,6-Dimethyl-4-(6-trifluorométhyl-pyridin-3-yl)-pipérazin-1-yl]-1-(2-phényl-6,7-dihydro-4H-thiazolo[5,4-c]pyridin-5-yl)-éthanone;
- Composé n°19 : Methyl ester de l'acide 6-{3-[2-Oxo-2-(2-phényl-2,4,6,7-tétrahydro-pyrazolo[4,3-c]pyridin-5-yl)-éthyl]-3,8-diaza-bicyclo[3.2.1]oct-8-yl}-nicotinique ;
- Composé n°20 : 2-[(2S,6R)-2,6-Diméthyl-4-(6-trifluoromethyl-pyridin-3-yl)-pipérazin -1-yl]-1-(2-phenyl-2,4,6,7-tétrahydro-pyrazolo[4,3-c]pyridin-5-yl)-éthanone;
- Composé n°21 : Acide 6-{(3S,5R)-3,5-Diméthyl-4-[2-oxo-2-(2-phényl-2,4,6,7-tétrahydropyrazolo[4,3-c]pyridin-5-yl)-éthyl]-pipérazin-1-yl}-nicotinique;
- Composé n°22 : 2-((2S,6R)-2,6-Dimethyl-4-pyrimidin-5-yl-pipérazin-1-yl)-1-(2-phényl-6,7-dihydro-4H-thiazolo[5,4-c]pyridin-5-yl)-éthanone;
- Composéu n°23 : Methyl ester de l'acide 6-{(3S,5R)-3,5-Diméthyl-4-[2-oxo-2-(2-phényl-2,4,6,7-tétrahydro-pyrazolo[4,3-c]pyridin-5-yl)-ethyl]-pipérazin-1-yl}-nicotinique;
- Composé n°24 : Acide 6-{3-[2-Oxo-2-(2-phenyl-2,4,6,7-tetrahydro-pyrazolo[4,3-c] pyridin-5-yl)-ethyl]-3,8-diaza-bicyclo[3.2.1]oct-8-yl}-nicotinique;
- Composé n°25 :Methyl ester de l'acide 6-{2-Oxo-4-[2-oxo-2-(2-phenyl-6,7-dihydro-4H-thiazolo[5,4-c]pyridin-5-yl)-ethyl]-piperazin-1-yl}-nicotinique;
- Composé n°26 2-[8-(5-Fluoro-pyrimidin-2-yl)-3,8-diaza-bicyclo[3.2.1]oct-3-yl]-1-(1-phényl-1,4,6,7-tétrahydro-pyrazolo[4,3-c]pyridin-5-yl)-éthanone ;
- Composé n°27 : 1-(2-Phényl-2,4,6,7-tétrahydro-pyrazolo[4,3-c]pyridin-5-yl)-2-(4-pyridin-3-yl-[1,4]diazepan-1-yl)-éthanone ;
- Composé n°28 : 1-(2-Phényl-2,4,6,7-tétrahydro-pyrazolo[4,3-c]pyridin-5-yl)-2-(8-pyridin-3-yl-3,8-diaza-bicyclo[3.2.1]oct-3-yl)-éthanone ;
- Composé n°29 : 1-(2-Phényl-2,4,6,7-tétrahydro-pyrazolo[4,3-c]pyridin-5-yl)-2-[8-(5-trifluorométhyl-pyridin-2-yl)-3,8-diaza-bicyclo[3.2.1]oct-3-yl]-éthanone ;
- Composé n°30 : 1-(2-Phényl-6,7-dihydro-4H-thiazolo[5,4-c]pyridin-5-yl)-2-(4-pyridin-3-yl-[1,4]diazepan-1-yl)-éthanone ;
- Composé n°31 : 1-(2-Phényl-2,4,6,7-tétrahydro-pyrazolo[4,3-c]pyridin-5-yl)-2-(8-pyrimidin-5-yl-3,8-diaza-bicyclo[3.2.1]oct-3-yl)-éthanone ;
- Composé n°32 : 2-[8-(5-Fluoro-pyrimidin-2-yl)-3,8-diaza-bicyclo[3.2.1]oct-3-yl]-1-(2-méthyl-2,4,6,7-tétrahydro-pyrazolo[4,3-c]pyridin-5-yl)-éthanone ;
- Composé n°33 : Méthyl ester de l'acide 6-{3-[2-Oxo-2-(2-phényl-4,6-dihydro-pyrrolo [3,4-d]thiazol-5-yl)-éthyl]-3,8-diaza-bicyclo[3.2.1]oct-8-yl}-nicotinique ;
- Composé n°34 : 4-{2-[2-(4-Méthoxy-phényl)-2,4,6,7-tétrahydro-pyrazolo[4,3-c] pyridin-5-yl]-2-oxo-éthyl}-1-(5-trifluorométhyl-pyridin-2-yl)-pipérazin-2-one ;
- Composé n°35 : 4-{2-[2-(4-Fluoro-phényl)-2,4,6,7-tétrahydro-pyrazolo[4,3-c]pyridin-5-yl]-2-oxo-éthyl}-1-(5-trifluorométhyl-pyridin-2-yl)-pipérazin-2-one ;
- Composé n°36 : 2-[8-(5-Fluoro-pyrimidin-2-yl)-3,8-diaza-bicyclo[3.2.1]oct-3-yl]-1-(2-méthyl-6,7-dihydro-4H-thiazolo[5,4-c]pyridin-5-yl)-éthanone ;
- Composé n°37 : Méthyl ester de l'acide 6-(3-{2-[2-(4-Méthoxy-phényl)-6,7-dihydro-4H-thiazolo[5,4-c]pyridin-5-yl]-2-oxo-éthyl}-3,8-diaza-bicyclo[3.2:1]oct-8-yl)-nicotinique ;
- Composé n°38 : Méthyl ester de l'acide 6-(3-{2-[2-(4-Fluoro-phényl)-6,7-dihydro-4H-thiazolo[5,4-c]pyridin-5-yl]-2-oxo-éthyl}-3,8-diaza-bicyclo[3.2.1]oct-8-yl)-nicotinique ;
- Composé n°39 : Méthyl ester de l'acide 6-(3-{2-Oxo-2-[2-(5-trifluorométhyl-pyridin-2-yl)-2,4,6,7-tétrahydro-pyrazolo[4,3-c]pyridin-5-yl]-éthyl}-3,8-diaza-bicyclo[3.2.1]oct-8-yl)-nicotinique ;
- Composé n°40 : 2-[8-(5-Fluoro-pyrimidin-2-yl)-3,8-diaza-bicyclo[3.2.1]oct-3-yl]-1-[2-(5-trifluorométhyl-pyridin-2-yl)-2,4,6,7-tétrahydro-pyrazolo[4,3-c]pyridin-5-yl]-éthanone ;
- Composé n°41 : 4-[2-Oxo-2-(2-phényl-7,8-dihydro-5H-pyrido[4,3-d]pyrimidin-6-yl)-éthyl]-1-(5-trifluorométhyl-pyridin-2-yl)-pipérazin-2-one ;
- Composé n°42 : 4-[2-Oxo-2-(2-thiophén-3-yl-6,7-dihydro-4H-thiazolo[5,4-c]pyridin-5-yl)-éthyl]-1-(5-trifluorométhyl-pyridin-2-yl)-pipérazin-2-one ;
- Composé n°43: 2-[8-(5-Fluoro-pyrimidin-2-yl)-3,8-diaza-bicyclo[3.2.1]oct-3-yl]-1-[2-(4-methoxy-phenyl)-2,4,6,7-tetrahydro-pyrazolo[4,3-c]pyridin-5-yl]-ethanone;
- Composé n°44: acide 6-{3-[2-Oxo-2-(2-thiophen-3-yl-6,7-dihydro-4H-thiazolo[5,4-c]pyridin-5-yl)-ethyl]-3,8-diaza-bicyclo[3.2.1]oct-8-yl}-nicotinique;
- Composé n°45: 2-[8-(5-Fluoro-pyrimidin-2-yi)-3,8-diaza-bicyclo[3.2.1]oct-3-yl]-1-[1-(2,2,2-trifluoro-ethyl)-4,5,6,7-tetrahydro-1H-indazo-5-yl]-ethanone;
- Composé n°46: 2-[8-(5-Fluoro-pyrimidin-2-yl)-3,8-diaza-bicyclo[3.2.1]oct-3-yl]-1-[1-(4-methoxy-phenyl)-1,4,6,7-tetrahydro-pyrazolo[4,3-c]pyridin-5-yl]-ethanone;
- Composé n°47: 2-[8-(5-Fluoro-pyrimidin-2-yl)-3,8-diaza-bicyclo[3.2.1]oct-3-yl]-1-(2,4,6,7-tetrahydro-pyrazolo[4,3-c]pyridin-5-yl)-ethanone;
- Composé n°48: méthyl ester de l'acide 6-{3-[2-Oxo-2-(1-phenyl-1,4,6,7-tetrahydropyrazolo[4,3-c]pyridin-5-yl)-ethyl]-3,8-diaza-bicyclo[3.2.1]oct-8-yl}-nicotinique;
- Composé n°49: acide 6-{3-[2-Oxo-2-(1-phenyl-1,4,6,7-tetrahydro-pyrazolo[4,3-c]pyridin-5-yl)-ethyl]-3,8-diaza-bicyclo[3.2.1]oct-8-yl}-nicotinique;
- Composé n°50: 1-(1-tert-Butyl-1,4,6,7-tetrahydro-pyrazolo[4,3-c]pyridin-5-yl)-2-[8-(5-fluoro-pyrimidin-2-yl)-3,8-diaza-bicyclo[3.2.1]oct-3-yl]-ethanone;
- Composé n°51: 1-[1-(4-Fluoro-phenyl)-1;4,6,7-tetrahydro-pyrazolo[4,3-c]pyridin-5-yl]-2-[8-(5-fluoro-pyrimidin-2-yl)-3,8-diaza-bicyclo[3.2.1]oct-3-yl]-ethanone;
- Composé n°52: 6-{(2R,5S)-2,5-Dimethyl-4-[2-oxo-2-(2-phenyl-2,4,6,7-tetrahydropyrazolo[4,3-c]pyridin-5-yl)-ethyl]-piperazin-1-yl}-nicotinonitrile;
- Composé n°53: acide 6-{(2R,5S)-2,5-Dimethyl-4-[2-oxo-2-(2-phenyl-2,4,6,7-tetrahydropyrazolo[4,3-c]pyridin-5-yl)-ethyl]-piperazin-1-yl}-nicotinique;
- Composé n°54: 2-[8-(5-Fluoro-pyrimidin-2-yl)-3,8-diaza-bicyclo[3.2.1]oct-3-yl]-1-(2-phenyl-4,7-dihydro-5H-furo[2,3-c]pyridin-6-yl)-ethanone;
- Composé n°55: 2-[8-(5-Fluoro-pyrimidin-2-yl)-3,8-diaza-bicyclo[3.2.1]oct-3-yl]-1-(2-phenyl-1,4,5,7-tetrahydro-pyrrolo[2,3-c]pyridin-6-yl)-ethanone;
- Composé n°56: méthyl ester de l'acide 6-{8-[2-Oxo-2-(2-phenyl-6,7-dihydro-4H-thiazolo[5,4-c]pyridin-5-yl)-ethyl]-3,8-diaza-bicyclo[3.2.1]oct-3-yl}-nicotinique;
- Composé n°57: méthyl ester de l'acide 6-{8-[2-Oxo-2-(2-phenyl-2,4,6,7-tetrahydropyrazolo[4,3-c]pyridin-5-yl)-ethyl]-3,8-diaza-bicyclo[3.2.1]oct-3-yl}-nicotinique;
- Composé n°58: 2-[8-(5-Fluoro-pyrimidin-2-yl)-3,8-diaza-bicyclo[3.2.1]oct-3-yl]-1-(2-phenyl-6,7-dihydro-4H-oxazolo[4,5-c]pyridin-5-yl)-ethanone;
- Composé n°59: 6-{(2R,5S)-2,5-Dimethyl-4-[2-oxo-2-(2-phenyl-6,7-dihydro-4H-thiazolo[5,4-c]pyridin-5-yl)-ethyl]-piperazin-1-yl}-nicotinonitrile;
- Composé n°60: 2-{8-[5-(5-Methyl-[1,2,4]oxadiazol-3-yl)-pyridin-2-yl]-3,8-diazabicyclo[3.2.1]oct-3-yl}-1-(2-phenyl-6,7-dihydro-4H-thiazolo[5,4-c]pyridin-5-yl)-ethanone;
- Composé n°61: acide 6-{8-[2-Oxo-2-(2-phenyl-2,4,6,7-tetrahydro-pyrazolo[4,3-c]pyridin-5-yl)-ethyl]-3,8-diaza-bicyclo[3.2.1]oct-3-yl}-nicotinique;
- Composé n°62: acide 6-{8-[2-Oxo-2-(2-phenyl-6,7-dihydro-4H-thiazolo[5,4-c]pyridin-5-yl)-ethyl]-3,8-diaza-bicyclo[3.2.1]oct-3-yl}-nicotinique;
- Composé n°63: acide 6-{(2R,5S)-2,5-Dimethyl-4-[2-oxo-2-(2-phenyl-6,7-dihydro-4H-thiazolo[5,4-c]pyridin-5-yl)-ethyl]-piperazin-1-yl}-nicotinique;
- Composé n°64: 2-[8-(5-Fluoro-pyrimidin-2-yl)-3,8-diaza-bicyclo[3.2.1]oct-3-yl]-1-(2-pyridin-4-yl-6,7-dihydro-4H-thiazolo[5,4-c]pyridin-5-yl)-ethanone;
- Composé n°65: 4-{2-Oxo-2-[2-(5-trifluoromethyl-pyridin-2-yl)-2,4,6,7-tetrahydropyrazolo[4,3-c]pyridin-5-yl]-ethyl}-1-(5-trifluoromethyl-pyridin-2-yl)-piperazin-2-one;
- Composé n°66: 4-{2-Oxo-2-[1-(2,2,2-trifluoro-ethyl)-1,4,6,7-tetrahydro-pyrazolo [4,3-c]pyridin-5-yl]-ethyl}-1-(5-trifluoromethyl-pyridin-2-yl)-piperazin-2-one;
- Composé n°67: 2-[8-(5-Fluoro-pyrimidin-2-yl)-3,8-diaza-bicyclo[3.2.1]oct-3-yl]-1-(2-phenyl-7,8-dihydro-5H-pyrido[4,3-d]pyrimidin-6-yl)-ethanone;
- Composé n°68: 3-(6-{3-[2-Oxo-2-(2-phenyl-2,4,6,7-tetrahydro-pyrazolo[4,3-c]pyridin-5-yl)-ethyl]-3,8-diaza-bicyclo[3.2.1]oct-8-yl}-pyridin-3-yl)-4H-[1,2,4]oxadiazol-5-one;
- Composé n°69: 2-[8-(5-Fluoro-pyrimidin-2-yl)-3,8-diaza-bicyclo[3.2.1]oct-3-yl]-1-(2-phenyl-3,4,6,7-tetrahydro-imidazo[4,5-c]pyridin-5-yl)-ethanone;
- Composé n°70: 3-(6-{3-[2-Oxo-2-(2-phenyl-6,7-dihydro-4H-thiazolo[5,4-c]pyridin-5-yl)-ethyl]-3,8-diaza-bicyclo[3.2.1]oct-8-yl}-pyridin-3-yl)-4H-[1,2,4]oxadiazol-5-one;
- Composé n°71: 2-[8-(5-Fluoro-pyrimidin-2-yl)-3,8-diaza-bicyclo[3.2.1]oct-3-yl]-1-(2-phenyl-6,7-dihydro-4H-thiazolo[4,5-c]pyridin-5-yl)-ethanone;
- Composé n°72: acide 2-{(3S,5R)-3,5-Dimethyl-4-[2-oxo-2-(2-phenyl-6,7-dihydro-4H-thiazolo[5,4-c]pyridin-5-yl)-ethyl]-piperazin-1-yl}-pyrimidine-5-carboxylique;
- Composé n°73: 2-[8-(5-Fluoro-pyrimidin-2-yl)-3,8-diaza-bicyclo[3.2.1]oct-3-yl]-1-(2-pyridin-3-yl-6,7-dihydro-4H-thiazolo[5,4-c]pyridin-5-yl)-ethanone;
- Composé n°74: 1-(2-Phenyl-6,7-dihydro-4H-thiazolo[5,4-c]pyridin-5-yl)-2-{8-[5-(1H-tetrazol-5-yl)-pyridin-2-yl]-3,8-diaza-bicyclo[3.2.1]oct-3-yl}-ethanone;
- Composé n°75: acide 2-{(3S,5R)-3,5-Dimethyl-4-[2-oxo-2-(2-phenyl-2,4,6,7-tetrahydropyrazolo[4,3-c]pyridin-5-yl)-ethyl]-piperazin-1-yl}-pyrimidine-5-carboxylique;
- Composé n°76: 6-(3-{2-[2-(4-Fluoro-phenyl)-6,7-dihydro-4H-thiazolo[5,4-c]pyridin-5-yl]-2-oxo-ethyl}-3,8-diaza-bicyclo[3.2.1]oct-8-yl)-nicotinonitrile;
- Composé n°77: 6-{3-[2-Oxo-2-(2-phenyl-2,4,6,7-tetrahydro-pyrazolo[4,3-c]pyridin-5-yl)-ethyl]-3,8-diaza-bicyclo[3.2.1]oct-8-yl}-nicotinonitrile;
- Composé n°78: 4-[2-Oxo-2-(2-phenyl-3,4,6,7-tetrahydro-imidazo[4,5-c]pyridin-5-yl)-ethyl]-1-(5-trifluoromethyl-pyridin-2-yl)-piperazin-2-one;
- Composé n°79: éthyl ester de l'acide 6-{(3S,5R)-3,5-Dimethyl-4-[2-oxo-2-(2-phenyl-2,4,6,7-tetrahydro-pyrazolo[4,3-c]pyridin-5-yl)-ethyl]-piperazin-1-yl}-nicotinique;
- Composé n°80: 1-[2-(4-Fluoro-phenyl)-6,7-dihydro-4H-thiazolo[5,4-c]pyridin-5-yl]-2-[8-(5-fluoro-pyrimidin-2-yl)-3,8-diaza-bicyclo[3.2.1]oct-3-yl]-ethanone;
- Composé n°81: 2-[8-(5-Fluoro-pyrimidin-2-yl)-3,8-diaza-bicyclo[3.2.1]oct-3-yl]-1-[2-(4-methoxy-phenyl)-6,7-dihydro-4H-thiazolo[5,4-c]pyridin-5-yl]-ethanone;
- Composé n°82: 4-[2-Oxo-2-(2-phenyl-6,7-dihydro-4H-thiazolo[4,5-c]pyridin-5-yl)-ethyl]-1-(5-trifluoromethyl-pyridin-2-yl)-piperazin-2-one;
- Composé n°83: 4-[2-Oxo-2-(2-phenyl-6,7-dihydro-4H-oxazolo[4,5-c]pyridin-5-yl)-ethyl]-1-(5-trifluoromethyl-pyridin-2-yl)-piperazin-2-one;
- Composé n°84: 4-[2-Oxo-2-(2-pyridin-3-yl-6,7-dihydro-4H-thiazolo[5,4-c]pyridin-5-yl)-ethyl]-1-(5-trifluoromethyl-pyridin-2-yl)-piperazin-2-one;
- Composé n°85: 2-{8-[5-(2-Methyl-2H-tetrazol-5-yl)-pyridin-2-yl]-3,8-diaza-bicyclo [3.2.1]oct-3-yl}-1-(2-phenyl-2,4,6,7-tetrahydro-pyrazolo[4,3-c]pyridin-5-yl)-ethanone;
- Composé n°86: 2-{(2S,6R)-2,6-Dimethyl-4-[5-(1-methyl-1H-tetrazol-5-yl)-pyridin-2-yl]-piperazin-1-yl}-1-(2-phenyl-2,4,6,7-tetrahydro-pyrazolo[4,3-c]pyridin-5-yl)-ethanone;
- Composé n°87: 2-{(2S,6R)-2,6-Dimethyl-4-[5-(2-methyl-2H-tetrazol-5-yl)-pyridin-2-yl]-piperazin-1-yl}-1-(2-phenyl-2,4,6,7-tetrahydro-pyrazolo[4,3-c]pyridin-5-yl)-ethanone;
- Composé n°88: 2-[8-(5-Fluoro-pyrimidin-2-yl)-3,8-diaza-bicyclo[3.2.1]oct-3-yl]-1-[1-(4-trifluoromethyl-phenyl)-1,4,6,7-tetrahydro-pyrazolo[4,3-c]pyridin-5-yl]-ethanone;
- Composé n°89: 2-{(2S,6R)-2,6-Dimethyl-4-[5-(5-methyl-[1,2,4]oxadiazol-3-yl)-pyridin-2-yl]-piperazin-1-yl}-1-(2-phenyl-2,4,6,7-tetrahydro-pyrazolo[4,3-c]pyridin-5-yl)-ethanone;
- Composé n°90: 2-[(2S,6R)-2,6-Dimethyl-4-(5-[1,3,4]oxadiazol-2-yl-pyridin-2-yl)-piperazin-1-yl]-1-(2-phenyl-2,4,6,7-tetrahydro-pyrazolo[4,3-c]pyridin-5-yl)-ethanone;
- Composé n°91: 2-{(2S,6R)-2,6-Dimethyl-4-[5-(3-methyl-[1,2,4]oxadiazol-5-yl)-pyridin-2-yl]-piperazin-1-yl}-1-(2-phenyl-2,4,6,7-tetrahydro-pyrazolo[4,3-c]pyridin-5-yl)-ethanone;
- Composé n°92: 6-{(3S,5R)-4-[2-(1-tert-Butyl-1,4,6,7-tetrahydro-pyrazolo[4,3-c]pyridin-5-yl)-2-oxo-ethyl]-3,5-dimethyl-piperazin-1-yl}-nicotinic acid methyl ester;
- Composé n°93: acide 6-{(3S,5R)-4-[2-(1-tert-Butyl-1,4,6,7-tetrahydro-pyrazolo[4,3-c] pyridin-5-yl)-2-oxo-ethyl]-3,5-dimethyl-piperazin-1-yl}-nicotinique;
- Composé n°94: acide 6-{3-[2-(1-tert-Butyl-1,4,6,7-tetrahydro-pyrazolo[4,3-c]pyridin-5-yl)-2-oxo-ethyl]-3,8-diaza-bicyclo[3.2.1]oct-8-yl}-nicotinique;
- Composé n°95: 2-[8-(5-Fluoro-pyrimidin-2-yl)-3,8-diaza-bicyclo[3.2.1]oct-3-yl]-1-(2-phenyl-6,7-dihydro-4H-oxazolo[5,4-c]pyridin-5-yl)-ethanone;
- Composé n°96: 4-[2-Oxo-2-(2-phenyl-6,7-dihydro-4H-oxazolo[5,4-c]pyridin-5-yl)-ethyl]-1-(5-trifluoromethyl-pyridin-2-yl)-piperazin-2-one;
- Composé n°97: 2-[(2S,6R)-2,6-Dimethyl-4-(5-trifluoromethyl-pyridin-2-yl)-piperazin-1-yl]-1-(2-phenyl-4,7-dihydro-5H-furo[2,3-c]pyridin-6-yl)-ethanone;
- Composé n°98: isopropyl ester de l'acide 6-{(3S,5R)-3,5-Dimethyl-4-[2-oxo-2-(2-phenyl-2,4,6,7-tetrahydro-pyrazolo[4,3-c]pyridin-5-yl)-ethyl]-piperazin-1-yl}-nicotinique;
- Composé n°99: 1-(2-Methyl-2,4,6,7-tetrahydro-pyrazolo[4,3-c]pyridin-5-yl)-2-[8-(5-trifluoromethyl-pyridin-2-yl)-3,8-diaza-bicyclo[3.2.1]oct-3-yl]-ethanone;
- Composé n°100: 2-[(2S,6R)-2,6-Dimethyl-4-(5-trifluoromethyl-pyridin-2-yl)-piperazin-1-yl]-1-(2-methyl-2,4,6,7-tetrahydro-pyrazolo[4,3-c]pyridin-5-yl)-ethanone;
- Composé n°101: 1-(2-Phenyl-4,7-dihydro-5H-furo[2,3-c]pyridin-6-yl)-2-[8-(5-trifluoromethyl-pyridin-2-yl)-3,8-diaza-bicyclo[3.2.1]oct-3-yl]-ethanone;
- Composé n°102: acide 6-(3-{2-[2-(4-Fluoro-phenyl)-6,7-dihydro-4H-thiazolo[5,4-c]pyridin-5-yl]-2-oxo-ethyl}-3,8-diaza-bicyclo[3.2.1]oct-8-yl)-nicotiniq ue;
- Composé n°103: 2-[(2S,6R)-2,6-Dimethyl-4-(5-trifluoromethyl-pyridin-2-yl)-piperazin-1-yl]-1-[2-(5-trifluoromethyl-pyridin-2-yl)-2,4,6,7-tetrahydro-pyrazolo[4,3-c]pyridin-5-yl]-ethanone;
- Composé n°104: 2-[8-(5-Trifluoromethyl-pyridin-2-yl)-3,8-diaza-bicyclo[3.2.1]oct-3-yl]-1-[2-(5-trifluoromethyl-pyridin-2-yl)-2,4,6,7-tetrahydro-pyrazolo[4,3-c]pyridin-5-yl]-ethanone;
- Composé n°105: acide 6-{(3S,5R)-4-[2-(2-tert-Butyl-2,4,6,7-tetrahydro-pyrazolo [4,3-c]pyridin-5-yl)-2-oxo-ethyl]-3,5-dimethyl-piperazin-1-yl}-nicotinique;
- Composé n°106: 2-(8-Pyridin-3-yl-3,8-diaza-bicyclo[3.2.1]oct-3-yl)-1-[2-(5-trifluoromethylpyridin-2-yl)-2,4,6,7-tetrahydro-pyrazolo[4,3-c]pyridin-5-yl]-ethanone;
- Composé n°107: 2-[5-(6-Trifluoromethyl-pyridazin-3-yl)-2,5-diaza-bicyclo [2.2.1] hept-2-yl]-1-[2-(5-trifluoromethyl-pyridin-2-yl)-2,4,6,7-tetrahydro-pyrazolo[4,3-c] pyridin-5-yl]-ethanone;
- Composé n°108: 2-(6'-Chloro-2,3,5,6-tetrahydro-[1,2']bipyrazinyl-4-yl)-1-[2-(5-trifluoromethyl-pyridin-2-yl)-2,4,6,7-tetrahydro-pyrazolo[4,3-c]pyridin-5-yl]-ethanone;
- Composé n°109: 1-(2-Phenyl-3,4,6,7-tetrahydro-imidazo[4,5-c]pyridin-5-yl)-2-[8-(5-trifluoromethyl-pyridin-2-yl)-3,8-diaza-bicyclo[3.2.1]oct-3-yl]-ethanone;
- Composé n°110: 2-[(2S,6R)-2,6-Dimethyl-4-(5-trifluoromethyl-pyridin-2-yl)-piperazin-1-yl]-1-(2-phenyl-3,4,6,7-tetrahydro-imidazo[4,5-c]pyridin-5-yl)-ethanone;
- Composé n°111: 2-((3S,5R)-3,5-Dimethyl-2,3,5,6-tetrahydro-[1,2']bipyrazinyl-4-yl)-1-[2-(5-trifluoromethyl-pyridin-2-yl)-2,4,6,7-tetrahydro-pyrazolo[4,3-c]pyridin-5-yl]-ethanone;
- Composé n°112: 2-[(2S,6R)-4-(5-Chloro-pyridin-2-yl)-2,6-dimethyl-piperazin-1-yl]-1-[2-(5-trifluoromethyl-pyridin-2-yl)-2,4,6,7-tetrahydro-pyrazolo[4,3-c]pyridin-5-yl]-ethanone;
- Composé n°113: 2-[4-(7-Chloro-quinolin-4-yl)-piperazin-1-yl]-1-(2-phenyl-2,4,6,7-tetrahydro-pyrazolo[4,3-c]pyridin-5-yl)-ethanone;
- Composé n°114: 2-[4-(6-Chloro-pyridin-2-yl)-piperazin-1-yl]-1-(2-phenyl-2,4,6,7-tetrahydro-pyrazolo[4,3-c]pyridin-5-yl)-ethanone;
- Composé n°115: 1-(2-Pyridin-4-yl-6,7-dihydro-4H-thiazolo[5,4-c]pyridin-5-yl)-2-[8-(5-trifluoromethyl-pyridin-2-yl)-3,8-diaza-bicyclo[3.2.1]oct-3-yl]-ethanone;
- Composé n°116: acide 6-{(3S,5R)-3,5-Dimethyl-4-[2-oxo-2-(2-phenyl-3,4,6,7-tetrahydroimidazo[4,5-c]pyridin-5-yl)-ethyl]-piperazin-1-yl}-nicotinique;
- Composé n°117: 1-(2-Pyridin-2-yl-2,4,6,7-tetrahydro-pyrazolo[4,3-c]pyridin-5-yl)-2-[8-(5-trifluoromethyl-pyridin-2-yl)-3,8-diaza-bicyclo[3.2.1]oct-3-yl]-ethanone;
- Composé n°118: 2-[(2S,6R)-2,6-Dimethyl-4-(5-trifluoromethyl-pyridin-2-yl)-piperazin-1-yl]-1-(2-pyridin-2-yl-2,4,6,7-tetrahydro-pyrazolo[4,3-c]pyridin-5-yl)-ethanone;
- Composé n 119: 4-[2-Oxo-2-(2-pyridin-2-yl-2,4,6,7-tetrahydro-pyrazolo[4,3-c] pyridin-5-yl)-ethyl]-1-(5-trifluoromethyl-pyridin-2-yl)-piperazin-2-one;
- Composé n 120: 4-{2-[2-(4-Fluoro-phenyl)-3,4,6,7-tetrahydro-imidazo[4,5-c]pyridin-5-yl]-2-oxo-ethyl}-1-(5-trifluoromethyl-pyridin-2-yl)-piperazin-2-one;
- Composé n°121: 2-(8-Pyridin-3-yl-3,8-diaza-bicyclo[3.2.1]oct-3-yl)-1-(2-pyridin-2-yl-2,4,6,7-tetrahydro-pyrazolo[4,3-c]pyridin-5-yl)-ethanone;
- Composé n°122: 1-[2-(2,2,2-Trifluoro-ethyl)-2,4,6,7-tetrahydro-pyrazolo[4,3-c] pyridin-5-yl]-2-[8-(5-trifluoromethyl-pyridin-2-yl)-3,8-diaza-bicyclo[3.2.1]oct-3-yl]-ethanone;
- Composé n°123: 1-[2-(4-Fluoro-phenyl)-3,4,6,7-tetrahydro-imidazo[4,5-c]pyridin-5-yl]-2-[8-(5-trifluoromethyl-pyridin-2-yl)-3,8-diaza-bicyclo[3.2.1]oct-3-yl]-ethanone;
- Composé n°124: 2-[(2S,6R)-2,6-Dimethyl-4-(5-trifluoromethyl-pyridin-2-yl)-piperazin-1-yl]-1-[2-(4-fluoro-phenyl)-3,4,6,7-tetrahydro-imidazo[4,5-c]pyridin-5-yl]-ethanone;
- Composé n°125: 4-{2-Oxo-2-[2-(2,2,2-trifluoro-ethyl)-2,4,6,7-tetrahydro-pyrazolo[4,3-c]pyridin-5-y1]-ethyl}-1-(5-trifluoromethyl-pyridin-2-yl)-piperazin-2-one;
- Composé n°126: 2-[(2S,6R)-2,6-Dimethyl-4-(5-trifluoromethyl-pyridin-2-yl)-piperazin-1-yl]-1-[2-(2,2,2-trifluoro-ethyl)-2,4,6,7-tetrahydro-pyrazolo[4,3-c]pyridin-5-yl]-ethanone;
- Composé n°127: 1-(2-Phenyl-2,4,6,7-tetrahydro-pyrazolo[4,3-c]pyridin-5-yl)-2-[5-(5-trifluoromethyl-pyridin-2-yl)-2,5-diaza-bicyclo[2.2.1]hept-2-yl]-ethanone;
- Composé n°128: cyclobutyl ester de l'acide 6-{3-[2-Oxo-2-(2-phenyl-2,4,6,7-tetrahydro-pyrazolo[4,3-c]pyridin-5-yl)-ethyl]-3,8-diaza-bicyclo[3.2.1]oct-8-yl}-nicotinique;
- Composé n°129: 2-((2S,6R)-2,6-Dimethyl-4-quinolin-2-yl-piperazin-1-yl)-1-(2-phenyl-2,4,6,7-tetrahydro-pyrazolo[4,3-c]pyridin-5-yl)-ethanone;
- Composé n°130: éthyl ester de l'acide 6-{(3S,5R)-3,5-Diméthyl-4-[2-oxo-2-(2-phényl-2,4,6,7-tétrahydro-pyrazolo[4,3-c]pyridin-5-yl)-ethyl]-pipérazin-1-yl}-nicotinique;
- Composé n°131: 2-[(2S,6R)-4-(5-Methanesulfonyl-pyridin-2-yl)-2,6-dimethyl-piperazin-1-yl]-1-(2-phenyl-2,4,6,7-tetrahydro-pyrazolo[4,3-c]pyridin-5-yl)-ethanone;
- Composé n°132: 2-[(2S,6R)-4-(5-Fluoro-pyrimidin-2-yl)-2,6-dimethyl-piperazin-1-yl]-1-[2-(4-methoxy-phenyl)-6,7-dihydro-4H-thiazolo[5,4-c]pyridin-5-yl]-ethanone;
- Composé n°133: 1-[2-(4-Methoxy-phenyl)-6,7-dihydro-4H-thiazolo[5,4-c]pyridin-5-yl]-2-[5-(5-trifluoromethyl-pyridin-2-yl)-2,5-diaza-bicyclo[2.2.1]hept-2-yl]-ethanone;
- Composé n°134: 1-(2-Phenyl-6,7-dihydro-4H-thiazolo[5,4-c]pyridin-5-yl)-2-{4-[5-(2H-pyrazol-3-yl)-pyridin-2-yl]-piperazin-1-yl}-ethanone;
- Composé n°135: 2-[(2S,6R)-2,6-Dimethyl-4-(5-thiazol-2-yl-pyridin-2-yl)-piperazin-1-yl]-1-(2-phenyl-2,4,6,7-tetrahydro-pyrazolo[4,3-c]pyridin-5-yl)-ethanone;
- Composé n°136: 1-(2-Phenyl-6,7-dihydro-4H-thiazolo[5,4-c]pyridin-5-yl)-2-[8-(5-thiazol-2-yl-pyridin-2-yl)-3,8-diaza-bicyclo[3.2.1]oct-3-yl]-ethanone;
- Composé n°137: 2-[8-(5-[1,2,4]Oxadiazol-3-yl-pyridin-2-yl)-3,8-diaza-bicyclo [3.2.1]oct-3-yl]-1-(2-phenyl-2,4,6,7-tetrahydro-pyrazolo[4,3-c]pyridin-5-yl)-ethanone;
- Composé n°138: 1-(2-Ethyl-2,4,6,7-tetrahydro-pyrazolo[4,3-c]pyridin-5-yl)-2-[8-(5-trifluoromethyl-pyridin-2-yl)-3,8-diaza-bicyclo[3.2.1]oct-3-yl]-ethanone;
- Composé n°139: 2-[(2S,6R)-2,6-Dimethyl-4-(5-[1,2,4]oxadiazol-5-yl-pyridin-2-yl)-piperazin-1-yl]-1-(2-phenyl-2,4,6,7-tetrahydro-pyrazolo[4,3-c]pyridin-5-yl)-ethanone;
- Composé n°140: acide 6-{(3S,5R)-3,5-Dimethyl-4-[2-oxo-2-(2-pyridin-2-yl-2,4,6,7-tetrahydro-pyrazolo[4,3-c]pyridin-5-yl)-ethyl]-piperazin-1-yl}-nicotinique;
- Composé n°141: 2-((2S,6R)-2,6-Dimethyl-4-pyridin-3-yl-piperazin-1-yl)-1-(2-pyridin-4-yl-2,4,6,7-tetrahydro-pyrazolo[4,3-c]pyridin-5-yl)-ethanone;
- Composé n°142: 2-((2S,6R)-2,6-Dimethyl-4-pyridin-3-yl-piperazin-1-yl)-1-[2-(5-fluoro-pyridin-2-yl)-2,4,6,7-tetrahydro-pyrazolo[4,3-c]pyridin-5-yl]-ethanone;
- Composé n°143: 2-((2S,6R)-2,6-Dimethyl-4-pyridin-3-yl-piperazin-1-yl)-1-[2-(5-trifluoromethyl-pyridin-2-yl)-2,4,6,7-tetrahydro-pyrazolo[4,3-c]pyridin-5-yl]-ethanone;
- Composé n°144: methyl ester de l'acide 6-{(3S,5R)-3,5-Dimethyl-4-[2-oxo-2-(2-pyridin-2-yl-2,4,6,7-tetrahydro-pyrazolo[4,3-c]pyridin-5-yl)-ethyl]-piperazin-1-yl}-nicotinique;
- Composé n°145: 2-((2S,6R)-2,6-Dimethyl-4-pyridin-3-yl-piperazin-1-y1)-1-(2-phenyl-3,4,6,7 -tetrahydro-imidazo[4,5-c]pyridin-5-yl)-ethanone;
- Composé n°146: 2-[(2S,6R)-2,6-Dimethyl-4-(5-trifluoromethyl-pyridin-2-yl)-piperazin-1-yl]-1-(2-pyridazin-3-y1-2,4,6,7-tetrahydro-pyrazolo[4,3-c]pyridin-5-yl)-ethanone;
- Composé n°147: 2-[(2S,6R)-2,6-Dimethyl-4-(5-trifluoromethyl-pyridin-2-yl)-piperazin-1-yl]-1-(2-ethyl-2,4,6,7-tetrahydro-pyrazolo[4,3-c]pyridin-5-yl)-ethanone;
- Composé n°148: 4-[2-(2-Ethyl-2,4,6,7-tetrahydro-pyrazolo[4,3-c]pyridin-5-yl)-2-oxo-ethyl]-1-(5-trifluoromethyl-pyridin-2-yl)-piperazin-2-one;
- Composé n°149: acide 6-{(3S,5R)-3,5-Dimethyl-4-[2-oxo-2-(2-phenyl-2,4,6,7-tetrahydro-pyrazolo[4,3-c]pyridin-5-yl)-ethyl]-piperazin-1-yl}-nicotinique;
- Composé n°150: acide 6-{(3S,5R)-3,5-Dimethyl-4-[2-oxo-2-(2-phenyl-2,4,6,7-tetrahydro-pyrazolo[4,3-c]pyridin-5-yl)-ethyl]-piperazin-1-yl}-nicotinique
à l'état de base ou de sel d'addition à un acide.

9. Composé selon l'une quelconque des revendications 1 à 7 choisi parmi les composés suivants :

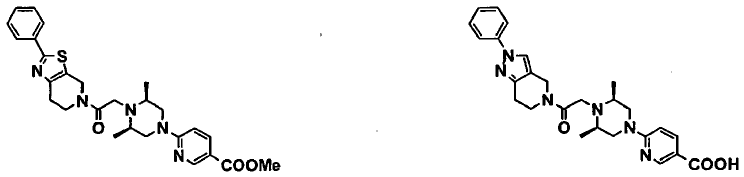

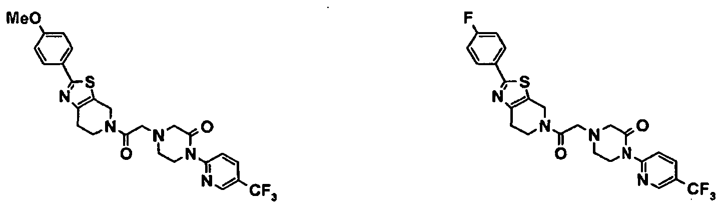

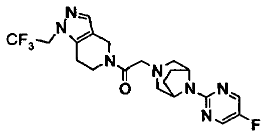

10. Composé selon l'une quelconque des revendications précédentes choisi comme étant l-'acide 6-{(3S,5R)-3,5-Diméthyl-4-[2-oxo-2-(2-phényl-2,4,6,7-tétrahydro-pyrazolo[4,3-c]pyridin-5-yl)-éthyl]-pipérazin-1-yl}-nicotinique.

11. Procédé de préparation d'un composé de formule (I) selon l'une quelconque des revendications 1 à 10 **caractérisé en ce que** l'on fait réagir un composé de formule (II) : 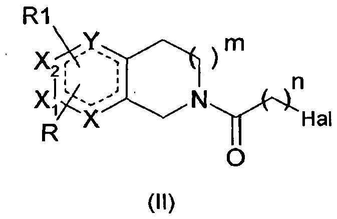 dans laquelle Y, X, X1, X2, R1, R, n et m sont définis selon l'une quelconque des revendications 1 à 10 et Hal représente un atome d'halogène,
et un composé de formule générale (III) :
H-W-R2 (III)
dans laquelle W et R2 sont définis selon l'une quelconque des revendications 1 à 10.

12. Composé de formule (II) : 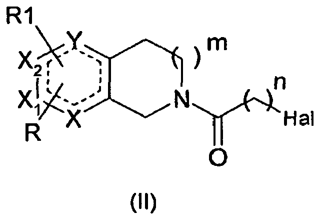 dans laquelle Y, X, X1, X2, R1, R, n et m sont définis selon l'une quelconque des revendication 1 à 10 et Hal représente un atome d'halogène ; à l'état de base ou de sel d'addition à un acide.

13. Médicament, **caractérisé en ce qu'**il comprend un composé de formule (I) selon l'une quelconque des revendications 1 à 10, ou un sel d'addition de ce composé à un acide pharmaceutiquement acceptable.

14. Composition pharmaceutique, **caractérisée en ce qu'**elle comprend un composé de formule (I) selon l'une quelconque des revendications 1 à 10, ou un sel pharmaceutiquement acceptable, ainsi qu'au moins un excipient pharmaceutiquement acceptable.

15. Composé selon l'une quelconque des revendications 1 à 10 comme médicament.

16. Composé selon l'une quelconque des revendications 1 à 10 pour la préparation d'un médicament destiné à la prévention ou le traitement de maladies neurodégénératives centrales et périphériques, la démence sénile, l'épilepsie, la maladie d'Alzheimer, la maladie de Parkinson, la chorée d'Huntington, le syndrome de Down, les maladies à prion, l'amnésie, la schizophrénie, la dépression, le désordre bipolaire, la sclérose latérale amyotrophique, la sclérose en plaques, les affections cardiovasculaires, les dommages cardiaques post-ischémiques, les cardiomyopathies, l'infarctus du myocarde, l'insuffisance cardiaque, l'ischémie cardiaque, l'infarctus cérébral ; les neuropathies périphériques, les dommages au nerf optique et à la rétine, la dégénérescence du pigment rétinien, le glaucome, l'ischémie rétinale-, la dégénérescence maculaire, les traumatismes de la moelle épinière, les traumatismes crâniens, l'athérosclérose, les sténoses, les désordres de la cicatrisation, l'alopécie, la pancréatite, la fibrose hépatique, les cancers, les tumeurs, les métastases, les leucémies, les désordres respiratoires, l'inflammation pulmonaire, l'allergie, l'asthme, la broncho-pneumopathie chronique obstructive, la douleur cutanée, somatique, viscérale, et neurologique, les douleurs chroniques neuropathiques et inflammatoires, les maladies auto-immunes, la polyarthrite rhumatoïde, la spondylarthrite ankylosante, le rhumatisme psoriasique, le psoriasis en plaques, les fractures osseuses, les maladies osseuses, l'ostéoporose.

17. Utilisation d'un composé selon l'une des revendications 1 à 10 pour la préparation de médicaments utiles dans le traitement et la prévention de maladies neurodégénératives centrales et périphériques, la démence sénile, l'épilepsie, la maladie d'Alzheimer, la maladie de Parkinson, la chorée d'Huntington, le syndrome de Down, les maladies à prion, l'amnésie, la schizophrénie, la dépression, le désordre bipolaire, la sclérose latérale amyotrophique, la sclérose en plaques, les affections cardiovasculaires, les dommages cardiaques post-ischémiques, les cardiomyopathies, l'infarctus du myocarde, l'insuffisance cardiaque, l'ischémie cardiaque, l'infarctus cérébral ; les neuropathies périphériques, les dommages au nerf optique et à la rétine, la dégénérescence du pigment rétinien, le glaucome, l'ischémie rétinale, la dégénérescence maculaire, les traumatismes de la moelle épinière, les traumatismes crâniens, l'athérosclérose, les sténoses, les désordres de la cicatrisation, l'alopécie, la pancréatite, la fibrose hépatique, les cancers, les tumeurs, les métastases, les leucémies, les désordres respiratoires, l'inflammation pulmonaire, l'allergie, l'asthme, la broncho-pneumopathie chronique obstructive, la douleur cutanée, somatique, viscérale, et neurologique, les douleurs chroniques neuropathiques et inflammatoires, les maladies auto-immunes, la polyarthrite rhumatoïde, la spondylarthrite ankylosante, le rhumatisme psoriasique, le psoriasis en plaques, les fractures osseuses, les maladies osseuses, l'ostéoporose.

## Patentansprüche

1. Verbindung der Formel (I): 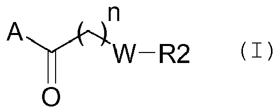 worin:
- A für eine Gruppe: 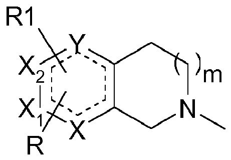 steht;
- n für 1 oder 2 steht;
- m für 0 oder 1 steht;
- Y für ein Kohlenstoff-, Stickstoff-, Schwefel- oder Sauerstoffatom oder eine Einfach- oder Doppelbindung steht;
- X, X₁ und X₂ für ein Kohlenstoff-, Stickstoff-, Schwefel- oder Sauerstoffatom stehen, mit der Maßgabe, dass mindestens eine der Variablen X, X₁ und X₂ von einem Kohlenstoffatom verschieden ist;
- R und R₁ an einer beliebigen der verfügbaren Positionen stehen und unabhängig voneinander für ein Wasserstoffatom, ein Halogenatom, eine (C1-C4)-Alkylgruppe, eine (C1-C4)-Alkoxygruppe, einen Perfluoralkylrest, einen Trifluormethoxyrest, einen Cyanorest, eine COOH-Gruppe, eine COO-Alkyl-Gruppe, eine CONR5R6-Gruppe oder eine NHCOR5-Gruppe stehen;
oder R1 für eine aus: 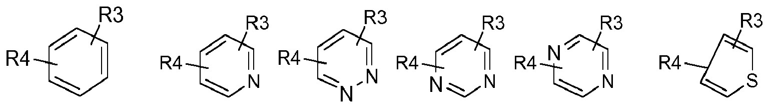 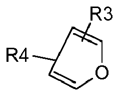 ausgewählte Gruppe steht, wobei die Definition von R unverändert bleibt;
- R3 und R4 an einer beliebigen der verfügbaren Positionen stehen und unabhängig voneinander für ein Wasserstoffatom, ein Halogenatom, eine (C1-C4)-Alkylgruppe, eine (C1-C4)-Alkoxygruppe, einen Perfluoralkylrest, einen Trifluormethoxyrest, einen Cyanorest, eine COOH-Gruppe, eine COO-Alkyl-Gruppe, eine CONR5R6-Gruppe oder eine NHCOR5-Gruppe stehen;
- -W- für einen stickstoffhaltigen Heterocyclus steht, der aus: 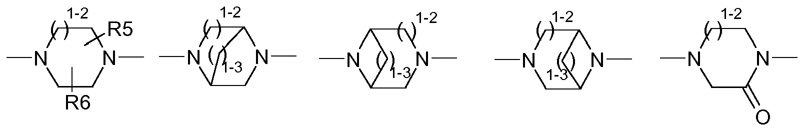 ausgewählt ist;
- 1-2 für 1 oder 2 steht;
- 1-3 für 1, 2 oder 3 steht;
- R2 für eine Gruppe der Formel: 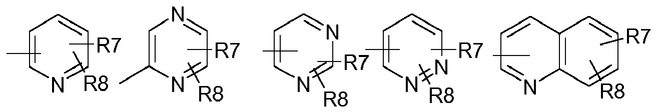 steht;
- R7 und R8 an einer beliebigen der verfügbaren Positionen stehen und unabhängig voneinander für ein Wasserstoffatom, ein Halogenatom, eine (C1-C4)-Alkylgruppe, eine (C1-C4)-Alkoxygruppe, einen Trifluormethylrest, einen Trifluormethoxyrest, einen Cyanorest, eine COOH-Gruppe, eine COO-Alkyl-Gruppe, eine COO-Cycloalkylgruppe, eine SO-AlkylGruppe, eine SO₂-Alkyl-Gruppe, eine CONH2-Gruppe, eine CONR5R6-Gruppe oder eine NHCOR5-Gruppe stehen;
oder eine der Variablen R7 und R8 für einen Heterocyclus steht, der aus: 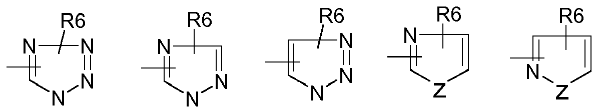 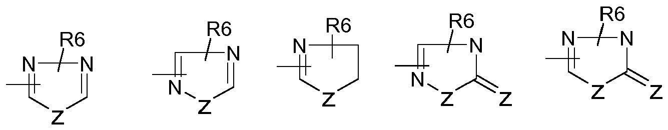 ausgewählt ist;
- Z für ein Sauerstoff- oder Schwefelatom steht;
- R5 und R6 für ein Wasserstoffatom oder eine (C1-C6)-Alkylgruppe stehen;
in Basen- oder Säureadditionssalzform.

2. Verbindung nach Anspruch 1, wobei:
- W für eine Gruppe der aus: 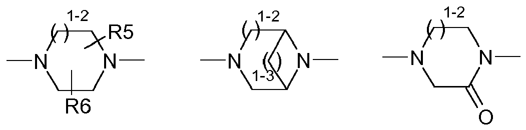 ausgewählten Formel steht;
- R5 und R6 für ein Wasserstoffatom oder eine Methylgruppe stehen;
in Basen- oder Säureadditionssalzform.

3. Zusammensetzung nach Anspruch 1 oder 2, wobei n für 1 steht; in Basen- oder Säureadditionssalzform.

4. Verbindung nach einem der Ansprüche 1 bis 3, wobei:
- R2 für: 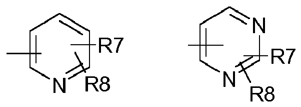 steht;
- R7 und R8 an einer beliebigen der verfügbaren Positionen stehen und unabhängig voneinander für ein Wasserstoffatom, ein Halogenatom, eine (C1-C4)-Alkylgruppe, einen Trifluormethylrest, eine COOH-Gruppe oder eine COO-Alkyl-Gruppe stehen; oder eine der Variablen R7 und R8 für einen Heterocyclus steht, der aus: 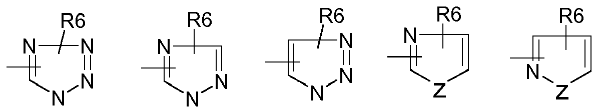 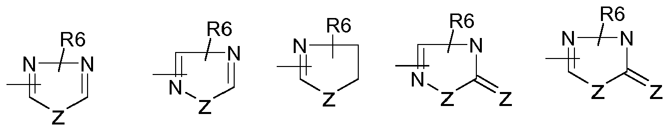 ausgewählt ist;
in Basen- oder Säureadditionssalzform.

5. Verbindung nach einem der Ansprüche 1 bis 4, wobei Y für ein Stickstoffatom oder eine Einfach- oder Doppelbindung steht; in Basen- oder Säureadditionssalzform.

6. Verbindung nach einem der Ansprüche 1 bis 5, wobei X, X₁ und X₂ für ein Kohlenstoff-, Stickstoff- oder Schwefelatom stehen, mit der Maßgabe, dass mindestens eine der Variablen X, X₁ und X₂ von einem Kohlenstoffatom verschieden ist; in Basen- oder Säureadditionssalzform.

7. Verbindung nach einem der Ansprüche 1 bis 6, wobei:
- R und R₁ an einer beliebigen der verfügbaren Positionen stehen und unabhängig voneinander für ein Wasserstoffatom oder ein Halogenatom oder eine (C1-C4)-Alkylgruppe stehen;
oder auch
R1 für eine Gruppe: 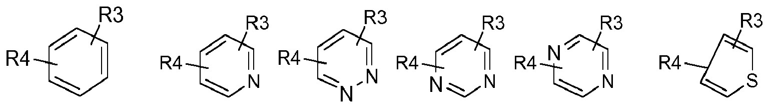 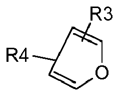 steht und R für ein Wasserstoffatom steht;
- R3 und R4 an einer beliebigen der verfügbaren Positionen stehen und für ein Wasserstoffatom, ein Halogenatom, eine (C1-C4)-Alkoxygruppe oder einen Perfluoralkylrest stehen;
in Basen- oder Säureadditionssalzform.

8. Verbindung nach einem der vorhergehenden Ansprüche, ausgewählt aus:
- Verbindung Nr. 1: 1-(6,7-Dihydro-4H-thieno[3,2-c]pyridin-5-yl)-2-[8-(5-fluorpyrimidin-2-yl)-3,8-diazabicyclo[3.2.1]oct-3-yl]ethanon;
- Verbindung Nr. 2: 2-[8-(5-Fluorpyrimidin-2-yl)-3,8-diazabicyclo[3.2.1]oct-3-yl]-1-(2-phenyl-6,7-dihydro-4H-thiazolo[5,4-c]pyridin-5-yl)ethanon;
- Verbindung Nr. 3: 1-(2-Chlor-7,8-dihydro-5H-[1,6]naphthyridin-6-yl)-2-[8-(5-fluorpyrimidin-2-yl)-3,8-diazabicyclo[3.2.1]oct-3-yl]ethanon;
- Verbindung Nr. 4: 2-[8-(5-Fluorpyrimidin-2-yl)-3,8-diazabicyclo[3.2.1]oct-3-yl]-1-(2-phenyl-2,4,6,7-tetrahydropyrazolo[4,3-c]pyridin-5-yl)ethanon;
- Verbindung Nr. 5: 6-{3-[2-Oxo-2-(2-phenyl-6,7-dihydro-4H-thiazolo[5,4-c]pyridin-5-yl)ethyl]-3,8-diazabicyclo[3.2.1]oct-8-yl}nicotinsäuremethylester;
- Verbindung Nr. 6: 6-{(3S,5R)-3,5-Dimethyl-4-[2-oxo-2-(2-phenyl-6,7-dihydro-4H-thiazolo[5,4-c]pyridin-5-yl)ethyl]piperazin-1-yl}nicotinsäure;
- Verbindung Nr. 7: 6-{3-[2-Oxo-2-(2-phenyl-6,7-dihydro-4H-thiazolo[5,4-c]pyridin-5-yl)ethyl]-3,8-diazabicyclo[3.2.1]oct-8-yl}nicotinsäure;
- Verbindung Nr. 8: 6-{(3S,5R)-3,5-Dimethyl-4-[2-oxo-2-(2-phenyl-6,7-dihydro-4H-thiazolo[5,4-c]pyridin-5-yl)ethyl]piperazin-1-yl}nicotinsäure;
- Verbindung Nr. 9: 2-[(2S,6R)-2,6-Dimethyl-4-(5-trifluormethylpyridin-2-yl)piperazin-1-yl]-1-(2-phenyl-6,7-dihydro-4H-thiazolo[5,4-c]pyridin-5-yl)ethanon;
- Verbindung Nr. 10: 2-[(2S,6R)-2,6-Dimethyl-4-(5-trifluormethylpyridin-2-yl)piperazin-1-yl]-1-(2-phenyl-2,4,6,7-tetrahydropyrazolo[4,3-c]pyridin-5-yl)ethanon;
- Verbindung Nr. 11: 4-[2-Oxo-2-(2-phenyl-6,7-dihydro-4H-thiazolo[5,4-c]pyridin-5-yl)ethyl]-1-(5-trifluormethylpyridin-2-yl)piperazin-2-one;
- Verbindung Nr. 12: 1-(2-Phenyl-6,7-dihydro-4H-thiazolo[5,4-c]pyridin-5-yl)-2-[4-(5-trifluormethylpyridin-2-yl)piperazin-1-yl]ethanon;
- Verbindung Nr. 13: 1-(2-Phenyl-2,4,6,7-tetrahydropyrazolo[4,3-c]pyridin-5-yl)-2-[4-(5-trifluormethylpyridin-2-yl)piperazin-1-yl]ethanon;
- Verbindung Nr. 14: 4-[2-Oxo-2-(2-phenyl-2,4,6,7-tetrahydropyrazolo[4,3-c]pyridin-5-yl)ethyl]-1-(5-trifluormethylpyridin-2-yl)piperazin-2-one;
- Verbindung Nr. 15: 2-[(2S, 6R) -4- (5-Fluorpyrimidin-2-yl)-2,6-dimethylpiperazin-1-yl]-1-(2-phenyl-6,7-dihydro-4H-thiazolo[5,4-c]pyridin-5-yl)ethanon;
- Verbindung Nr. 16: 2-((2S,6R)-2,6-Dimethyl-4-pyrimidin-5-yl-piperazin-1-yl)-1-(2-phenyl-2,4,6,7-tetrahydropyrazolo[4,3-c]pyridin-5-yl)ethanon;
- Verbindung Nr. 17: 2-[(2S,6R)-4-(5-Fluorpyrimidin-2-yl)-2,6-dimethylpiperazin-1-yl]-1-(2-phenyl-2,4,6,7-tetrahydropyrazolo[4,3-c]pyridin-5-yl)ethanon;
- Verbindung Nr. 18: 2-[(2S,6R)-2,6-Dimethyl-4-(6-trifluormethylpyridin-3-yl)piperazin-1-yl]-1-(2-phenyl-6,7-dihydro-4H-thiazolo[5,4-c]pyridin-5-yl)ethanon;
- Verbindung Nr. 19: 6-{3-[2-Oxo-2-(2-phenyl-2,4,6,7-tetrahydropyrazolo[4,3-c]pyridin-5-yl)ethyl]-3,8-diazabicyclo[3.2.1]oct-8-yl}nicotinsäuremethylester;
- Verbindung Nr. 20: 2-[(2S,6R)-2,6-Dimethyl-4-(6-trifluormethylpyridin-3-yl)piperazin-1-yl]-1-(2-phenyl-2,4,6,7-tetrahydropyrazolo[4,3-c]pyridin-5-yl)ethanon;
- Verbindung Nr. 21: 6-{(3S,5R)-3,5-Dimethyl-4-[2-oxo-2-(2-phenyl-2,4,6,7-tetrahydropyrazolo[4,3-c]pyridin-5-yl)ethyl]piperazin-1-yl}nicotinsäure;
- Verbindung Nr. 22: 2-((2S,6R)-2,6-Dimethyl-4-pyrimidin-5-yl-piperazin-1-yl)-1-(2-phenyl-6,7-dihydro-4H-thiazolo[5,4-c]pyridin-5-yl)ethanon;
- Verbindung Nr. 23: 6-{(3S,5R)-3,5-Dimethyl-4-[2-oxo-2-(2-phenyl-2,4,6,7-tetrahydropyrazolo[4,3-c]pyridin-5-yl)ethyl]piperazin-1-yl}nicotinsäuremethylester;
- Verbindung Nr. 24: 6-{3-[2-Oxo-2-(2-phenyl-2,4,6,7-tetrahydropyrazolo[4,3-c]pyridin-5-yl)ethyl]-3,8-diazabicyclo[3.2.1]oct-8-yl}nicotinsäure;
- Verbindung Nr. 25: 6-{2-Oxo-4-[2-oxo-2-(2-phenyl-6,7-dihydro-4H-thiazolo[5,4-c]pyridin-5-yl)ethyl]piperazin-1-yl}nicotinsäuremethylester;
- Verbindung Nr. 26: 2-[8-(5-Fluorpyrimidin-2-yl)-3,8-diazabicyclo[3.2.1]oct-3-yl]-1-(1-phenyl-1,4,6,7-tetrahydropyrazolo[4,3-c]pyridin-5-yl)ethanon;
- Verbindung Nr. 27: 1-(2-Phenyl-2,4,6,7-tetrahydropyrazolo[4,3-c]pyridin-5-yl)-2-(4-pyridin-3-yl[1,4]diazepan-1-yl)ethanon;
- Verbindung Nr. 28: 1-(2-Phenyl-2,4,6,7-tetrahydropyrazolo[4,3-c]pyridin-5-yl)-2-(8-pyridin-3-yl-3,8-diazabicyclo[3.2.1]oct-3-yl)ethanon;
- Verbindung Nr. 29: 1-(2-Phenyl-2,4,6,7-tetrahydropyrazolo[4,3-c]pyridin-5-yl)-2-[8-(5-trifluormethylpyridin-2-yl)-3,8-diazabicyclo[3.2.1]oct-3-yl]ethanon;
- Verbindung Nr. 30: 1-(2-Phenyl-6,7-dihydro-4H-thiazolo[5,4-c]pyridin-5-yl)-2-(4-pyridin-3-yl-[1,4]diazepan-1-yl)ethanon;
- Verbindung Nr. 31: 1-(2-Phenyl-2,4,6,7-tetrahydropyrazolo[4,3-c]pyridin-5-yl)-2-(8-pyrimidin-5-yl-3,8-diazabicyclo[3.2.1]oct-3-yl)ethanon;
- Verbindung Nr. 32: 2-[8-(5-Fluorpyrimidin-2-yl)-3,8-diazabicyclo[3.2.1]oct-3-yl]-1-(2-methyl-2,4,6,7-tetrahydropyrazolo[4,3-c]pyridin-5-yl)ethanon;
- Verbindung Nr. 33: 6-{3-[2-Oxo-2-(2-phenyl-4,6-dihydropyrrolo[3,4-d]thiazol-5-yl)ethyl]-3,8-diazabicyclo[3.2.1]oct-8-yl}nicotinsäuremethylester;
- Verbindung Nr. 34: 4-{2-[2-(4-Methoxyphenyl)-2,4,6,7-tetrahydropyrazolo[4,3-c]pyridin-5-yl]-2-oxoethyl}-1-(5-trifluormethylpyridin-2-yl)piperazin-2-on;
- Verbindung Nr. 35: 4-{2-[2-(4-Fluorphenyl)-2,4,6,7-tetrahydropyrazolo[4,3-c]pyridin-5-yl]-2-oxoethyl}-1-(5-trifluormethylpyridin-2-yl)piperazin-2-on;
- Verbindung Nr. 36: 2-[8-(5-Fluorpyrimidin-2-yl)-3,8-diazabicyclo[3.2.1]oct-3-yl]-1-(2-methyl-6,7-dihydro-4H-thiazolo[5,4-c]pyridin-5-yl)ethanon;
- Verbindung Nr. 37: 6-(3-{2-[2-(4-Methoxyphenyl)-6,7-dihydro-4H-thiazolo[5,4-c]pyridin-5-yl]-2-oxoethyl}-3,8-diazabicyclo[3.2.1]oct-8-yl)nicotinsäuremethylester;
- Verbindung Nr. 38: 6-(3-{2-[2-(4-Fluorphenyl)-6,7-dihydro-4H-thiazolo[5,4-c]pyridin-5-yl]-2-oxoethyl}-3,8-diazabicyclo[3.2.1]oct-8-yl)nicotinsäuremethylester;
- Verbindung Nr. 39: 6-(3-{2-Oxo-2-[2-(5-trifluormethylpyridin-2-yl)-2,4,6,7-tetrahydropyrazolo[4,3-c]pyridin-5-yl]ethyl}-3,8-diazabicyclo[3.2.1]oct-8-yl)nicotinsäuremethylester;
- Verbindung Nr. 40: 2-[8-(5-Fluorpyrimidin-2-yl)-3,8-diazabicyclo[3.2.1]oct-3-yl]-1-[2-(5-trifluormethylpyridin-2-yl)-2,4,6,7-tetrahydropyrazolo[4,3-c]pyridin-5-yl]ethanon;
- Verbindung Nr. 41: 4-[2-Oxo-2-(2-phenyl-7,8-dihydro-5H-pyrido[4,3-d]pyrimidin-6-yl)ethyl]-1-(5-trifluormethylpyridin-2-yl)piperazin-2-on;
- Verbindung Nr. 42: 4-[2-Oxo-2-(2-thiophen-3-yl-6,7-dihydro-4H-thiazolo[5,4-c]pyridin-5-yl)ethyl]-1-(5-trifluormethylpyridin-2-yl)piperazin-2-on;
- Verbindung Nr. 43: 2-[8-(5-Fluorpyrimidin-2-yl)-3,8-diazabicyclo[3.2.1]oct-3-yl]-1-[2-(4-methoxyphenyl)-2,4,6,7-tetrahydropyrazolo[4,3-c]pyridin-5-yl]ethanon;
- Verbindung Nr. 44: 6-{3-[2-Oxo-2-(2-thiophen-3-yl-6,7-dihydro-4H-thiazolo[5,4-c]pyridin-5-yl)ethyl]-3,8-diazabicyclo[3.2.1]oct-8-yl}nicotinsäure;
- Verbindung Nr. 45: 2-[8-(5-Fluorpyrimidin-2-yl)-3,8-diazabicyclo[3.2.1]oct-3-yl]-1-[1-(2,2,2-trifluorethyl)-4,5,6,7-tetrahydro-1H-indazol-5-yl]ethanon;
- Verbindung Nr. 46: 2-[8-(5-Fluorpyrimidin-2-yl)-3,8-diazabicyclo[3.2.1]oct-3-yl]-1-[1-(4-methoxyphenyl)-1,4,6,7-tetrahydropyrazolo[4,3-c]pyridin-5-yl]ethanon;
- Verbindung Nr. 47: 2-[8-(5-Fluorpyrimidin-2-yl)-3,8-diazabicyclo[3.2.1]oct-3-yl]-1-(2,4,6,7-tetrahydropyrazolo[4,3-c]pyridin-5-yl)ethanon;
- Verbindung Nr. 48: 6-{3-[2-Oxo-2-(1-phenyl-1,4,6,7-tetrahydropyrazolo[4,3-c]pyridin-5-yl)ethyl]-3,8-diazabicyclo[3.2.1]oct-8-yl}nicotinsäuremethylester;
- Verbindung Nr. 49: 6-{3-[2-Oxo-2-(1-phenyl-1,4,6,7-tetrahydropyrazolo[4,3-c]pyridin-5-yl)ethyl]-3,8-diazabicyclo[3.2.1]oct-8-yl}nicotinsäure;
- Verbindung Nr. 50: 1-(1-tert-Butyl-1,4,6,7-tetrahydropyrazolo[4,3-c]pyridin-5-yl)-2-[8-(5-fluorpyrimidin-2-yl)-3,8-diazabicyclo[3.2.1]oct-3-yl]ethanon;
- Verbindung Nr. 51: 1-[1-(4-Fluorphenyl)-1,4,6,7-tetrahydropyrazolo[4,3-c]pyridin-5-yl]-2-[8-(5-fluorpyrimidin-2-yl)-3,8-diazabicyclo[3.2.1]oct-3-yl]ethanon;
- Verbindung Nr. 52: 6-{(2R,5S)-2,5-Dimethyl-4-[2-oxo-2-(2-phenyl-2,4,6,7-tetrahydropyrazolo[4,3-c]pyridin-5-yl)ethyl]piperazin-1-yl}nicotinonitril;
- Verbindung Nr. 53: 6-{(2R,5S)-2,5-Dimethyl-4-[2-oxo-2-(2-phenyl-2,4,6,7-tetrahydropyrazolo[4,3-c]pyridin-5-yl)ethyl]piperazin-1-yl}nicotinsäure;
- Verbindung Nr. 54: 2-[8-(5-Fluorpyrimidin-2-yl)-3,8-diazabicyclo[3.2.1]oct-3-yl]-1-(2-phenyl-4,7-dihydro-5H-fluor[2,3-c]pyridin-6-yl)ethanon;
- Verbindung Nr. 55: 2-[8-(5-Fluorpyrimidin-2-yl)-3,8-diazabicyclo[3.2.1]oct-3-yl]-1-(2-phenyl-1,4,5,7-tetrahydropyrrolo[2,3-c]pyridin-6-yl)ethanon;
- Verbindung Nr. 56: 6-{8-[2-Oxo-2-(2-phenyl-6,7-dihydro-4H-thiazolo[5,4-c]pyridin-5-yl)ethyl]-3,8-diazabicyclo[3.2.1]oct-3-yl}nicotinsäuremethylester;
- Verbindung Nr. 57: 6-{8-[2-Oxo-2-(2-phenyl-2,4,6,7-tetrahydropyrazolo[4,3-c]pyridin-5-yl)ethyl]-3,8-diazabicyclo[3.2.1]oct-3-yl}nicotinsäuremethylester;
- Verbindung Nr. 58: 2-[8-(5-Fluorpyrimidin-2-yl)-3,8-diazabicyclo[3.2.1]oct-3-yl]-1-(2-phenyl-6,7-dihydro-4H-oxazolo[4,5-c]pyridin-5-yl)ethanon;
- Verbindung Nr. 59: 6-{(2R,5S)-2,5-Dimethyl-4-[2-oxo-2-(2-phenyl-6,7-dihydro-4H-thiazolo[5,4-c]pyridin-5-yl)ethyl]piperazin-1-yl}nicotinonitril;
- Verbindung Nr. 60: 2-{8-[5-(5-Methyl-[1,2,4]oxadiazol-3-yl)pyridin-2-yl]-3,8-diazabicyclo[3.2.1]oct-3-yl}-1-(2-phenyl-6,7-dihydro-4H-thiazolo[5,4-c]pyridin-5-yl)ethanon;
- Verbindung Nr. 61: 6-{8-[2-Oxo-2-(2-phenyl-2,4,6,7-tetrahydropyrazolo[4,3-c] pyridin-5-yl)ethyl]-3,8-diazabicyclo[3.2.1]oct-3-yl}nicotinsäure;
- Verbindung Nr. 62: 6-{8-[2-Oxo-2-(2-phenyl-6,7-dihydro-4H-thiazolo[5,4-c]pyridin-5-yl)ethyl]-3,8-diazabicyclo[3.2.1]oct-3-yl}nicotinsäure;
- Verbindung Nr. 63: 6-{(2R,5S)-2,5-Dimethyl-4-[2-oxo-2-(2-phenyl-6,7-dihydro-4H-thiazolo[5,4-c]pyridin-5-yl)ethyl]piperazin-1-yl}nicotinsäure;
- Verbindung Nr. 64: 2-[8-(5-Fluorpyrimidin-2-yl)-3,8-diazabicyclo[3.2.1]oct-3-yl]-1-(2-pyridin-4-yl-6,7-dihydro-4H-thiazolo[5,4-c]pyridin-5-yl)ethanon;
- Verbindung Nr. 65: 4-{2-Oxo-2-[2-(5-trifluormethylpyridin-2-yl)-2,4,6,7-tetrahydropyrazolo[4,3-c]pyridin-5-yl]ethyl}-1-(5-trifluormethylpyridin-2-yl)piperazin-2-on;
- Verbindung Nr. 66: 4-{2-Oxo-2-[1-(2,2,2-trifluorethyl)-1,4,6,7-tetrahydropyrazolo[4,3-c]pyridin-5-yl]ethyl}-1-(5-trifluormethylpyridin-2-yl)piperazin-2-on;
- Verbindung Nr. 67: 2-[8-(5-Fluorpyrimidin-2-yl)-3,8-diazabicyclo[3.2.1]oct-3-yl]-1-(2-phenyl-7,8-dihydro-5H-pyrido[4,3-d]pyrimidin-6-yl)ethanon;
- Verbindung Nr. 68: 3-(6-{3-[2-Oxo-2-(2-phenyl-2,4,6,7-tetrahydropyrazolo[4,3-c] pyridin-5-yl)ethyl]-3,8-diazabicyclo[3.2.1]oct-8-yl}pyridin-3-yl)-4H-[1,2,4]oxadiazol-5-on;
- Verbindung Nr. 69: 2-[8-(5-Fluorpyrimidin-2-yl)-3,8-diazabicyclo[3.2.1]oct-3-yl]-1-(2-phenyl-3,4,6,7-tetrahydroimidazo[4,5-c]pyridin-5-yl)ethanon;
- Verbindung Nr. 70: 3-(6-{3-[2-Oxo-2-(2-phenyl-6,7-dihydro-4H-thiazolo[5,4-c]pyridin-5-yl)ethyl]-3,8-diazabicyclo[3.2.1]oct-8-yl}pyridin-3-yl)-4H-[1,2,4]oxadiazol-5-on;
- Verbindung Nr. 71: 2-[8-(5-Fluorpyrimidin-2-yl)-3,8-diazabicyclo[3.2.1]oct-3-yl]-1-(2-phenyl-6,7-dihydro-4H-thiazolo[4,5-c]pyridin-5-yl)ethanon;
- Verbindung Nr. 72: 2-{(3S,5R)-3,5-Dimethyl-4-[2-oxo-2-(2-phenyl-6,7-dihydro-4H-thiazolo[5,4-c]pyridin-5-yl)ethyl]piperazin-1-yl}pyrimidin-5-carbonsäure;
- Verbindung Nr. 73: 2-[8-(5-Fluorpyrimidin-2-yl)-3,8-diazabicyclo[3.2.1]oct-3-yl]-1-(2-pyridin-3-yl-6,7-dihydro-4H-thiazolo[5,4-c]pyridin-5-yl)ethanon;
- Verbindung Nr. 74: 1-(2-Phenyl-6,7-dihydro-4H-thiazolo[5,4-c]pyridin-5-yl)-2-{8-[5-(1H-tetrazol-5-yl)pyridin-2-yl]-3,8-diazabicyclo[3.2.1]oct-3-yl}ethanon;
- Verbindung Nr. 75: 2-{(3S,5R)-3,5-Dimethyl-4-[2-oxo-2-(2-phenyl-2,4,6,7-tetrahydropyrazolo[4,3-c]pyridin-5-yl)ethyl]piperazin-1-yl}pyrimidin-5-carbonsäure;
- Verbindung Nr. 76: 6-(3-{2-[2-(4-Fluorphenyl)-6,7-dihydro-4H-thiazolo[5,4-c]pyridin-5-yl]-2-oxoethyl}-3,8-diazabicyclo[3.2.1]oct-8-yl)nicotinonitril;
- Verbindung Nr. 77: 6-{3-[2-Oxo-2-(2-phenyl-2,4,6,7-tetrahydropyrazolo[4,3-c]pyridin-5-yl)ethyl]-3,8-diazabicyclo[3.2.1]oct-8-yl}nicotinonitril;
- Verbindung Nr. 78: 4-[2-Oxo-2-(2-phenyl-3,4,6,7-tetrahydroimidazo[4,5-c]pyridin-5-yl)ethyl]-1-(5-trifluormethylpyridin-2-yl)piperazin-2-on;
- Verbindung Nr. 79: 6-{(3S,5R)-3,5-Dimethyl-4-[2-oxo-2-(2-phenyl-2,4,6,7-tetrahydropyrazolo[4,3-c]pyridin-5-yl)ethyl]piperazin-1-yl}nicotinsäureethylester;
- Verbindung Nr. 80: 1-[2-(4-Fluorphenyl)-6,7-dihydro-4H-thiazolo[5,4-c]pyridin-5-yl]-2-[8-(5-fluorpyrimidin-2-yl)-3,8-diazabicyclo[3.2.1]oct-3-yl]ethanon;
- Verbindung Nr. 81: 2-[8-(5-Fluorpyrimidin-2-yl)-3,8-diazabicyclo[3.2.1]oct-3-yl]-1-[2-(4-methoxyphenyl)-6,7-dihydro-4H-thiazolo[5,4-c]pyridin-5-yl]ethanon;
- Verbindung Nr. 82: 4-[2-Oxo-2-(2-phenyl-6,7-dihydro-4H-thiazolo[4,5-c]pyridin-5-yl)ethyl]-1-(5-trifluormethylpyridin-2-yl)piperazin-2-on;
- Verbindung Nr. 83: 4-[2-Oxo-2-(2-phenyl-6,7-dihydro-4H-oxazolo[4,5-c]pyridin-5-yl)ethyl]-1-(5-trifluormethylpyridin-2-yl)piperazin-2-on;
- Verbindung Nr. 84: 4-[2-Oxo-2-(2-pyridin-3-yl-6,7-dihydro-4H-thiazolo[5,4-c]pyridin-5-yl)ethyl]-1-(5-trifluormethylpyridin-2-yl)piperazin-2-on;
- Verbindung Nr. 85: 2-{8-[5-(2-Methyl-2H-tetrazol-5-yl)pyridin-2-yl]-3,8-diazabicyclo[3.2.1]oct-3-yl}-1-(2-phenyl-2,4,6,7-tetrahydropyrazolo[4,3-c]pyridin-5-yl)ethanon;
- Verbindung Nr. 86: 2-{(2S,6R)-2,6-Dimethyl-4-[5-(1-methyl-1H-tetrazol-5-yl)-pyridin-2-yl]piperazin-1-yl}-1-(2-phenyl-2,4,6,7-tetrahydropyrazolo[4,3-c]pyridin-5-yl)ethanon;
- Verbindung Nr. 87: 2-{(2S,6R)-2,6-Dimethyl-4-[5-(2-methyl-2H-tetrazol-5-yl)-pyridin-2-yl]piperazin-1-yl}-1-(2-phenyl-2,4,6,7-tetrahydropyrazolo[4,3-c]pyridin-5-yl)ethanon;
- Verbindung Nr. 88: 2-[8-(5-Fluorpyrimidin-2-yl)-3,8-diazabicyclo[3.2.1]oct-3-yl]-1-[1-(4-trifluormethylphenyl)-1,4,6,7-tetrahydropyrazolo[4,3-c]pyridin-5-yl]ethanon;
- Verbindung Nr. 89: 2-{(2S,6R)-2,6-Dimethyl-4-[5-(5-methyl[1,2,4]oxadiazol-3-yl)-pyridin-2-yl]piperazin-1-yl}-1-(2-phenyl-2,4,6,7-tetrahydropyrazolo[4,3-c]pyridin-5-yl)ethanon;
- Verbindung Nr. 90: 2-[(2S,6R)-2,6-Dimethyl-4-(5-[1,3,4]oxadiazol-2-yl-pyridin-2-yl)piperazin-1-yl]-1-(2-phenyl-2,4,6,7-tetrahydropyrazolo[4,3-c]pyridin-5-yl)ethanon;
- Verbindung Nr. 91: 2-{(2S,6R)-2,6-Dimethyl-4-[5-(3-methyl-[1,2,4]oxadiazol-5-yl)-pyridin-2-yl]piperazin-1-yl}-1-(2-phenyl-2,4,6,7-tetrahydropyrazolo[4,3-c]pyridin-5-yl)ethanon;
- Verbindung Nr. 92: 6-{(3S,5R)-4-[2-(1-tert-Butyl-1,4,6,7-tetrahydropyrazolo[4,3-c]pyridin-5-yl)-2-oxoethyl]-3,5-dimethylpiperazin-1-yl}nicotinsäuremethylester;
- Verbindung Nr. 93: 6-{(3S,5R)-4-[2-(1-tert-Butyl-1,4,6,7-tetrahydropyrazolo[4,3-c]pyridin-5-yl)-2-oxoethyl]-3,5-dimethylpiperazin-1-yl}nicotinsäure;
- Verbindung Nr. 94: 6-{3-[2-(1-tert-Butyl-1,4,6,7-tetrahydropyrazolo[4,3-c]pyridin-5-yl)-2-oxoethyl]-3,8-diazabicyclo[3.2.1]oct-8-yl}nicotinsäure;
- Verbindung Nr. 95: 2-[8-(5-Fluorpyrimidin-2-yl)-3,8-diazabicyclo[3.2.1]oct-3-yl]-1-(2-phenyl-6,7-dihydro-4H-oxazolo[5,4-c]pyridin-5-yl)ethanon;
- Verbindung Nr. 96: 4-[2-Oxo-2-(2-phenyl-6,7-dihydro-4H-oxazolo[5,4-c]pyridin-5-yl)ethyl]-1-(5-trifluormethylpyridin-2-yl)piperazin-2-on;
- Verbindung Nr. 97: 2-[(2S,6R)-2,6-Dimethyl-4-(5-trifluormethylpyridin-2-yl)piperazin-1-yl]-1-(2-phenyl-4,7-dihydro-5H-fluor[2,3-c]pyridin-6-yl)ethanon;
- Verbindung Nr. 98: 6-{(3S,5R)-3,5-Dimethyl-4-[2-oxo-2-(2-phenyl-2,4,6,7-tetrahydropyrazolo[4,3-c]pyridin-5-yl)ethyl]piperazin-1-yl}nicotinsäureisopropylester;
- Verbindung Nr. 99: 1-(2-Methyl-2,4,6,7-tetrahydropyrazolo[4,3-c]pyridin-5-yl)-2-[8-(5-trifluormethylpyridin-2-yl)-3,8-diazabicyclo[3.2.1]oct-3-yl]ethanon;
- Verbindung Nr. 100: 2-[(2S,6R)-2,6-Dimethyl-4-(5-trifluormethylpyridin-2-yl)piperazin-1-yl]-1-(2-methyl-2,4,6,7-tetrahydropyrazolo[4,3-c]pyridin-5-yl)ethanon;
- Verbindung Nr. 101: 1-(2-Phenyl-4,7-dihydro-5H-fluor[2,3-c]pyridin-6-yl)-2-[8-(5-trifluormethylpyridin-2-yl)-3,8-diazabicyclo[3.2.1]oct-3-yl]ethanon;
- Verbindung Nr. 102: 6-(3-{2-[2-(4-Fluorphenyl)-6,7-dihydro-4H-thiazolo[5,4-c] pyridin-5-yl]-2-oxoethyl}-3,8-diazabicyclo[3.2.1]oct-8-yl)nicotinsäure;
- Verbindung Nr. 103: 2-[(2S,6R)-2,6-Dimethyl-4-(5-trifluormethylpyridin-2-yl)piperazin-1-yl]-1-[2-(5-trifluormethylpyridin-2-yl)-2,4,6,7-tetrahydropyrazolo[4,3-c]pyridin-5-yl]ethanon;
- Verbindung Nr. 104: 2-[8-(5-Trifluormethylpyridin-2-yl)-3,8-diazabicyclo[3.2.1]oct-3-yl]-1-[2-(5-trifluormethylpyridin-2-yl)-2,4,6,7-tetrahydropyrazolo[4,3-c]pyridin-5-yl]ethanon;
- Verbindung Nr. 105: 6-{(3S,5R)-4-[2-(2-tert-Butyl-2,4,6,7-tetrahydropyrazolo[4,3-c]pyridin-5-yl)-2-oxoethyl]-3,5-dimethylpiperazin-1-yl}nicotinsäure;
- Verbindung Nr. 106: 2-(8-Pyridin-3-yl-3,8-diazabicyclo[3.2.1]oct-3-yl)-1-[2-(5-trifluormethylpyridin-2-yl)-2,4,6,7-tetrahydropyrazolo[4,3-c]pyridin-5-yl]ethanon;
- Verbindung Nr. 107: 2-[5-(6-Trifluormethylpyridazin-3-yl)-2,5-diazabicyclo[2.2.1]hept-2-yl]-1-[2-(5-trifluormethylpyridin-2-yl)-2,4,6,7-tetrahydropyrazolo[4,3-c] pyridin-5-yl]ethanon;
- Verbindung Nr. 108: 2-(6'-Chlor-2,3,5,6-tetrahydro[1,2']bipyrazinyl-4-yl)-1-[2-(5-trifluormethylpyridin-2-yl)-2,4,6,7-tetrahydropyrazolo[4,3-c]pyridin-5-yl]ethanon;
- Verbindung Nr. 109: 1-(2-Phenyl-3,4,6,7-tetrahydroimidazo[4,5-c]pyridin-5-yl)-2-[8-(5-trifluormethylpyridin-2-yl)-3,8-diazabicyclo[3.2.1]oct-3-yl]ethanon;
- Verbindung Nr. 110: 2-[(2S,6R)-2,6-Dimethyl-4-(5-trifluormethylpyridin-2-yl)piperazin-1-yl]-1-(2-phenyl-3,4,6,7-tetrahydroimidazo[4,5-c]pyridin-5-yl)ethanon;
- Verbindung Nr. 111: 2-((3S,5R)-3,5-Dimethyl-2,3,5,6-tetrahydro[1,2']bipyrazinyl-4-yl)-1-[2-(5-trifluormethylpyridin-2-yl)-2,4,6,7-tetrahydropyrazolo[4,3-c]pyridin-5-yl]ethanon;
- Verbindung Nr. 112: 2-[(2S,6R)-4-(5-Chlorpyridin-2-yl)-2,6-dimethylpiperazin-1-yl]-1-[2-(5-trifluormethylpyridin-2-yl)-2,4,6,7-tetrahydropyrazolo[4,3-c]pyridin-5-yl]ethanon;
- Verbindung Nr. 113: 2-[4-(7-Chlorchinolin-4-yl)piperazin-1-yl]-1-(2-phenyl-2,4,6,7-tetrahydropyrazolo[4,3-c]pyridin-5-yl)ethanon;
- Verbindung Nr. 114: 2-[4-(6-Chlorpyridin-2-yl)piperazin-1-yl]-1-(2-phenyl-2,4,6,7-tetrahydropyrazolo[4,3-c]pyridin-5-yl)ethanon;
- Verbindung Nr. 115: 1-(2-Pyridin-4-yl-6,7-dihydro-4H-thiazolo[5,4-c]pyridin-5-yl)-2-[8-(5-trifluormethylpyridin-2-yl)-3,8-diazabicyclo[3.2.1]oct-3-yl]ethanon;
- Verbindung Nr. 116: 6-{(3S,5R)-3,5-Dimethyl-4-[2-oxo-2-(2-phenyl-3,4,6,7-tetrahydroimidazo[4,5-c]pyridin-5-yl)ethyl]piperazin-1-yl}nicotinsäure;
- Verbindung Nr. 117: 1-(2-Pyridin-2-yl-2,4,6,7-tetrahydropyrazolo[4,3-c]pyridin-5-yl)-2-[8-(5-trifluormethylpyridin-2-yl)-3,8-diazabicyclo[3.2.1]oct-3-yl]ethanon;
- Verbindung Nr. 118: 2-[(2S,6R)-2,6-Dimethyl-4-(5-trifluormethylpyridin-2-yl)piperazin-1-yl]-1-(2-pyridin-2-yl-2,4,6,7-tetrahydropyrazolo[4,3-c]pyridin-5-yl)ethanon;
- Verbindung Nr. 119: 4-[2-Oxo-2-(2-pyridin-2-yl-2,4,6,7-tetrahydropyrazolo[4,3-c]pyridin-5-yl)ethyl]-1-(5-trifluormethylpyridin-2-yl)piperazin-2-on;
- Verbindung Nr. 120: 4-{2-[2-(4-Fluorphenyl)-3,4,6,7-tetrahydroimidazo[4,5-c]pyridin-5-yl]-2-oxoethyl}-1-(5-trifluormethylpyridin-2-yl)piperazin-2-on;
- Verbindung Nr. 121: 2-(8-Pyridin-3-yl-3,8-diazabicyclo[3.2.1]oct-3-yl)-1-(2-pyridin-2-yl-2,4,6,7-tetrahydropyrazolo[4,3-c]pyridin-5-yl)ethanon;
- Verbindung Nr. 122: 1-[2-(2,2,2-Trifluorethyl)-2,4,6,7-tetrahydropyrazolo[4,3-c]pyridin-5-yl]-2-[8-(5-trifluormethylpyridin-2-yl)-3,8-diazabicyclo[3.2.1]oct-3-yl]ethanon;
- Verbindung Nr. 123: 1-[2-(4-Fluorphenyl)-3,4,6,7-tetrahydroimidazo[4,5-c]pyridin-5-yl]-2-[8-(5-trifluormethylpyridin-2-yl)-3,8-diazabicyclo[3.2.1]oct-3-yl]ethanon;
- Verbindung Nr. 124: 2-[(2S,6R)-2,6-Dimethyl-4-(5-trifluormethylpyridin-2-yl)piperazin-1-yl]-1-[2-(4-fluorphenyl)-3,4,6,7-tetrahydroimidazo[4,5-c]pyridin-5-yl]ethanon;
- Verbindung Nr. 125: 4-{2-Oxo-2-[2-(2,2,2-trifluorethyl)-2,4,6,7-tetrahydropyrazolo[4,3-c]pyridin-5-yl]ethyl}-1-(5-trifluormethylpyridin-2-yl)piperazin-2-on;
- Verbindung Nr. 126: 2-[(2S,6R)-2,6-Dimethyl-4-(5-trifluormethylpyridin-2-yl)piperazin-1-yl]-1-[2-(2,2,2-trifluorethyl)-2,4,6,7-tetrahydropyrazolo[4,3-c]pyridin-5-yl]ethanon;
- Verbindung Nr. 127: 1-(2-Phenyl-2,4,6,7-tetrahydropyrazolo[4,3-c]pyridin-5-yl)-2-[5-(5-trifluormethylpyridin-2-yl)-2,5-diazabicyclo[2.2.1]hept-2-yl]ethanon;
- Verbindung Nr. 128: 6-{3-[2-Oxo-2-(2-phenyl-2,4,6,7-tetrahydropyrazolo[4,3-c]pyridin-5-yl)ethyl]-3,8-diazabicyclo[3.2.1]oct-8-yl}nicotinsäurecyclobutylester;
- Verbindung Nr. 129: 2-((2S,6R)-2,6-Dimethyl-4-chinolin-2-ylpiperazin-1-yl)-1-(2-phenyl-2,4,6,7-tetrahydropyrazolo[4,3-c]pyridin-5-yl)ethanon;
- Verbindung Nr. 130: 6-{(3S,5R)-3,5-Dimethyl-4-[2-oxo-2-(2-phenyl-2,4,6,7-tetrahydropyrazolo[4,3-c]pyridin-5-yl)ethyl]piperazin-1-yl}nicotinsäureethylester;
- Verbindung Nr. 131: 2-[(2S,6R)-4-(5-Methansulfonylpyridin-2-yl)-2,6-dimethylpiperazin-1-yl]-1-(2-phenyl-2,4,6,7-tetrahydropyrazolo[4,3-c]pyridin-5-yl)ethanon;
- Verbindung Nr. 132: 2-[(2S,6R)-4-(5-Fluorpyrimidin-2-yl)-2,6-dimethylpiperazin-1-yl]-1-[2-(4-methoxyphenyl)-6,7-dihydro-4H-thiazolo[5,4-c]pyridin-5-yl]ethanon;
- Verbindung Nr. 133: 1-[2-(4-Methoxyphenyl)-6,7-dihydro-4H-thiazolo[5,4-c]pyridin-5-yl]-2-[5-(5-trifluormethylpyridin-2-yl)-2,5-diazabicyclo[2.2.1]hept-2-yl]ethanon;
- Verbindung Nr. 134: 1-(2-Phenyl-6,7-dihydro-4H-thiazolo[5,4-c]pyridin-5-yl)-2-{4-[5-(2H-pyrazol-3-yl)pyridin-2-yl]piperazin-1-yl}ethanon;
- Verbindung Nr. 135: 2-[(2S,6R)-2,6-Dimethyl-4-(5-thiazol-2-ylpyridin-2-yl)piperazin-1-yl]-1-(2-phenyl-2,4,6,7-tetrahydropyrazolo[4,3-c]pyridin-5-yl)ethanon;
- Verbindung Nr. 136: 1-(2-Phenyl-6,7-dihydro-4H-thiazolo[5,4-c]pyridin-5-yl)-2-[8-(5-thiazol-2-ylpyridin-2-yl)-3,8-diazabicyclo[3.2.1]oct-3-yl]ethanon;
- Verbindung Nr. 137: 2-[8-(5-[1,2,4]Oxadiazol-3-ylpyridin-2-yl)-3,8-diazabicyclo[3.2.1]oct-3-yl]-1-(2-phenyl-2,4,6,7-tetrahydropyrazolo[4,3-c]pyridin-5-yl)ethanon;
- Verbindung Nr. 138: 1-(2-Ethyl-2,4,6,7-tetrahydropyrazolo[4,3-c]pyridin-5-yl)-2-[8-(5-trifluormethylpyridin-2-yl)-3,8-diazabicyclo[3.2.1]oct-3-yl]ethanon;
- Verbindung Nr. 139: 2-[(2S,6R)-2,6-Dimethyl-4-(5-[1,2,4]oxadiazol-5-ylpyridin-2-yl)piperazin-1-yl]-1-(2-phenyl-2,4,6,7-tetrahydropyrazolo[4,3-c]pyridin-5-yl)ethanon;
- Verbindung Nr. 140: 6-{(3S,5R)-3,5-Dimethyl-4-[2-oxo-2-(2-pyridin-2-yl-2,4,6,7-tetrahydropyrazolo[4,3-c]pyridin-5-yl)ethyl]piperazin-1-yl}nicotinsäure;
- Verbindung Nr. 141: 2-((2S,6R)-2,6-Dimethyl-4-pyridin-3-ylpiperazin-1-yl)-1-(2-pyridin-4-yl-2,4,6,7-tetrahydropyrazolo[4,3-c]pyridin-5-yl)ethanon;
- Verbindung Nr. 142: 2-((2S,6R)-2,6-Dimethyl-4-pyridin-3-ylpiperazin-1-yl)-1-[2-(5-fluorpyridin-2-yl)-2,4,6,7-tetrahydropyrazolo[4,3-c]pyridin-5-yl]ethanon;
- Verbindung Nr. 143: 2-((2S,6R)-2,6-Dimethyl-4-pyridin-3-ylpiperazin-1-yl)-1-[2-(5-trifluormethylpyridin-2-yl)-2,4,6,7-tetrahydropyrazolo[4,3-c]pyridin-5-yl]ethanon;
- Verbindung Nr. 144: 6-{(3S,5R)-3,5-Dimethyl-4-[2-oxo-2-(2-pyridin-2-yl-2,4,6,7-tetrahydropyrazolo[4,3-c]pyridin-5-yl)ethyl]piperazin-1-yl}nicotinsäuremethylester;
- Verbindung Nr. 145: 2-((2S,6R)-2,6-Dimethyl-4-pyridin-3-ylpiperazin-1-yl)-1-(2-phenyl-3,4,6,7-tetrahydroimidazo[4,5-c]pyridin-5-yl)ethanon;
- Verbindung Nr. 146: 2-[(2S,6R)-2,6-Dimethyl-4-(5-trifluormethylpyridin-2-yl)piperazin-1-yl]-1-(2-pyridazin-3-yl-2,4,6,7-tetrahydropyrazolo[4,3-c]pyridin-5-yl)ethanon;
- Verbindung Nr. 147: 2-[(2S,6R)-2,6-Dimethyl-4-(5-trifluormethylpyridin-2-yl)piperazin-1-yl]-1-(2-ethyl-2,4,6,7-tetrahydropyrazolo[4,3-c]pyridin-5-yl)ethanon;
- Verbindung Nr. 148: 4-[2-(2-Ethyl-2,4,6,7-tetrahydropyrazolo[4,3-c]pyridin-5-yl)-2-oxoethyl]-1-(5-trifluormethylpyridin-2-yl)piperazin-2-on;
- Verbindung Nr. 149: 6-{(3S,5R)-3,5-Dimethyl-4-[2-oxo-2-(2-phenyl-2,4,6,7-tetrahydropyrazolo[4,3-c]pyridin-5-yl)ethyl]piperazin-1-yl}nicotinsäure;
- Verbindung Nr. 150: 6-{(3S,5R)-3,5-Dimethyl-4-[2-oxo-2-(2-phenyl-2,4,6,7-tetrahydropyrazolo[4,3-c]pyridin-5-yl)ethyl]piperazin-1-yl}nicotinsäure;
in Basen- oder Säureadditionssalzform.

9. Verbindung nach einem der Ansprüche 1 bis 7, ausgewählt aus den folgenden Verbindungen:

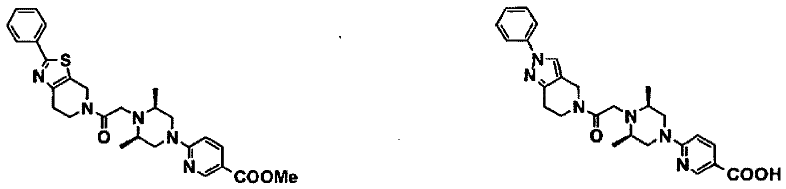

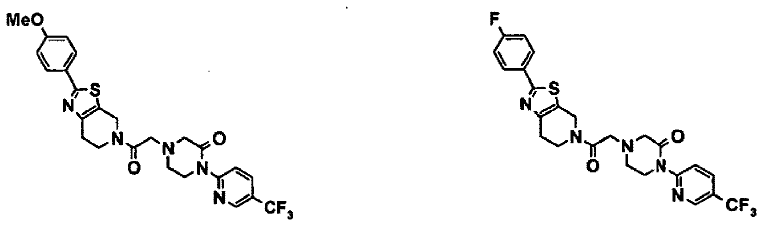

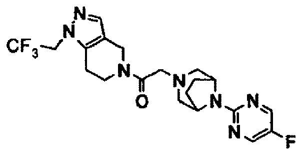

10. Verbindung nach einem der vorhergehenden Ansprüche, bei der es sich um 6-{(3S,5R)-3,5-Dimethyl-4-[2-oxo-2-(2-phenyl-2,4,6,7-tetrahydropyrazolo[4,3-c]pyridin-5-yl)ethyl]piperazin-1-yl}nicotinsäure handelt.

11. Verfahren zur Herstellung einer Verbindung der Formel (I) nach einem der Ansprüche 1 bis 10, **dadurch gekennzeichnet, dass** man eine Verbindung der Formel (II): 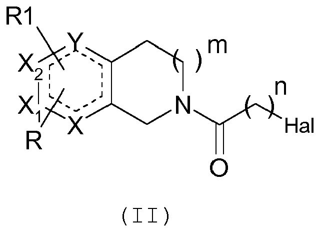 worin Y, X, X1, X2, R1, R, n und m gemäß einem der Ansprüche 1 bis 10 definiert sind und Hal für ein Halogenatom steht,
und eine Verbindung der allgemeinen Formel (III):
H-W-R2 (III)
worin W und R2 gemäß einem der Ansprüche 1 bis 10 definiert sind, umsetzt.

12. Verbindung der Formel (II): 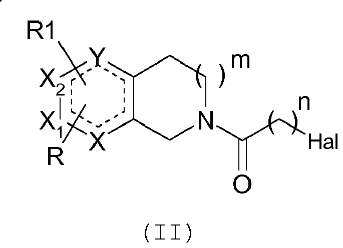 worin Y, X, X1, X2, R1, R, n und m gemäß einem der Ansprüche 1 bis 10 definiert sind und Hal für ein Halogenatom steht; in Basen- oder Säureadditionssalzform.

13. Medikament, **dadurch gekennzeichnet, dass** es eine Verbindung der Formel (I) nach einem der Ansprüche 1 bis 10 oder ein Additionssalz dieser Verbindung mit einer pharmazeutisch unbedenklichen Säure umfasst.

14. Pharmazeutische Zusammensetzung, **dadurch gekennzeichnet, dass** es eine Verbindung der Formel (I) nach einem der Ansprüche 1 bis 10 oder ein pharmazeutisch unbedenkliches Salz sowie mindestens einen pharmazeutisch unbedenklichen Hilfsstoff umfasst.

15. Verbindung nach einem der Ansprüche 1 bis 10 als Medikament.

16. Verbindung nach einem der Ansprüche 1 bis 10 zur Herstellung eines Medikaments zur Prävention oder Behandlung von degenerativen Erkrankungen des zentralen und peripheren Nervensystems, Altersdemenz, Epilepsie, Morbus Alzheimer, Morbus Parkinson, Huntington-Chorea, Down-Syndrom, Prionerkrankungen, Gedächtnisschwund, Schizophrenie, Depression, manisch-depressiver Psychose, amyotropher Lateralsklerose, multipler Sklerose, Herz-Kreislauf-Leiden, postischämischen Herzschäden, Kardiomyopathien, Myokardinfarkt, Herzinsuffizienz, Herzischämie, Hirninfarkt; peripheren Neuropathien, Schädigungen des Sehnervs und der Retina, Degeneration des Retinapigments, Glaukom, retinaler Ischämie, Makuladegeneration, Rückenmarkstraumen, Schädeltraumen, Atherosklerose, Stenosen, Vernarbungsleiden, Alopecia, Bauchspeicheldrüsenentzündung, Leberfibrose, Krebserkrankungen, Tumoren, Metastasen, Leukämien, Erkrankungen der Atemwege, Lungenentzündung, Allergie, Asthma, chronisch-obstruktiver Bronchopneumopathie, Haut-, Körper-, Eingeweide- und Nervenschmerz, chronischen neuropathischen und entzündlichen Schmerzen, Autoimmunerkrankungen, rheumatoider Polyarthritis, ankylosierender Spondylarthritis, Psoriasis-Rheumatismus, Plaque-Psoriasis, Knochenbrüchen, Knochenerkrankungen oder Osteoporose.

17. Verwendung einer Verbindung nach einem der Ansprüche 1 bis 10 bei der Herstellung von Medikamenten, die bei der Behandlung und Prävention von degenerativen Erkrankungen des zentralen und peripheren Nervensystems, Altersdemenz, Epilepsie, Morbus Alzheimer, Morbus Parkinson, Huntington-Chorea, Down-Syndrom, Prionerkrankungen, Gedächtnisschwund, Schizophrenie, Depression, manisch-depressiver Psychose, amyotropher Lateralsklerose, multipler Sklerose, Herz-Kreislauf-Leiden, postischämischen Herzschäden, Kardiomyopathien, Myokardinfarkt, Herzinsuffizienz, Herzischämie, Hirninfarkt; peripheren Neuropathien, Schädigungen des Sehnervs und der Retina, Degeneration des Retinapigments, Glaukom, retinaler Ischämie, Makuladegeneration, Rückenmarkstraumen, Schädeltraumen, Atherosklerose, Stenosen, Vernarbungsleiden, Alopecia, Bauchspeicheldrüsenentzündung, Leberfibrose, Krebserkrankungen, Tumoren, Metastasen, Leukämien, Erkrankungen der Atemwege, Lungenentzündung, Allergie, Asthma, chronisch-obstruktiver Bronchopneumopathie, Haut-, Körper-, Eingeweide- und Nervenschmerz, chronischen neuropathischen und entzündlichen Schmerzen, Autoimmunerkrankungen, rheumatoider Polyarthritis, ankylosierender Spondylarthritis, Psoriasis-Rheumatismus, Plaque-Psoriasis, Knochenbrüchen, Knochenerkrankungen oder Osteoporose verwendet werden können.

## Claims

1. Compound corresponding to formula (I): 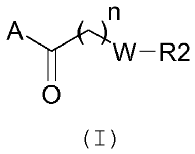 in which:
- A represents a group: 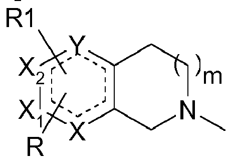
- n represents 1 or 2;
- m represents 0 or 1;
- Y represents a carbon, nitrogen, sulphur or oxygen atom or a single or double bond;
- X, X₁ and X₂ represent a carbon, nitrogen, sulphur or oxygen atom, it being understood that at least one of X, X₁ and X₂ is other than a carbon atom;
- R and R1, located on any one of the available positions, independently represent a hydrogen atom, a halogen atom, a (C1-C4)alkyl group, a (C1-C4)alkoxy group, a perfluoroalkyl radical, a trifluoromethoxy radical, a cyano, or a COOH, COOalkyl, CONR5R6 or NHCOR5 group;
or R1 represents a group chosen from: 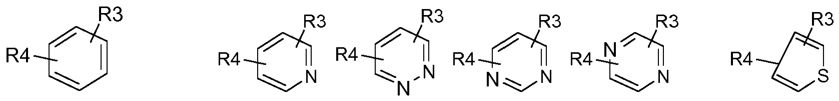 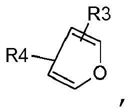 the definition of R remaining unchanged;
- R3 and R4, located on any one of the available positions, independently represent a hydrogen atom, a halogen atom, a (C1-C4)alkyl group, a (C1-C4)alkoxy group, a perfluoroalkyl radical, a trifluoromethoxy radical, a cyano, or a COOH, COOalkyl, CONR5R6 or NHCOR5 group;
- -W- is a nitrogenous heterocycle chosen from: 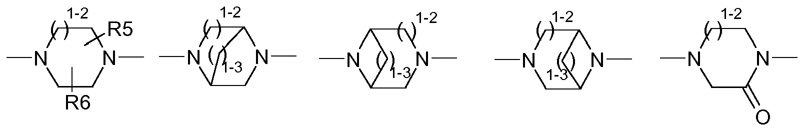
- 1-2 represents 1 or 2;
- 1-3 represents 1, 2 or 3;
- R2 represents a group of formula: 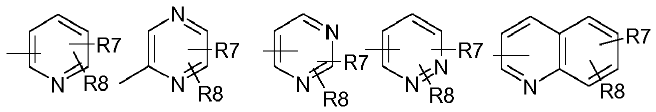
- R7 and R8, located on any one of the available positions, independently represent a hydrogen atom, a halogen atom, a (C1-C4)alkyl group, a (C1-C4)alkoxy group, a trifluoromethyl radical, a trifluoromethoxy radical, a cyano, or a COOH, COOalkyl, COOcycloalkyl, SOalkyl, SO₂alkyl, CONH₂, CONR5R6 or NHCOR5 group;
or one of R7 and R8 represents a heterocycle chosen from: 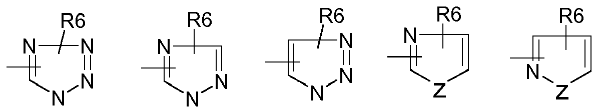 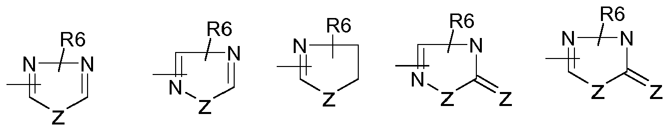
- Z represents an oxygen or sulphur atom;
- R5 and R6 represent a hydrogen or a (C1-C6) alkyl group;
in the form of a base or of an addition salt with an acid.

2. Compound according to Claim 1, such that:
- W is a group of formula chosen from: 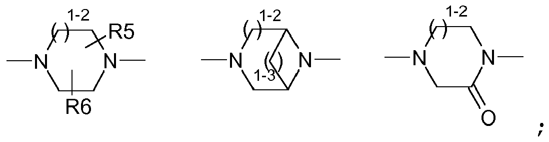
- R5 and R6 represent a hydrogen or a methyl group;
in the form of a base or of an addition salt with an acid.

3. Compound according to Claim 1 or 2, such that n represents 1;
in the form of a base or of an addition salt with an acid.

4. Compound according to any one of Claims 1 to 3, such that:
- R2 represents: 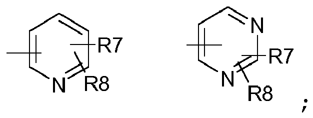
- R7 and R8, located on any one of the available positions, independently represent a hydrogen atom, a halogen atom, a (C1-C4)alkyl group, a trifluoromethyl radical, a COOH group or a COOalkyl group;
or one of R7 and R8 represents a heterocycle chosen from: 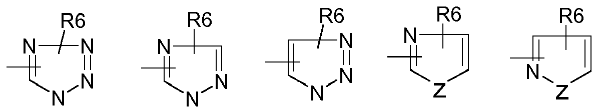 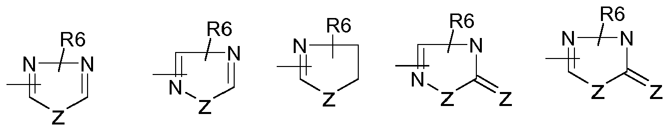
in the form of a base or of an addition salt with an acid.

5. Compound according to any one of Claims 1 to 4, such that Y represents a nitrogen atom or a single or double bond;
in the form of a base or of an addition salt with an acid.

6. Compound according to any one of Claims 1 to 5, such that X, X₁ and X₂ represent a carbon, nitrogen or sulphur atom, it being understood that at least one of X, X₁ and X₂ is other than a carbon atom;
in the form of a base or of an addition salt with an acid.

7. Compound according to any one of Claims 1 to 6, such that:
- R and R1, located on any one of the available positions, independently represent a hydrogen atom, a halogen atom or a (C1-C4)alkyl group;
or else
R1 represents a group: 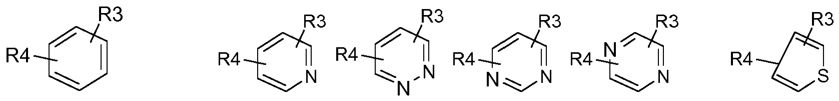 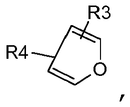 and R is a hydrogen atom;
- R3 and R4, located on any one of the available positions, represent a hydrogen atom, a halogen atom, a (C1-C4)alkoxy group or a perfluoroalkyl radical;
in the form of a base or of an addition salt with an acid.

8. Compound according to any one of the preceding claims, chosen from:
- Compound No. 1: 1-(6,7-dihydro-4H-thieno[3,2-c]pyridin-5-yl)-2-[8-(5-fluoropyrimidin-2-yl)-3,8-diazabicyclo[3.2.1]oct-3-yl]ethanone;
- Compound No. 2: 2-[8-(5-fluoropyrimidin-2-yl)-3,8-diazabicyclo[3.2.1]oct-3-yl]-1-(2-phenyl-6,7-dihydro-4H-thiazolo[5,4-c]pyridin-5-yl)ethanone;
- Compound No. 3: 1-(2-chloro-7,8-dihydro-5H-[1,6]naphthyridin-6-yl)-2-[8-(5-fluoropyrimidin-2-yl)-3,8-diazabicyclo[3.2.1]oct-3-yl]ethanone;
- Compound No. 4: 2-[8-(5-fluoropyrimidin-2-yl)-3,8-diazabicyclo[3.2.1]oct-3-yl]-1-(2-phenyl-2,4,6,7-tetrahydropyrazolo[4,3-c]pyridin-5-yl)ethanone;
- Compound No. 5: 6-{3-[2-oxo-2-(2-phenyl-6,7-dihydro-4H-thiazolo[5,4-c]pyridin-5-yl)ethyl]-3,8-diazabicyclo[3.2.1]oct-8-yl}nicotinic acid methyl ester;
- Compound No. 6: 6-{(3S,5R)-3,5-dimethyl-4-[2-oxo-2-(2-phenyl-6,7-dihydro-4H-thiazolo[5,4-c]pyridin-5-yl)ethyl]piperazin-1-yl}nicotinic acid;
- Compound No. 7: 6-{3-[2-oxo-2-(2-phenyl-6,7-dihydro-4H-thiazolo[5,4-c]pyridin-5-yl)ethyl]-3,8-diazabicyclo[3.2.1]oct-8-yl}nicotinic acid;
- Compound No. 8: 6-{(3S,5R)-3,5-dimethyl-4-[2-oxo-2-(2-phenyl-6,7-dihydro-4H-thiazolo[5,4-c]pyridin-5-yl)ethyl]piperazin-1-yl}nicotinic acid;
- Compound No. 9: 2-[(2S,6R)-2,6-dimethyl-4-(5-trifluoromethylpyridin-2-yl)piperazin-1-yl]-1-(2-phenyl-6,7-dihydro-4H-thiazolo[5,4-c]pyridin-5-yl)ethanone;
- Compound No. 10: 2-[(2S,6R)-2,6-dimethyl-4-(5-trifluoromethylpyridin-2-yl)piperazin-1-yl]-1-(2-phenyl-2,4,6,7-tetrahydropyrazolo[4,3-c]pyridin-5-yl)ethanone;
- Compound No. 11: 4-[2-oxo-2-(2-phenyl-6,7-dihydro-4H-thiazolo[5,4-c]pyridin-5-yl)ethyl]-1-(5-trifluoromethylpyridin-2-yl)piperazin-2-one;
- Compound No. 12: 1-(2-phenyl-6,7-dihydro-4H-thiazolo[5,4-c]pyridin-5-yl)-2-[4-(5-trifluoromethylpyridin-2-yl)piperazin-1-yl]ethanone;
- Compound No. 13: 1-(2-phenyl-2,4,6,7-tetrahydropyrazolo[4,3-c]pyridin-5-yl)-2-[4-(5-trifluoromethylpyridin-2-yl)piperazin-1-yl]ethanone;
- Compound No. 14: 4-[2-oxo-2-(2-phenyl-2,4,6,7-tetrahydropyrazolo[4,3-c]pyridin-5-yl)ethyl]-1-(5-trifluoromethylpyridin-2-yl)piperazin-2-one;
- Compound No. 15: 2-[(2S,6R)-4-(5-fluoropyrimidin-2-yl)-2,6-dimethylpiperazin-1-yl]-1-(2-phenyl-6,7-dihydro-4H-thiazolo[5,4-c]pyridin-5-yl)ethanone;
- Compound No. 16: 2-((2S,6R)-2,6-dimethyl-4-pyrimidin-5-yl-piperazin-1-yl)-1-(2-phenyl-2,4,6,7-tetrahydropyrazolo[4,3-c]pyridin-5-yl)ethanone;
- Compound No. 17: 2-[(2S,6R)-4-(5-fluoropyrimidin-2-yl)-2,6-dimethylpiperazin-1-yl]-1-(2-phenyl-2,4,6,7-tetrahydropyrazolo[4,3-c]pyridin-5-yl)ethanone;
- Compound No. 18: 2-[(2S,6R)-2,6-dimethyl-4-(6-trifluoromethylpyridin-3-yl)piperazin-1-yl]-1-(2-phenyl-6,7-dihydro-4H-thiazolo[5,4-c]pyridin-5-yl)ethanone;
- Compound No. 19: 6-{3-[2-oxo-2-(2-phenyl-2,4,6,7-tetrahydropyrazolo[4,3-c]pyridin-5-yl)ethyl]-3,8-diazabicyclo[3.2.1]oct-8-yl}nicotinic acid methyl ester;
- Compound No. 20: 2-[(2S,6R)-2,6-dimethyl-4-(6-trifluoromethylpyridin-3-yl)piperazin-1-yl]-1-(2-phenyl-2,4,6,7-tetrahydropyrazolo[4,3-c]pyridin-5-yl)ethanone;
- Compound No. 21: 6-{(3S,5R)-3,5-dimethyl-4-[2-oxo-2-(2-phenyl-2,4,6,7-tetrahydropyrazolo[4,3-c]pyridin-5-yl)ethyl]piperazin-1-yl}nicotinic acid;
- Compound No. 22: 2-((2S,6R)-2,6-dimethyl-4-pyrimidin-5-yl-piperazin-1-yl)-1-(2-phenyl-6,7-dihydro-4H-thiazolo[5,4-c]pyridin-5-yl)ethanone;
- Compound No. 23: 6-{(3S,5R)-3,5-dimethyl-4-[2-oxo-2-(2-phenyl-2,4,6,7-tetrahydropyrazolo[4,3-c]pyridin-5-yl)ethyl]piperazin-1-yl}nicotinic acid methyl ester;
- Compound No. 24: 6-{3-[2-oxo-2-(2-phenyl-2,4,6,7-tetrahydropyrazolo[4,3-c]pyridin-5-yl)ethyl]-3,8-diazabicyclo[3.2.1]oct-8-yl}nicotinic acid;
- Compound No. 25: 6-{2-oxo-4-[2-oxo-2-(2-phenyl-6,7-dihydro-4H-thiazolo[5,4-c]pyridin-5-yl)ethyl]piperazin-1-yl}nicotinic acid methyl ester;
- Compound No. 26: 2-[8-(5-fluoropyrimidin-2-yl)-3,8-diazabicyclo[3.2.1]oct-3-yl]-1-(1-phenyl-1,4,6,7-tetrahydropyrazolo[4,3-c]pyridin-5-yl)ethanone;
- Compound No. 27: 1-(2-phenyl-2,4,6,7-tetrahydropyrazolo[4,3-c]pyridin-5-yl)-2-(4-pyridin-3-yl[1,4]diazepan-1-yl)ethanone;
- Compound No. 28: 1-(2-phenyl-2,4,6,7-tetrahydropyrazolo[4,3-c]pyridin-5-yl)-2-(8-pyridin-3-yl-3,8-diazabicyclo[3.2.1]oct-3-yl)ethanone;
- Compound No. 29: 1-(2-phenyl-2,4,6,7-tetrahydropyrazolo[4,3-c]pyridin-5-yl)-2-[8-(5-trifluoromethylpyridin-2-yl)-3,8-diazabicyclo[3.2.1]oct-3-yl]ethanone;
- Compound No. 30: 1-(2-phenyl-6,7-dihydro-4H-thiazolo[5,4-c]pyridin-5-yl)-2-(4-pyridin -3-yl-[1,4]diazepan-1-yl)ethanone;
- Compound No. 31: 1-(2-phenyl-2,4,6,7-tetrahydropyrazolo[4,3-c]pyridin-5-yl)-2-(8-pyrimidin-5-yl-3,8-diazabicyclo[3.2.1]oct-3-yl)ethanone;
- Compound No. 32: 2-[8-(5-fluoropyrimidin-2-yl)-3,8-diazabicyclo[3.2.1]oct-3-yl]-1-(2-methyl-2,4,6,7-tetrahydropyrazolo[4,3-c]pyridin-5-yl)ethanone;
- Compound No. 33: 6-{3-[2-oxo-2-(2-phenyl-4,6-dihydropyrrolo[3,4-d]thiazol-5-yl)ethyl]-3,8-diazabicyclo[3.2.1]oct-8-yl}nicotinic acid methyl ester;
- Compound No. 34: 4-{2-[2-(4-methoxyphenyl)-2,4,6,7-tetrahydropyrazolo[4,3-c]pyridin-5-yl]-2-oxoethyl}-1-(5-trifluoromethylpyridin-2-yl)piperazin-2-one;
- Compound No. 35: 4-{2-[2-(4-fluorophenyl)-2,4,6,7-tetrahydropyrazolo[4,3-c]pyridin-5-yl]-2-oxoethyl}-1-(5-trifluoromethylpyridin-2-yl)piperazin-2-one;
- Compound No. 36: 2-[8-(5-fluoropyrimidin-2-yl)-3,8-diazabicyclo[3.2.1]oct-3-yl]-1-(2-methyl-6,7-dihydro-4H-thiazolo[5,4-c]pyridin-5-yl)ethanone;
- Compound No. 37: 6-(3-{2-[2-(4-methoxyphenyl)-6,7-dihydro-4H-thiazolo[5,4-c]pyridin-5-yl]-2-oxoethyl}-3,8-diazabicyclo[3.2.1]oct-8-yl)nicotinic acid methyl ester;
- Compound No. 38: 6-(3-{2-[2-(4-fluorophenyl)-6,7-dihydro-4H-thiazolo[5,4-c]pyridin-5-yl]-2-oxoethyl}-3,8-diazabicyclo[3.2.1]oct-8-yl)nicotinic acid methyl ester;
- Compound No. 39: 6-(3-{2-oxo-2-[2-(5-trifluoromethylpyridin-2-yl)-2,4,6,7-tetrahydropyrazolo[4,3-c]pyridin-5-yl]ethyl}-3,8-diazabicyclo[3.2.1]oct-8-yl)nicotinic acid methyl ester;
- Compound No. 40: 2-[8-(5-fluoropyrimidin-2-yl)-3,8-diazabicyclo[3.2.1]oct-3-yl]-1-[2-(5-trifluoromethylpyridin-2-yl)-2,4,6,7-tetrahydropyrazolo[4,3-c]pyridin-5-yl]ethanone;
- Compound No. 41: 4-[2-oxo-2-(2-phenyl-7,8-dihydro-5H-pyrido[4,3-d]pyrimidin-6-yl)ethyl]-1-(5-trifluoromethylpyridin-2-yl)piperazin-2-one;
- Compound No. 42: 4-[2-oxo-2-(2-thiophen-3-yl-6,7-dihydro-4H-thiazolo[5,4-c]pyridin-5-yl)ethyl]-1-(5-trifluoromethylpyridin-2-yl)piperazin-2-one;
- Compound No. 43: 2-[8-(5-fluoropyrimidin-2-yl)-3,8-diazabicyclo[3.2.1]oct-3-yl]-1-[2-(4-methoxyphenyl)-2,4,6,7-tetrahydropyrazolo[4,3-c]pyridin-5-yl]ethanone;
- Compound No. 44: 6-{3-[2-oxo-2-(2-thiophen-3-yl-6,7-dihydro-4H-thiazolo[5,4-c]pyridin-5-yl)ethyl]-3,8-diazabicyclo[3.2.1]oct-8-yl}nicotinic acid;
- Compound No. 45: 2-[8-(5-fluoropyrimidin-2-yl)-3,8-diazabicyclo[3.2.1]oct-3-yl]-1-[1-(2,2,2-trifluoroethyl)-4,5,6,7-tetrahydro-1H-indazol-5-yl]ethanone;
- Compound No. 46: 2-[8-(5-fluoropyrimidin-2-yl)-3,8-diazabicyclo[3.2.1]oct-3-yl]-1-[1-(4-methoxyphenyl)-1,4,6,7-tetrahydropyrazolo[4,3-c]pyridin-5-yl]ethanone;
- Compound No. 47: 2-[8-(5-fluoropyrimidin-2-yl)-3,8-diazabicyclo[3.2.1]oct-3-yl]-1-(2,4,6,7-tetrahydropyrazolo[4,3-c]pyridin-5-yl)ethanone;
- Compound No. 48: 6-{3-[2-oxo-2-(1-phenyl-1,4,6,7-tetrahydropyrazolo[4,3-c]pyridin-5-yl)ethyl]-3,8-diazabicyclo[3.2.1]oct-8-yl}nicotinic acid methyl ester;
- Compound No. 49: 6-{3-[2-oxo-2-(1-phenyl-1,4,6,7-tetrahydropyrazolo[4,3-c]pyridin-5-yl)ethyl]-3,8-diazabicyclo[3.2.1]oct-8-yl}nicotinic acid;
- Compound No. 50: 1-(1-tert-butyl-1,4,6,7-tetrahydropyrazolo[4,3-c]pyridin-5-yl)-2-[8-(5-fluoropyrimidin-2-yl)-3,8-diazabicyclo[3.2.1]oct-3-yl]ethanone;
- Compound No. 51 : 1-[1-(4-fluorophenyl)-1,4,6,7-tetrahydropyrazolo[4,3-c]pyridin-5-yl]-2-[8-(5-fluoropyrimidin-2-yl)-3,8-diazabicyclo[3.2.1]oct-3-yl]ethanone;
- Compound No. 52: 6-{(2R,5S)-2,5-dimethyl-4-[2-oxo-2-(2-phenyl-2,4,6,7-tetrahydropyrazolo[4,3-c]pyridin-5-yl)ethyl]piperazin-1-yl}nicotinonitrile;
- Compound No. 53: 6-{(2R,5S)-2,5-dimethyl-4-[2-oxo-2-(2-phenyl-2,4,6,7-tetrahydropyrazolo[4,3-c]pyridin-5-yl)ethyl]piperazin-1-yl}nicotinic acid;
- Compound No. 54: 2-[8-(5-fluoropyrimidin-2-yl)-3,8-diazabicyclo[3.2.1]oct-3-yl]-1-(2-phenyl-4,7-dihydro-5H-fluoro[2,3-c]pyridin-6-yl)ethanone;
- Compound No. 55: 2-[8-(5-fluoropyrimidin-2-yl)-3,8-diazabicyclo[3.2.1]oct-3-yl]-1-(2-phenyl-1,4,5,7-tetrahydropyrrolo[2,3-c]pyridin-6-yl)ethanone;
- Compound No. 56: 6-{8-[2-oxo-2-(2-phenyl-6,7-dihydro-4H-thiazolo[5,4-c]pyridin-5-yl)ethyl]-3,8-diazabicyclo[3.2.1]oct-3-yl}nicotinic acid methyl ester;
- Compound No. 57: 6-{8-[2-oxo-2-(2-phenyl-2,4,6,7-tetrahydropyrazolo[4,3-c]pyridin-5-yl)ethyl]-3,8-diazabicyclo[3.2.1]oct-3-yl}nicotinic acid methyl ester;
- Compound No. 58: 2-[8-(5-fluoropyrimidin-2-yl)-3,8-diazabicyclo[3.2.1]oct-3-yl]-1-(2-phenyl-6,7-dihydro-4H-oxazolo[4,5-c]pyridin-5-yl)ethanone;
- Compound No. 59: 6-{(2R,5S)-2,5-dimethyl-4-[2-oxo-2-(2-phenyl-6,7-dihydro-4H-thiazolo[5,4-c]pyridin-5-yl)ethyl]piperazin-1-yl}nicotinonitrile;
- Compound No. 60: 2-{8-[5-(5-methyl-[1,2,4]oxadiazol-3-yl)pyridin-2-yl]-3,8-diazabicyclo[3.2.1]oct-3-yl}-1-(2-phenyl-6,7-dihydro-4H-thiazolo[5,4-c]pyridin-5-yl)ethanone;
- Compound No. 61: 6-{8-[2-oxo-2-(2-phenyl-2,4,6,7-tetrahydropyrazolo[4,3-c] pyridin-5-yl)ethyl]-3,8-diazabicyclo[3.2.1]oct-3-yl}nicotinic acid;
- Compound No. 62: 6-{8-[2-oxo-2-(2-phenyl-6,7-dihydro-4H-thiazolo[5,4-c]pyridin-5-yl)ethyl]-3,8-diazabicyclo[3.2.1]oct-3-yl}nicotinic acid;
- Compound No. 63: 6-{(2R,5S)-2,5-dimethyl-4-[2-oxo-2-(2-phenyl-6,7-dihydro-4H-thiazolo[5,4-c]pyridin-5-yl)ethyl]piperazin-1-yl}nicotinic acid;
- Compound No. 64: 2-[8-(5-fluoropyrimidin-2-yl)-3,8-diazabicyclo[3.2.1]oct-3-yl]-1-(2-pyridin-4-yl-6,7-dihydro-4H-thiazolo[5,4-c]pyridin-5-yl)ethanone;
- Compound No. 65: 4-{2-oxo-2-[2-(5-trifluoromethylpyridin-2-yl)-2,4,6,7-tetrahydropyrazolo[4,3-c]pyridin-5-yl]ethyl}-1-(5-trifluoromethylpyridin-2-yl)piperazin-2-one;
- Compound No. 66: 4-{2-oxo-2-[1-(2,2,2-trifluoroethyl)-1,4,6,7-tetrahydropyrazolo [4,3-c]pyridin-5-yl]ethyl}-1-(5-trifluoromethylpyridin-2-yl)piperazin-2-one;
- Compound No. 67: 2-[8-(5-fluoropyrimidin-2-yl)-3,8-diazabicyclo[3.2.1]oct-3-yl]-1-(2-phenyl-7,8-dihydro-5H-pyrido[4,3-d]pyrimidin-6-yl)ethanone;
- Compound No. 68: 3-(6-{3-[2-oxo-2-(2-phenyl-2,4,6,7-tetrahydropyrazolo[4,3-c] pyridin-5-yl)ethyl]-3,8-diazabicyclo[3.2.1]oct-8-yl}pyridin-3-yl)-4H-[1,2,4]oxadiazol-5-one;
- Compound No. 69: 2-[8-(5-fluoropyrimidin-2-yl)-3,8-diazabicyclo[3.2.1]oct-3-yl]-1-(2-phenyl-3,4,6,7-tetrahydroimidazo[4,5-c]pyridin-5-yl)ethanone;
- Compound No. 70: 3-(6-{3-[2-oxo-2-(2-phenyl-6,7-dihydro-4H-thiazolo[5,4-c]pyridin-5-yl)ethyl]-3,8-diazabicyclo[3.2.1]oct-8-yl}pyridin-3-yl)-4H-[1,2,4]oxadiazol-5-one;
- Compound No. 71: 2-[8-(5-fluoropyrimidin-2-yl)-3,8-diazabicyclo[3.2.1]oct-3-yl]-1-(2-phenyl-6,7-dihydro-4H-thiazolo[4,5-c]pyridin-5-yl)ethanone;
- Compound No. 72: 2-{(3S,5R)-3,5-dimethyl-4-[2-oxo-2-(2-phenyl-6,7-dihydro-4H-thiazolo[5,4-c]pyridin-5-yl)ethyl]piperazin-1-yl}pyrimidine-5-carboxylic acid;
- Compound No. 73: 2-[8-(5-fluoropyrimidin-2-yl)-3,8-diazabicyclo[3.2.1]oct-3-yl]-1-(2-pyridin-3-yl-6,7-dihydro-4H-thiazolo[5,4-c]pyridin-5-yl)ethanone;
- Compound No. 74: 1-(2-phenyl-6,7-dihydro-4H-thiazolo[5,4-c]pyridin-5-yl)-2-{8-[5-(1H-tetrazol-5-yl)pyridin-2-yl]-3,8-diazabicyclo[3.2.1]oct-3-yl}ethanone;
- Compound No. 75: 2-{(3S,5R)-3,5-dimethyl-4-[2-oxo-2-(2-phenyl-2,4,6,7-tetrahydropyrazolo[4,3-c]pyridin-5-yl)ethyl]piperazin-1-yl}pyrimidine-5-carboxylic acid;
- Compound No. 76: 6-(3-{2-[2-(4-fluorophenyl)-6,7-dihydro-4H-thiazolo[5,4-c]pyridin-5-yl]-2-oxoethyl}-3,8-diazabicyclo[3.2.1]oct-8-yl)nicotinonitrile;
- Compound No. 77: 6-{3-[2-oxo-2-(2-phenyl-2,4,6,7-tetrahydropyrazolo[4,3-c]pyridin-5-yl)ethyl]-3,8-diazabicyclo[3.2.1]oct-8-yl}nicotinonitrile;
- Compound No. 78: 4-[2-oxo-2-(2-phenyl-3,4,6,7-tetrahydroimidazo[4,5-c]pyridin-5-yl)ethyl]-1-(5-trifluoromethylpyridin-2-yl)piperazin-2-one;
- Compound No. 79: 6-{(3S,5R)-3,5-dimethyl-4-[2-oxo-2-(2-phenyl-2,4,6,7-tetrahydropyrazolo[4,3-c]pyridin-5-yl)ethyl]piperazin-1-yl}nicotinic acid ethyl ester;
- Compound No. 80: 1-[2-(4-fluorophenyl)-6,7-dihydro-4H-thiazolo[5,4-c]pyridin-5-yl]-2-[8-(5-fluoropyrimidin-2-yl)-3,8-diazabicyclo[3.2.1]oct-3-yl]ethanone;
- Compound No. 81: 2-[8-(5-fluoropyrimidin-2-yl)-3,8-diazabicyclo[3.2.1]oct-3-yl]-1-[2-(4-methoxyphenyl)-6,7-dihydro-4H-thiazolo[5,4-c]pyridin-5-yl]ethanone;
- Compound No. 82: 4-[2-oxo-2-(2-phenyl-6,7-dihydro-4H-thiazolo[4,5-c]pyridin-5-yl)ethyl]-1-(5-trifluoromethylpyridin-2-yl)piperazin-2-one;
- Compound No. 83: 4-[2-oxo-2-(2-phenyl-6,7-dihydro-4H-oxazolo[4,5-c]pyridin-5-yl)ethyl]-1-(5-trifluoromethylpyridin-2-yl)piperazin-2-one;
- Compound No. 84: 4-[2-oxo-2-(2-pyridin-3-yl-6,7-dihydro-4H-thiazolo[5,4-c]pyridin-5-yl)ethyl]-1-(5-trifluoromethylpyridin-2-yl)piperazin-2-one;
- Compound No. 85: 2-{8-[5-(2-methyl-2H-tetrazol-5-yl)pyridin-2-yl]-3,8-diazabicyclo [3.2.1]oct-3-yl}-1-(2-phenyl-2,4,6,7-tetrahydropyrazolo[4,3-c]pyridin-5-yl)ethanone;
- Compound No. 86: 2-{(2S,6R)-2,6-dimethyl-4-[5-(1-methyl-1H-tetrazol-5-yl)-pyridin-2-yl]piperazin-1-yl}-1-(2-phenyl-2,4,6,7-tetrahydropyrazolo[4,3-c]pyridin-5-yl)ethanone;
- Compound No. 87: 2-{(2S,6R)-2,6-dimethyl-4-[5-(2-methyl-2H-tetrazol-5-yl)-pyridin-2-yl]piperazin-1-yl}-1-(2-phenyl-2,4,6,7-tetrahydropyrazolo[4,3-c]pyridin-5-yl)ethanone;
- Compound No. 88: 2-[8-(5-fluoropyrimidin-2-yl)-3,8-diazabicyclo[3.2.1]oct-3-yl]-1-[1-(4-trifluoromethylphenyl)-1,4,6,7-tetrahydropyrazolo[4,3-c]pyridin-5-yl]ethanone;
- Compound No. 89: 2-{(2S,6R)-2,6-dimethyl-4-[5-(5-methyl[1,2,4]oxadiazol-3-yl)-pyridin-2-yl]piperazin-1-yl}-1-(2-phenyl-2,4,6,7-tetrahydropyrazolo[4,3-c]pyridin-5-yl)ethanone;
- Compound No. 90: 2-[(2S,6R)-2,6-dimethyl-4-(5-[1,3,4]oxadiazol-2-ylpyridin-2-yl)piperazin-1-yl]-1-(2-phenyl-2,4,6,7-tetrahydropyrazolo[4,3-c]pyridin-5-yl)ethanone;
- Compound No. 91: 2-{(2S,6R)-2,6-dimethyl-4-[5-(3-methyl-[1,2,4]oxadiazol-5-yl)-pyridin-2-yl]piperazin-1-yl}-1-(2-phenyl-2,4,6,7-tetrahydropyrazolo[4,3-c]pyridin-5-yl)ethanone;
- Compound No. 92: 6-{(3S,5R)-4-[2-(1-tert-butyl-1,4,6,7-tetrahydropyrazolo[4,3-c]pyridin-5-yl)-2-oxoethyl]-3,5-dimethylpiperazin-1-yl}nicotinic acid methyl ester;
- Compound No. 93: 6-{(3S,5R)-4-[2-(1-tert-butyl-1,4,6,7-tetrahydropyrazolo[4,3-c]pyridin-5-yl)-2-oxoethyl]-3,5-dimethylpiperazin-1-yl}nicotinic acid;
- Compound No. 94: 6-{3-[2-(1-tert-butyl-1,4,6,7-tetrahydropyrazolo[4,3-c]pyridin-5-yl)-2-oxoethyl]-3,8-diazabicyclo[3.2.1]oct-8-yl}nicotinic acid;
- Compound No. 95: 2-[8-(5-fluoropyrimidin-2-yl)-3,8-diazabicyclo[3.2.1]oct-3-yl]-1-(2-phenyl-6,7-dihydro-4H-oxazolo[5,4-c]pyridin-5-yl)ethanone;
- Compound No. 96: 4-[2-oxo-2-(2-phenyl-6,7-dihydro-4H-oxazolo[5,4-c]pyridin-5-yl)ethyl]-1-(5-trifluoromethylpyridin-2-yl)piperazin-2-one;
- Compound No. 97: 2-[(2S,6R)-2,6-dimethyl-4-(5-trifluoromethylpyridin-2-yl)piperazin-1-yl]-1-(2-phenyl-4,7-dihydro-5H-fluoro[2,3-c]pyridin-6-yl)ethanone;
- Compound No. 98: 6-{(3S,5R)-3,5-dimethyl-4-[2-oxo-2-(2-phenyl-2,4,6,7-tetrahydropyrazolo[4,3-c]pyridin-5-yl)ethyl]piperazin-1-yl}nicotinic acid isopropyl ester;
- Compound No. 99: 1-(2-methyl-2,4,6,7-tetrahydropyrazolo[4,3-c]pyridin-5-yl)-2-[8-(5-trifluoromethylpyridin-2-yl)-3,8-diazabicyclo[3.2.1]oct-3-yl]ethanone;
- Compound No. 100: 2-[(2S,6R)-2,6-dimethyl-4-(5-trifluoromethylpyridin-2-yl)piperazin-1-yl]-1-(2-methyl-2,4,6,7-tetrahydropyrazolo[4,3-c]pyridin-5-yl)ethanone;
- Compound No. 101: 1-(2-phenyl-4,7-dihydro-5H-fluoro[2,3-c]pyridin-6-yl)-2-[8-(5-trifluoromethylpyridin-2-yl)-3,8-diazabicyclo[3.2.1]oct-3-yl]ethanone;
- Compound No. 102: 6-(3-{2-[2-(4-fluorophenyl)-6,7-dihydro-4H-thiazolo[5,4-c] pyridin-5-yl]-2-oxoethyl}-3,8-diazabicyclo[3.2.1]oct-8-yl)nicotinic acid;
- Compound No. 103: 2-[(2S,6R)-2,6-dimethyl-4-(5-trifluoromethylpyridin-2-yl)piperazin-1-yl]-1-[2-(5-trifluoromethylpyridin-2-yl)-2,4,6,7-tetrahydropyrazolo[4,3-c]pyridin-5-yl]ethanone;
- Compound No. 104: 2-[8-(5-trifluoromethylpyridin-2-yl)-3,8-diazabicyclo[3.2.1]oct-3-yl]-1-[2-(5-trifluoromethylpyridin-2-yl)-2,4,6,7-tetrahydropyrazolo[4,3-c]pyridin-5-yl]ethanone;
- Compound No. 105: 6-{(3S,5R)-4-[2-(2-tert-butyl-2,4,6,7-tetrahydropyrazolo[4,3-c]pyridin-5-yl)-2-oxoethyl]-3,5-dimethylpiperazin-1-yl}nicotinic acid;
- Compound No. 106: 2-(8-pyridin-3-yl-3,8-diazabicyclo[3.2.1]oct-3-yl)-1-[2-(5-trifluoromethylpyridin-2-yl)-2,4,6,7-tetrahydropyrazolo[4,3-c]pyridin-5-yl]ethanone;
- Compound No. 107: 2-[5-(6-trifluoromethylpyridazin-3-yl)-2,5-diazabicyclo[2.2.1] hept-2-yl]-1-[2-(5-trifluoromethylpyridin-2-yl)-2,4,6,7-tetrahydropyrazolo[4,3-c] pyridin-5-yl]ethanone;
- Compound No. 108: 2-(6'-chloro-2,3,5,6-tetrahydro[1,2']bipyrazinyl-4-yl)-1-[2-(5-trifluoromethylpyridin-2-yl)-2,4,6,7-tetrahydropyrazolo[4,3-c]pyridin-5-yl]ethanone;
- Compound No. 109: 1-(2-phenyl-3,4,6,7-tetrahydroimidazo[4,5-c]pyridin-5-yl)-2-[8-(5-trifluoromethylpyridin-2-yl)-3,8-diazabicyclo[3.2.1]oct-3-yl]ethanone;
- Compound No. 110: 2-[(2S,6R)-2,6-dimethyl-4-(5-trifluoromethylpyridin-2-yl)piperazin-1-yl]-1-(2-phenyl-3,4,6,7-tetrahydroimidazo[4,5-c]pyridin-5-yl)ethanone;
- Compound No. 111: 2-((3S,5R)-3,5-dimethyl-2,3,5,6-tetrahydro[1,2']bipyrazinyl-4-yl)-1-[2-(5-trifluoromethylpyridin-2-yl)-2,4,6,7-tetrahydropyrazolo[4,3-c]pyridin-5-yl]ethanone;
- Compound No. 112: 2-[(2S,6R)-4-(5-chloropyridin-2-yl)-2,6-dimethylpiperazin-1-yl]-1-[2-(5-trifluoromethylpyridin-2-yl)-2,4,6,7-tetrahydropyrazolo[4,3-c]pyridin-5-yl]ethanone;
- Compound No. 113: 2-[4-(7-chloroquinolin-4-yl)piperazin-1-yl]-1-(2-phenyl-2,4,6,7-tetrahydropyrazolo[4,3-c]pyridin-5-yl)ethanone;
- Compound No. 114: 2-[4-(6-chloropyridin-2-yl)piperazin-1-yl]-1-(2-phenyl-2,4,6,7-tetrahydropyrazolo[4,3-c]pyridin-5-yl)ethanone;
- Compound No. 115: 1-(2-pyridin-4-yl-6,7-dihydro-4H-thiazolo[5,4-c]pyridin-5-yl)-2-[8-(5-trifluoromethylpyridin-2-yl)-3,8-diazabicyclo[3.2.1]oct-3-yl]ethanone;
- Compound No. 116: 6-{(3S,5R)-3,5-dimethyl-4-[2-oxo-2-(2-phenyl-3,4,6,7-tetrahydroimidazo[4,5-c]pyridin-5-yl)ethyl]piperazin-1-yl}nicotinic acid;
- Compound No. 117: 1-(2-pyridin-2-yl-2,4,6,7-tetrahydropyrazolo[4,3-c]pyridin-5-yl)-2-[8-(5-trifluoromethylpyridin-2-yl)-3,8-diazabicyclo[3.2.1]oct-3-yl]ethanone;
- Compound No. 118: 2-[(2S,6R)-2,6-dimethyl-4-(5-trifluoromethylpyridin-2-yl)piperazin-1-yl]-1-(2-pyridin-2-yl-2,4,6,7-tetrahydropyrazolo[4,3-c]pyridin-5-yl)ethanone;
- Compound No. 119: 4-[2-oxo-2-(2-pyridin-2-yl-2,4,6,7-tetrahydropyrazolo[4,3-c] pyridin-5-yl)ethyl]-1-(5-trifluoromethylpyridin-2-yl)piperazin-2-one;
- Compound No. 120: 4-{2-[2-(4-fluorophenyl)-3,4,6,7-tetrahydroimidazo[4,5-c]pyridin-5-yl]-2-oxoethyl}-1-(5-trifluoromethylpyridin-2-yl)piperazin-2-one;
- Compound No. 121: 2-(8-pyridin-3-yl-3,8-diazabicyclo[3.2.1]oct-3-yl)-1-(2-pyridin-2-yl-2,4,6,7-tetrahydropyrazolo[4,3-c]pyridin-5-yl)ethanone;
- Compound No. 122: 1-[2-(2,2,2-trifluoroethyl)-2,4,6,7-tetrahydropyrazolo[4,3-c]pyridin-5-yl]-2-[8-(5-trifluoromethylpyridin-2-yl)-3,8-diazabicyclo[3.2.1]oct-3-yl]ethanone;
- Compound No. 123: 1-[2-(4-fluorophenyl)-3,4,6,7-tetrahydroimidazo[4,5-c]pyridin-5-yl]-2-[8-(5-trifluoromethylpyridin-2-yl)-3,8-diazabicyclo[3.2.1]oct-3-yl]ethanone;
- Compound No. 124: 2-[(2S,6R)-2,6-dimethyl-4-(5-trifluoromethylpyridin-2-yl)piperazin-1-yl]-1-[2-(4-fluorophenyl)-3,4,6,7-tetrahydroimidazo[4,5-c]pyridin-5-yl]ethanone;
- Compound No. 125: 4-{2-oxo-2-[2-(2,2,2-trifluoroethyl)-2,4,6,7-tetrahydropyrazolo [4,3-c]pyridin-5-yl]ethyl}-1-(5-trifluoromethylpyridin-2-yl)piperazin-2-one;
- Compound No. 126: 2-[(2S,6R)-2,6-dimethyl-4-(5-trifluoromethylpyridin-2-yl)piperazin-1-yl]-1-[2-(2,2,2-trifluoroethyl)-2,4,6,7-tetrahydropyrazolo[4,3-c]pyridin-5-yl]ethanone;
- Compound No. 127: 1-(2-phenyl-2,4,6,7-tetrahydropyrazolo[4,3-c]pyridin-5-yl)-2-[5-(5-trifluoromethylpyridin-2-yl)-2,5-diazabicyclo[2.2.1]hept-2-yl]ethanone;
- Compound No. 128: 6-{3-[2-oxo-2-(2-phenyl-2,4,6,7-tetrahydropyrazolo[4,3-c]pyridin-5-yl)ethyl]-3,8-diazabicyclo[3.2.1]oct-8-yl}nicotinic acid cyclobutyl ester;
- Compound No. 129: 2-((2S,6R)-2,6-dimethyl-4-quinolin-2-ylpiperazin-1-yl)-1-(2-phenyl-2,4,6,7-tetrahydropyrazolo[4,3-c]pyridin-5-yl)ethanone;
- Compound No. 130: 6-{(3S,5R)-3,5-dimethyl-4-[2-oxo-2-(2-phenyl-2,4,6,7-tetrahydropyrazolo[4,3-c]pyridin-5-yl)ethyl]piperazin-1-yl}nicotinic acid ethyl ester;
- Compound No. 131 : 2- [(2S,6R)-4-(5-methanesulphonylpyridin-2-yl)-2,6-dimethylpiperazin-1-yl]-1-(2-phenyl-2,4,6,7-tetrahydropyrazolo[4,3-c]pyridin-5-yl)ethanone;
- Compound No. 132: 2-[(2S,6R)-4-(5-fluoropyrimidin-2-yl)-2,6-dimethylpiperazin-1-yl]-1-[2-(4-methoxyphenyl)-6,7-dihydro-4H-thiazolo[5,4-c]pyridin-5-yl]ethanone;
- Compound No. 133: 1-[2-(4-methoxyphenyl)-6,7-dihydro-4H-thiazolo[5,4-c]pyridin-5-yl]-2-[5-(5-trifluoromethylpyridin-2-yl)-2,5-diazabicyclo[2.2.1]hept-2-yl]ethanone;
- Compound No. 134: 1-(2-phenyl-6,7-dihydro-4H-thiazolo[5,4-c]pyridin-5-yl)-2-{4-[5-(2H-pyrazol-3-yl)pyridin-2-yl]piperazin-1-yl}ethanone;
- Compound No. 135: 2-[(2S,6R)-2,6-dimethyl-4-(5-thiazol-2-ylpyridin-2-yl)piperazin-1-yl]-1-(2-phenyl-2,4,6,7-tetrahydropyrazolo[4,3-c]pyridin-5-yl)ethanone;
- Compound No. 136: 1-(2-phenyl-6,7-dihydro-4H-thiazolo[5,4-c]pyridin-5-yl)-2-[8-(5-thiazol-2-ylpyridin-2-yl)-3,8-diazabicyclo[3.2.1]oct-3-yl]ethanone;
- Compound No. 137: 2-[8-(5-[1,2,4]oxadiazol-3-ylpyridin-2-yl)-3,8-diazabicyclo [3.2.1]oct-3-yl]-1-(2-phenyl-2,4,6,7-tetrahydropyrazolo[4,3-c]pyridin-5-yl)ethanone;
- Compound No. 138: 1-(2-ethyl-2,4,6,7-tetrahydropyrazolo[4,3-c]pyridin-5-yl)-2-[8-(5-trifluoromethylpyridin-2-yl)-3,8-diazabicyclo[3.2.1]oct-3-yl]ethanone;
- Compound No. 139: 2-[(2S,6R)-2,6-dimethyl-4-(5-[1,2,4]oxadiazol-5-ylpyridin-2-yl)piperazin-1-yl]-1-(2-phenyl-2,4,6,7-tetrahydropyrazolo[4,3-c]pyridin-5-yl)ethanone;
- Compound No. 140: 6-{(3S,5R)-3,5-dimethyl-4-[2-oxo-2-(2-pyridin-2-yl-2,4,6,7-tetrahydropyrazolo[4,3-c]pyridin-5-yl)ethyl]piperazin-1-yl}nicotinic acid;
- Compound No. 141: 2-((2S,6R)-2,6-dimethyl-4-pyridin-3-ylpiperazin-1-yl)-1-(2-pyridin-4-yl-2,4,6,7-tetrahydropyrazolo[4,3-c]pyridin-5-yl)ethanone;
- Compound No. 142: 2-((2S,6R)-2,6-dimethyl-4-pyridin-3-ylpiperazin-1-yl)-1-[2-(5-fluoropyridin-2-yl)-2,4,6,7-tetrahydropyrazolo[4,3-c]pyridin-5-yl]ethanone;
- Compound No. 143: 2-((2S,6R)-2,6-dimethyl-4-pyridin-3-ylpiperazin-1-yl)-1-[2-(5-trifluoromethylpyridin-2-yl)-2,4,6,7-tetrahydropyrazolo[4,3-c]pyridin-5-yl]ethanone;
- Compound No. 144: 6-{(3S,5R)-3,5-dimethyl-4-[2-oxo-2-(2-pyridin-2-yl-2,4,6,7-tetrahydropyrazolo[4,3-c]pyridin-5-yl)ethyl]piperazin-1-yl}nicotinic acid methyl ester;
- Compound No. 145: 2-((2S,6R)-2,6-dimethyl-4-pyridin-3-ylpiperazin-1-yl)-1-(2-phenyl-3,4,6,7-tetrahydroimidazo[4,5-c]pyridin-5-yl)ethanone;
- Compound No. 146: 2-[(2S,6R)-2,6-dimethyl-4-(5-trifluoromethylpyridin-2-yl)piperazin-1-yl]-1-(2-pyridazin-3-yl-2,4,6,7-tetrahydropyrazolo[4,3-c]pyridin-5-yl)ethanone;
- Compound No. 147: 2-[(2S,6R)-2,6-dimethyl-4-(5-trifluoromethylpyridin-2-yl)piperazin-1-yl]-1-(2-ethyl-2,4,6,7-tetrahydropyrazolo[4,3-c]pyridin-5-yl)ethanone;
- Compound No. 148: 4-[2-(2-ethyl-2,4,6,7-tetrahydropyrazolo[4,3-c]pyridin-5-yl)-2-oxoethyl]-1-(5-trifluoromethylpyridin-2-yl)piperazin-2-one;
- Compound No. 149: 6-{(3S,5R)-3,5-dimethyl-4-[2-oxo-2-(2-phenyl-2,4,6,7-tetrahydropyrazolo[4,3-c]pyridin-5-yl)ethyl]piperazin-1-yl}nicotinic acid;
- Compound No. 150: 6-{(3S,5R)-3,5-dimethyl-4-[2-oxo-2-(2-phenyl-2,4,6,7-tetrahydropyrazolo[4,3-c]pyridin-5-yl)ethyl]piperazin-1-yl}nicotinic acid;
in the form of a base or of an addition salt with an acid.

9. Compound according to any one of Claims 1 to 7, chosen from the following compounds:

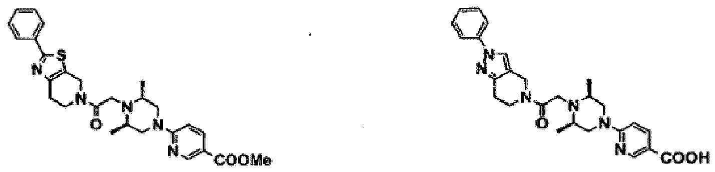

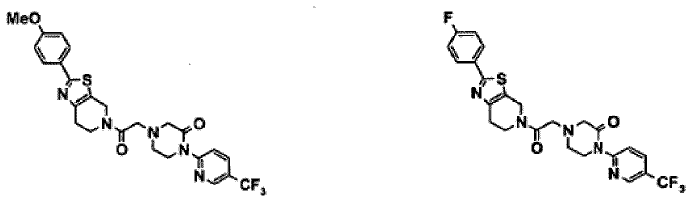

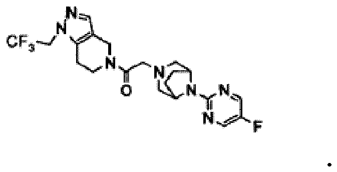

10. Compound according to any one of the preceding claims, chosen as being 6-{(3S,5R)-3,5-dimethyl-4-[2-oxo-2-(2-phenyl-2,4,6,7-tetrahydropyrazolo[4,3-c]pyridin-5-yl)ethyl]piperazin-1-yl}nicotinic acid.

11. Process for preparing a compound of formula (I) according to any one of Claims 1 to 10, **characterized in that** a compound of formula (II): 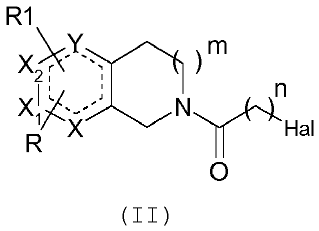 in which Y, X, X1, X2, R1, R, n and m are defined according to any one of Claims 1 to 10 and Hal represents a halogen atom,
is reacted with a compound of general formula (III):
H-W-R2 (III)
in which W and R2 are as defined in any one of Claims 1 to 10.

12. Compound of formula (II): 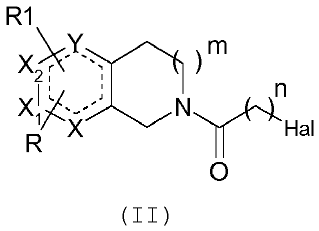 in which Y, X, X1, X2, R1, R, n and m are defined according to any one of Claims 1 to 10 and Hal represents a halogen atom;
in the form of a base or of an addition salt with an acid.

13. Medicament, **characterized in that** it comprises a compound of formula (I) according to any one of Claims 1 to 10, or an addition salt of this compound with a pharmaceutically acceptable acid.

14. Pharmaceutical composition, **characterized in that** it comprises a compound of formula (I) according to any one of Claims 1 to 10, or a pharmaceutically acceptable salt, and also at least one pharmaceutically acceptable excipient.

15. Compound according to any one of Claims 1 to 10, as a medicament.

16. Compound according to any one of Claims 1 to 10, for the preparation of a medicament for use in the prevention or treatment of central and peripheral neurodegenerative diseases, senile dementia, epilepsy, Alzheimer's disease, Parkinson's disease, Huntington's chorea, Down's syndrome, prion diseases, amnesia, schizophrenia, depression, bipolar disorder, amyotrophic lateral sclerosis, multiple sclerosis, cardiovascular conditions, post-ischaemic cardiac damage, cardiomyopathies, myocardial infarction, heart failure, cardiac ischaemia, cerebral infarction; peripheral neuropathies, damage to the optic nerve and to the retina, retinal pigment degeneration, glaucoma, retinal ischaemia, macular degeneration, spinal cord traumas, cranial traumas, atherosclerosis, stenoses, cicatrization disorders, alopecia, pancreatitis, hepatic fibrosis, cancers, tumours, metastases, leukaemias, respiratory disorders, pulmonary inflammation, allergy, asthma, chronic obstructive pulmonary disease, cutaneous, somatic, visceral and neurological pain, chronic neuropathic and inflammatory pain, autoimmune diseases, rheumatoid arthritis, ankylosing spondylarthritis, psoriatic arthritis, plaque psoriasis, bone fractures, bone diseases and osteoporosis.

17. Use of a compound according to one of Claims 1 to 10, for the preparation of medicaments of use in the treatment and prevention of central and peripheral neurodegenerative diseases, senile dementia, epilepsy, Alzheimer's disease, Parkinson's disease, Huntington's chorea, Down's syndrome, prion diseases, amnesia, schizophrenia, depression, bipolar disorder, amyotrophic lateral sclerosis, multiple sclerosis, cardiovascular conditions, post-ischaemic cardiac damage, cardiomyopathies, myocardial infarction, heart failure, cardiac ischaemia, cerebral infarction; peripheral neuropathies, damage to the optic nerve and to the retina, retinal pigment degeneration, glaucoma, retinal ischaemia, macular degeneration, spinal cord traumas, cranial traumas, atherosclerosis, stenoses, cicatrization disorders, alopecia, pancreatitis, hepatic fibrosis, cancers, tumours, metastases, leukaemias, respiratory disorders, pulmonary inflammation, allergy, asthma, chronic obstructive pulmonary disease, cutaneous, somatic, visceral and neurological pain, chronic neuropathic and inflammatory pain, autoimmune diseases, rheumatoid arthritis, ankylosing spondylarthritis, psoriatic arthritis, plaque psoriasis, bone fractures, bone diseases and osteoporosis.
